# EUROPEAN PATENT APPLICATION

(11) **EP 2 762 142 A1**
(43) Date of publication of application: **06.08.2014**
(21) Application number: 13189355.4
(22) Date of filing: 01.11.2010
(51) Int. Cl.: A61K 31/5025, A61P 35/00, A61P 35/02

(54) **Compositions for treating cancer**

(30) Priority: 30.10.2009 US 256669 P; 30.10.2009 US 256690 P; 13.11.2009 US 261014 P
(62) Divisional of application: 10827625.4
(71) Applicant: ARIAD Pharmaceuticals, Inc., Cambridge, MA 02139 (US)
(72) Inventor: Huang, Wei-Sheng, Acton, MA 01720 (US); Rivera, Victor, M., Arlington, MA 02476 (US); Clackson, Timothy, P., Lexington, MA 02420 (US); Shakespeare, William, C., Southbrough, MA 01772 (US); Squillace, Rachel, M., Wilmington, MA 01887 (US); Gozgit, Joseph, M., Framingham, MA 01701 (US)
(74) Representative: Duncan, Garreth Andrew

(57) **Abstract**

The invention features pharmaceutical compositions for treating cancer using 3-(imidazole[1,2-b]pyridazin-3-ylethynyl)-4-methyl-N-(4-((4-methylpiperazin-1-yl)-methyl)-3-(trifluoromethyl)phenyl)benzamide.

## Description

### Background of the Invention

This invention relates to pharmaceutical compositions and therapeutic methods based on the multi-kinase inhibitor, ponatinib ("compound 1") for the treatment of disorders associated with pathological cellular proliferation, such as neoplasms, cancer, and conditions associated with pathological angiogenesis.

The protein kinases are a large family of proteins which play a central role in the regulation of a wide variety of cellular processes. A partial, non limiting, list of such kinases includes abl, Akt, BCR-ABL, Blk, Brk, c-KIT, c-met, c-src, CDK1, CDK2, CDK3, CDK4, CDK5, CDK6, CDK7, CDK8, CDK9, CDK10, cRafl, CSK, EGFR, ErbB2, ErbB3, ErbB4, Erk, Pak, fes, FGFR1, FGFR2, FGFR3, FGFR4, FGFR5, Fgr, FLT1, FLT3, Fps, Frk, Fyn, Hck, IGF-1R, INS-R, Jak, KDR, Lck, Lyn, MEK, p38, PDGFR, PIK, PKC, PYK2, ros, tie, tie2, TRK, and Zap70. Abnormal protein kinase activity has been related to several disorders, ranging from non-life threatening diseases such as psoriasis to extremely serious diseases such as cancers.

Kinase inhibitors have been developed and used therapeutically with some important successes. However, not all of the targeted patients respond to those kinase inihibitors, and some become refractory to a given inhibitor through the emergence of mutation in the kinase or by other mechanisms. Currently approved kinase inhibitors can cause problematic side effects, and some patients are or become intolerant to a given inhibitor. Unfortunately, a significant unmet medical need for new and better treatments persists.

The abnormal tyrosine kinase, BCR-ABL, for example, is the hallmark of chronic myeloid leukemia (CML) and Philadelphia chromosome positive acute lymphoblastic leukemia (Ph+ ALL). Some patients treated with imatinib, a tyrosine inhibitor ("TKI") of BCR-ABL, develop resistance to imatinib. Resistance to imatinib has been linked to the emergence of a variety of mutations in BCR-ABL. The second generation BCR-ABL inhibitors, dasatinib and nilotinib, have made an important contribution and inhibit many mutant BCR-ABL species, but are still ineffective against at least one such mutant, the T315I mutant. Currently, there is no standard therapy for CML or Ph⁺ acute myeloid leukemia patients after failure of second generation TKIs. Importantly, there is no available targeted therapy for patients carrying the T3151 mutation- a mutant resistant to all currently approved TKIs.

Other examples of tyrosine kinases implicated in the initiation and progression of multiple cancers include FMS-like tyrosine kinase 3 (FLT3), fibroblast growth factor receptors (FGFR), vascular endothelial growth factor (VEGF) receptors, and the angiopoietin receptor, TIE2.

Constitutive activation of FLT3 due to an internal tandem duplication (ITD) is found in approximately one-third of patients with acute myeloblastic leukemia (AML).

Fibroblast growth factor receptors (FGFR) are known to be activated in several solid tumors, including endometrial cancer, breast cancer, non-small cell lung cancer (NSCLC) and gastric cancer, as well as multiple myeloma.

Inappropriate angiogenesis mediated by VEGFR and other kinases is implicated in various cancers such as glioblastoma and colorectal cancer and in a variety of other proliferative disorders as well.

In view of the large number of protein kinases and associated diseases, there is an ever-existing need for new inhibitors, or combinations of inhibitors, that are selective for various protein kinases and might be useful in the treatment of related diseases, including among others, an ABL inhibitors capable of inhibiting BCR-ABL^{T315I}.

### Summary of the Invention

This invention concerns a potent, orally active inhibitor, ponatinib ("compound 1") and pharmaceutical compositions and uses thereof. A very promising pharmacological profile of compound 1 has taken shape, based on biochemical testing, cell-based experiments, animal studies and results to date from human clinical studies.

As disclosed in further detail below, compound 1 is an orally active multi-targeted kinase inhibitor. It is the most potent BCR-ABL inhibitor yet described and the first pan-BCR-ABL inhibitor able to inhibit all known mutant forms of the target, including the currently untreatable T315I mutant that leads to resistance to other drugs. In a phase 1 clinical trial, it demonstrated an attractive safety profile and substantial antileukemic activity in patients with refractory hematological cancers (including a majority of patients with CML and Ph⁺ ALL), including patients in which dasatinib and nilotinib are not effective.

The pharmacokinetic and pharmacodynamic characteristics of compound 1, representing the sum of its kinase inhibitory activities, absorption, distribution, metabolism and excretion behavior in the body, are the characteristics of an orally bioavailable compound capable of achieving concentrations effective for inhibiting a targeted kinase, and in the case of BCR-ABL, for suppressing the outgrowth of cells expressing resistant mutants. The attractive safety profile to date reflects success in achieving those objectives without undue unintended inhibition of kinase activity required for normal functions.

The significance of that selectivity and safety profile is underscored by the potent activity of compound 1 in inhibiting a range of kinases beyond BCR-ABL and its mutants. For example, compound 1 inhibited FLT3, all 4 members of the FGF receptor family, all 3 VEGF receptors, the angiopoietin receptor TIE2, but was inactive against numerous other kinase classes including the insulin receptor, Aurora kinase, and cyclin-dependent kinase families.

The invention thus features pharmaceutical compositions and kits containing 3-(imidazo[1,2-b]pyridazin-3-ylethynyl)4-methyl-N-(4-((4-methylpiperazin-1-yl)-methyl)-3-(trifluoromethyl)phenyl)benzamide (compound 1), depicted below:

### compound 1

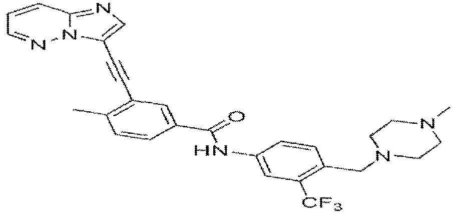

or a pharmaceutically acceptable salt thereof, and therapeutic uses thereof for treating cancer and other diseases.

Accordingly, an aspect of the invention features a pharmaceutical composition suitable for oral administration including compound 1, or a pharmaceutically acceptable salt thereof, in an amount effective to treat a neoplasm, a cancer, or a hyperproliferative disorder when administered to a subject, and one or more pharmaceutically acceptable excipients. The compound 1, or a pharmaceutically acceptable salt thereof, can be, for example, the hydrochloride salt. In particular embodiments, the pharmaceutical composition is formulated in unit dosage form. In certain embodiments, the unit dosage form can contain from 30 to 300 mg of compound 1. Exemplary unit dosage forms include from 5 to 100 mg, 5 to 80 mg, 5 to 50 mg, 5 to 20 mg, 7 to 100 mg, 7 to 80 mg, 7 to 50 mg, 7 to 20 mg, 10 to 100 mg, 10 to 80 mg, 10 to 50 mg, 15 to 100 mg, 15 to 80 mg, 15 to 60 mg, 15 mg to 50 mg, 20 to 100 mg, 20 to 80 mg, 20 to 50 mg, 30 to 100 mg, 35 to 100 mg, 40 to 100 mg, 50 to 100 mg, 60 to 100 mg, 70 to 100 mg, 30 to 80 mg, 35 to 80 mg, 40 to 80 mg, 50 to 80 mg, 60 to 80 mg, 50 to 300 mg, 60 to 300 mg, 70 to 300 mg, 50 to 200 mg, 60 to 200 mg, 70 to 200 mg, 100 to 300 mg, 120 to 300 mg, 140 to 300 mg, or 100 to 200 mg of compound 1, or a pharmaceutically acceptable salt thereof. In other embodiments, the unit dosage form can contain from 20 ± 4 mg, 25 ± 5 mg, 30 ± 6 mg, 40 ± 8 mg, 45 ± 9 mg. Exemplary unit dosage forms include those having 5 ± 1 mg, 7 ± 1.5 mg, 10 ± 2 mg, 15 ± 3 mg, 20 ± 4 mg, 25 ± 5 mg, 30 ± 6 mg, 40 ± 8 mg, 45 ± 9 mg, 55 ± 11 mg, 60 ± 12 mg, 65 ± 13 mg, 70 ± 14 mg, 75 ± 15 mg, 80 ± 16 mg, 90 ± 18 mg, 100 ± 20 mg, 120 ± 24 mg, 140 ± 28 mg, 160 ± 32 mg, 180 ± 36 mg, 200 ± 40 mg, 220 ± 44 mg, 240 ± 48 mg, or 260 ± 52 mg of compound 1 or a pharmaceutically acceptable salt thereof.

In another embodiment, the pharmaceutical composition is formulated in a solid unit dosage form (e.g., a tablet, a soft capsule, or a hard capsule). In certain embodiments, the unit dosage form can contain from 30 to 300 mg of compound 1. Exemplary unit dosage forms include from 5 to 100 mg, 5 to 80 mg, 5 to 50 mg, 5 to 20 mg, 7 to 100 mg, 7 to 80 mg, 7 to 50 mg, 7 to 20 mg, 10 to 100 mg, 10 to 80 mg, 10 to 50 mg, 15 to 100 mg, 15 to 80 mg, 15 to 60 mg, 15 mg to 50 mg, 20 to 100 mg, 20 to 80 mg, 20 to 50 mg, 30 to 100 mg, 35 to 100 mg, 40 to 100 mg, 50 to 100 mg, 60 to 100 mg, 70 to 100 mg, 30 to 80 mg, 35 to 80 mg, 40 to 80 mg, 50 to 80 mg, 60 to 80 mg, 50 to 300 mg, 60 to 300 mg, 70 to 300 mg, 50 to 200 mg, 60 to 200 mg, 70 to 200 mg, 100 to 300 mg, 120 to 300 mg, 140 to 300 mg, or 100 to 200 mg of compound 1, or a pharmaceutically acceptable salt thereof. In other embodiments, the unit dosage form can contain from 5 ± 1 mg, 7 ± 1.5 mg, 10 ± 2 mg, 15 ± 3 mg, 20 ± 4 mg, 25 ± 5 mg, 30 ± 6 mg, 40 ± 8 mg, 45 ± 9 mg. Exemplary unit dosage forms include those having 5 ± 1 mg, 7 ± 1.5 mg, 10 ± 2 mg, 15 ± 3 mg, 20 ± 4 mg, 25 ± 5 mg, 30 ± 6 mg, 40 ± 8 mg, 45 ± 9 mg, 55 ± 11 mg, 60 ± 12 mg, 65 ± 13 mg, 70 ± 14 mg, 75 ± 15 mg, 80 ± 16 mg, 90 ± 18 mg, 100 ± 20 mg, 120 ± 24 mg, 140 ± 28 mg, 160 ± 32 mg, 180 ± 36 mg, 200 ± 40 mg, 220 ± 44 mg, 240 ± 48 mg, or 260 ± 52 mg of compound 1 or a pharmaceutically acceptable salt thereof.

In another aspect, the invention features a method of treating a neoplasm, a cancer, or a hyperproliferative disorder in a subject in need thereof by orally administering to said subject from 30 to 300 mg of compound 1, or a pharmaceutically acceptable salt thereof. Exemplary unit dosage forms include from 5 to 100 mg, 5 to 80 mg, 5 to 50 mg, 5 to 20 mg, 7 to 100 mg, 7 to 80 mg, 7 to 50 mg, 7 to 20 mg, 10 to 100 mg, 10 to 80 mg, 10 to 50 mg, 15 to 100 mg, 15 to 80 mg, 15 to 60 mg, 15 mg to 50 mg, 20 to 100 mg, 20 to 80 mg, 20 to 50 mg, 30 to 100 mg, 35 to 100 mg, 40 to 100 mg, 50 to 100 mg, 60 to 100 mg, 70 to 100 mg, 30 to 80 mg, 35 to 80 mg, 40 to 80 mg, 50 to 80 mg, 60 to 80 mg, 50 to 300 mg, 60 to 300 mg, 70 to 300 mg, 50 to 200 mg, 60 to 200 mg, 70 to 200 mg, 100 to 300 mg, 120 to 300 mg, 140 to 300 mg, or 100 to 200 mg of compound 1, or a pharmaceutically acceptable salt thereof. In other embodiments, the unit dosage form can contain from 5 ± 1 mg, 7 ± 1.5 mg, 10 ± 2 mg, 15 ± 3 mg, 20 ±4 mg, 25 ± 5 mg, 30 ± 6 mg, 40 ± 8 mg, 45 ± 9 mg. Exemplary unit dosage forms include those having 5 ± 1 mg, 7 ± 1.5 mg, 10 ± 2 mg, 15 ± 3 mg, 20 ± 4 mg, 25 ± 5 mg, 30 ± 6 mg, 40 ± 8 mg, 45 ± 9 mg, 55 ± 11 mg, 60 ± 12 mg, 65 ± 13 mg, 70 ± 14 mg, 75 ± 15 mg, 80 ± 16 mg, 90 ± 18 mg, 100 ± 20 mg, 120 ± 24 mg, 140 ± 28 mg, 160 ± 32 mg, 180 ± 36 mg, 200 ± 40 mg, 220 ± 44 mg, 240 ± 48 mg, or 260 ± 52 mg of compound 1 or a pharmaceutically acceptable salt thereof. In particular embodiments, an average daily dose of from 30 to 300 mg of compound 1, or a pharmaceutically acceptable salt thereof, is orally administered to the subject in a unit dosage form (e.g., an average daily dose of from 20 to 100 mg, 20 to 80 mg, 20 to 50 mg, 30 to 100 mg, 35 to 100 mg, 40 to 100 mg, 50 to 100 mg, 60 to 100 mg, 70 to 100 mg, 30 to 80 mg, 35 to 80 mg, 40 to 80 mg, 50 to 80 mg, 60 to 80 mg, 50 to 300 mg, 60 to 300 mg, 70 to 300 mg, 50 to 200 mg, 60 to 200 mg, 70 to 200 mg, 100 to 300 mg, 120 to 300 mg, 140 to 300 mg, or 100 to 200 mg of compound 1, or a pharmaceutically acceptable salt thereof; or an average daily dose of 20 ± 4 mg, 25 ± 5 mg, 30 ± 6 mg, 40 ± 8 mg, 45 ± 9 mg, 55 ± 11 mg, 60 ± 12 mg, 65 ± 13 mg, 70 ± 14 mg, 75 ± 15 mg, 80 ± 16 mg, 90 ± 18 mg, 100 ± 20 mg, 120 ± 24 mg, 140 ± 28 mg, 160 ± 32 mg, 180 ± 36 mg, 200 ± 40 mg, 220 ± 44 mg, 240 ± 48 mg, or 260 ± 52 mg of compound 1, or a pharmaceutically acceptable salt thereof). Compound 1, or a pharmaceutically acceptable salt thereof, can be administered to the subject more than one day a week or on average 4 to 7 times every 7 day period (e.g., 4 times a week, 5 times a week, 6 times a week, or 7 times a week). In certain embodiments, compound 1, or a pharmaceutically acceptable salt thereof, is administered to the subject daily. In particular embodiments, the subject has chronic myelogenous leukemia, acute lymphoblastic leukemia, acute myelogenous leukemia, a myelodysplastic syndrome, gastric cancer, endometrial cancer, bladder cancer, multiple myeloma, breast cancer, prostate cancer, lung cancer, colorectal cancer, renal cancer, or glioblastoma. In yet other embodiments, the subject has a condition refractory to treatment with imatinib, nilotinib, or dasatinib. In further embodiments, the subject has a condition intolerant to treatment with imatinib, nilotinib, or dasatinib. In other embodiments, the subject has a Philadelphia chromosome positive condition. In yet other embodiments, the subject has a solid cancer refractory to treatment with a VEGF or VEGF-R inhibitor or antagonist (e.g., bevacizumab, sorafenib, or sunitinib). In further embodiments, the subject has a condition intolerant to treatment with a VEGF or VEGF-R inhibitor or antagonist (e.g., bevacizumab, sorafenib, or sunitinib). In some embodiments, the subject has a cancer expressing a BCR-ABL mutant (e.g., BCR-ABL^{T3151}, BCR-ABL^{F317L}, or BCR-ABL^{F359C}). In other embodiments, the subject has a cancer expressing a FLT3, KIT, FGFR1, or PDGFRα mutant (e.g., FLT3-ITD, c-KIT, FGFR1OP2-FGFR1, or F1P1L1-PDGFRα). In a further embodiment, the compound 1, or a pharmaceutically acceptable salt thereof, is administered together or concurrently with an mTOR inhibitor each in an amount that together is effective to treat said neoplasm, cancer, or hyperproliferative disorder. In some embodiments, the mTOR inhibitor is selected from sirolimus, everolimus, temsirolimus, ridaforolimus, biolimus, zotarolimus, LY294002, Pp242, WYE-354, Ku-0063794, XL765, AZD8055, NVP-BEZ235, OSI-027, wortmannin, quercetin, myricentin, and staurosporine, and pharmaceutically acceptable salts thereof.

In a further aspect, the invention features a kit including (i) a pharmaceutical composition suitable for oral administration of compound 1, or a pharmaceutically acceptable salt thereof, in an amount effective to treat a neoplasm, a cancer, or a hyperproliferative disorder when administered to a subject, and one or more pharmaceutically acceptable excipients; and (ii) instruction for administering the pharmaceutical composition to a subject for the treatment of neoplasm, cancer, or hyperproliferative disorder. In some embodiments, the subject has chronic myelogenous leukemia, acute lymphoblastic leukemia, acute myelogenous leukemia, a myelodysplastic syndrome, gastric cancer, endometrial cancer, bladder cancer, multiple myeloma, breast cancer, prostate cancer, lung cancer, colorectal cancer, renal cancer, or glioblastoma.

In yet another aspect, the invention features a method of treating a neoplasm, a cancer, or a hyperproliferative disorder in a subject in need thereof by orally administering to said subject from 5 to 300 mg of compound 1, or a pharmaceutically acceptable salt thereof. Exemplary unit dosage forms include from 5 to 100 mg, 5 to 80 mg, 5 to 50 mg, 5 to 20 mg, 7 to 100 mg, 7 to 80 mg, 7 to 50 mg, 7 to 20 mg, 10 to 100 mg, 10 to 80 mg, 10 to 50 mg, 15 to 100 mg, 15 to 80 mg, 15 to 60 mg, 15 mg to 50 mg, 20 to 100 mg, 20 to 80 mg, 20 to 50 mg, 30 to 100 mg, 35 to 100 mg, 40 to 100 mg, 50 to 100 mg, 60 to 100 mg, 70 to 100 mg, 30 to 80 mg, 35 to 80 mg, 40 to 80 mg, 50 to 80 mg, 60 to 80 mg, 50 to 300 mg, 60 to 300 mg, 70 to 300 mg, 50 to 200 mg, 60 to 200 mg, 70 to 200 mg, 100 to 300 mg, 120 to 300 mg, 140 to 300 mg, or 100 to 200 mg of compound 1, or a pharmaceutically acceptable salt thereof. In other embodiments, the unit dosage form can contain from 5 ± 1 mg, 7 ± 1.5 mg, 10 ± 2 mg, 15 ± 3 mg, 20 ± 4 mg, 25 ± 5 mg, 30 ± 6 mg, 40 ± 8 mg, 45 ± 9 mg, 55 ± 11 mg, 60 ± 12 mg, 65 ± 13 mg, 70 ± 14 mg, 75 ± 15 mg, 80 ± 16 mg, 90 ± 18 mg, 100 ± 20 mg, 120 ± 24 mg, 140 ± 28 mg, 160 ± 32 mg, 180 ± 36 mg, 200 ± 40 mg, 220 ± 44 mg, 240 ± 48 mg, or 260 ± 52 mg of compound 1 or a pharmaceutically acceptable salt thereof. In particular embodiments, an average daily dose of from 5 to 300 mg of compound 1, or a pharmaceutically acceptable salt thereof, is orally administered to the subject in a unit dosage form (e.g., an average daily dose of from 5 to 100 mg, 5 to 80 mg, 5 to 50 mg, 5 to 20 mg, 7 mg to 100 mg, 7 mg to 80 mg, 7 to 50 mg, 7 to 20 mg, 10 mg to 100 mg, 10 mg to 80 mg, 10 to 50 mg, 15 mg to 100 mg, 15 mg to 80 mg, 15 to 60 mg, 15 mg to 50 mg, 20 to 100 mg, 20 to 80 mg, 20 to 50 mg, 30 to 100 mg, 35 to 100 mg, 40 to 100 mg, 50 to 100 mg, 60 to 100 mg, 70 to 100 mg, 30 to 80 mg, 35 to 80 mg, 40 to 80 mg, 50 to 80 mg, 60 to 80 mg, 50 to 300 mg, 60 to 300 mg, 70 to 300 mg, 50 to 200 mg, 60 to 200 mg, 70 to 200 mg, 100 to 300 mg, 120 to 300 mg, 140 to 300 mg, or 100 to 200 mg of compound 1, or a pharmaceutically acceptable salt thereof; or an average daily dose of 5 ± 1 mg, 7 ± 1.5 mg, 10 ± 2 mg, 15 ± 3 mg, 20 ± 4 mg, 25 ± 5 mg, 30 ± 6 mg, 40 ± 8 mg, 45 ± 9 mg, 55 ± 11 mg, 60 ± 12 mg, 65 ± 13 mg, 70 ± 14 mg, 75 ± 15 mg, 80 ± 16 mg, 90 ± 18 mg, 100 ± 20 mg, 120 ± 24 mg, 140 ± 28 mg, 160 ± 32 mg, 180 ± 36 mg, 200 ± 40 mg, 220 ± 44 mg, 240 ± 48 mg, or 260 ± 52 mg of compound 1, or a pharmaceutically acceptable salt thereof). In further aspects, the method includes inhibiting the proliferation of cancer cells in a subject by administering to the subject compound 1, or a pharmaceutically acceptable salt thereof, in an amount, dosing frequency, and for a period of time which produces a mean steady state trough concentration for compound 1 of from 40 to 600 nM; inhibiting angiogenesis in a subject by administering to the subject compound 1, or a pharmaceutically acceptable salt thereof, in an amount, dosing frequency, and for a period of time which produces a mean steady state trough concentration for compound 1 of from 40 to 600 nM; inhibiting angiogenesis in a subject in need thereof by orally administering daily to the subject from 30 to 300 mg of compound 1, or a pharmaceutically acceptable salt thereof; inhibiting the proliferation of BCR-ABL-expressing cells in a subject by administering to the subject compound 1, or a pharmaceutically acceptable salt thereof, in an amount, dosing frequency, and for a period of time which produces a mean steady state trough concentration for compound 1 of from 40 to 600 nM; inhibiting the proliferation of BCR-ABL-expressing cells while suppressing the emergence of resistant subclones by contacting the cells with compound 1, or a pharmaceutically acceptable salt thereof, in an amount sufficient to suppress the emergence of resistant subclones; inhibiting the proliferation of BCR-ABL-expressing cells while suppressing the emergence of compound mutants, the method including contacting the cells with compound 1, or a pharmaceutically acceptable salt thereof, in an amount sufficient to suppress the emergence of compound mutants; inhibiting the proliferation of BCR-ABL-expressing cells or a mutant thereof in a subject in need thereof by orally administering daily to the subject from 30 to 300 mg of compound 1, or a pharmaceutically acceptable salt thereof; or inhibiting the proliferation of mutant-expressing cells in a subject in need thereof by orally administering daily to said subject from 30 to 300 mg of compound 1, or a pharmaceutically acceptable salt thereof.

In any of the aspects described herein, the amount of compound 1 in a unit dosage form and the average daily dose can be modified for lower dosing (e.g., lower dosing for a child). In some embodiments, the unit dosage includes from 5 to 300 mg or the average daily dose is of 5 to 300 mg. In certain embodiments, the unit dosage form can contain from 5 to 100 mg, 5 to 80 mg, 5 to 50 mg, 5 to 20 mg, 7 to 100 mg, 7 to 80 mg, 7 to 50 mg, 7 to 20 mg, 10 to 100 mg, 10 to 80 mg, 10 to 50 mg, 15 to 100 mg, 15 to 80 mg, 15 to 60 mg, 15 mg to 50 mg, 20 to 100 mg, 20 to 80 mg, 20 to 50 mg, 30 to 100 mg, 35 to 100 mg, 40 to 100 mg, 50 to 100 mg, 60 to 100 mg, 70 to 100 mg, 30 to 80 mg, 35 to 80 mg, 40 to 80 mg, 50 to 80 mg, 60 to 80 mg, 50 to 300 mg, 60 to 300 mg, 70 to 300 mg, 50 to 200 mg, 60 to 200 mg, 70 to 200 mg, 100 to 300 mg, 120 to 300 mg, 140 to 300 mg, or 100 to 200 mg of compound 1, or a pharmaceutically acceptable salt thereof. Exemplary unit dosage forms include those having 5 ± 1 mg, 7 ± 1.5 mg, 10 ± 2 mg, 15 ± 3 mg, 20 ± 4 mg, 25 ± 5 mg, 30 ± 6 mg, 40 ± 8 mg, 45 ± 9 mg, 55 ± 11 mg, 60 ± 12 mg, 65 ± 13 mg, 70 ± 14 mg, 75 ± 15 mg, 80 ± 16 mg, 90 ± 18 mg, 100 ± 20 mg, 120 ± 24 mg, 140 ± 28 mg, 160 ± 32 mg, 180 ± 36 mg, 200 ± 40 mg, 220 ± 44 mg, 240 ± 48 mg, or 260 ± 52 mg of compound 1 or a pharmaceutically acceptable salt thereof.

In one aspect, the invention features a pharmaceutical composition formulated for oral administration in unit dosage form including from 30 to 300 mg of compound 1, or a pharmaceutically acceptable salt thereof. In certain embodiments, the unit dosage form can contain from 20 to 100 mg, 20 to 80 mg, 20 to 50 mg, 30 to 100 mg, 35 to 100 mg, 40 to 100 mg, 50 to 100 mg, 60 to 100 mg, 70 to 100 mg, 30 to 80 mg, 35 to 80 mg, 40 to 80 mg, 50 to 80 mg, 60 to 80 mg, 50 to 300 mg, 60 to 300 mg, 70 to 300 mg, 50 to 200 mg, 60 to 200 mg, 70 to 200 mg, 100 to 300 mg, 120 to 300 mg, 140 to 300 mg, or 100 to 200 mg of compound 1, or a pharmaceutically acceptable salt thereof. In particular embodiments the unit dosage form can contain 20 ± 4 mg, 25 ± 5 mg, 30 ± 6 mg, 40 ± 8 mg, 45 ± 9 mg, 55 ± 11 mg, 60 ± 12 mg, 65 ± 13 mg, 70 ± 14 mg, 75 ± 15 mg, 80 ± 16 mg, 90 ± 18 mg, 100 ± 20 mg, 120 ± 24 mg, 140 ± 28 mg, 160 ± 32 mg, 180 ± 36 mg, 200 ± 40 mg, 220 ± 44 mg, 240 ± 48 mg, or 260 ± 52 mg of compound 1, or a pharmaceutically acceptable salt thereof. The compound 1, or a pharmaceutically acceptable salt thereof, can be, for example, the hydrochloride salt.

In another aspect, the invention features a method of inhibiting the proliferation of cancer cells in a subject by administering to the subject compound 1, or a pharmaceutically acceptable salt thereof, in an amount, dosing frequency, and for a period of time which produces a mean steady state trough concentration for compound 1 of from 40 to 600 nM. In certain embodiments, the mean steady state trough concentration for compound 1 is from 40 to 200 nM, 50 to 200 nM, 60 to 200 nM, 70 to 200 nM, 80 to 200 nM, 90 to 200 nM, 40 to 120 nM, 50 to 120 nM, 60 to 120 nM, 70 to 120 nM, 80 to 120 nM, 200 to 600 nM, 220 to 600 nM, 240 to 600 nM, 250 to 600 nM, 270 to 600 nM, 280 to 600 nM, 200 to 400 nM, 200 to 300 nM, 250 to 400 nM, 300 to 500 nM, 350 to 550 nM, 400 to 600 nM, or 450 to 600 nM. Compound 1, or a pharmaceutically acceptable salt thereof, can be administered to the subject on average 4 to 7 times every 7 day period (e.g., 4 times a week, 5 times a week, 6 times a week, or 7 times a week). In certain embodiments, compound 1, or a pharmaceutically acceptable salt thereof, is administered to the subject daily. In particular embodiments, an average daily dose of from 30 to 300 mg of compound 1, or a pharmaceutically acceptable salt thereof, is orally administered to the subject in a unit dosage form (e.g., an average daily dose of from 20 to 100 mg, 20 to 80 mg, 20 to 50 mg, 30 to 100 mg, 35 to 100 mg, 40 to 100 mg, 50 to 100 mg, 60 to 100 mg, 70 to 100 mg, 30 to 80 mg, 35 to 80 mg, 40 to 80 mg, 50 to 80 mg, 60 to 80 mg, 50 to 300 mg, 60 to 300 mg, 70 to 300 mg, 50 to 200 mg, 60 to 200 mg, 70 to 200 mg, 100 to 300 mg, 120 to 300 mg, 140 to 300 mg, or 100 to 200 mg of compound 1, or a pharmaceutically acceptable salt thereof; or an average daily dose of 20 ± 4 mg, 25 ± 5 mg, 30 ± 6 mg, 40 ± 8 mg, 45 ± 9 mg, 55 ± 11 mg, 60 ± 12 mg, 65 ± 13 mg, 70 ± 14 mg, 75 ± 15 mg, 80 ± 16 mg, 90 ± 18 mg, 100 ± 20 mg, 120 ± 24 mg, 140 ± 28 mg, 160 ± 32 mg, 180 ± 36 mg, 200 ± 40 mg, 220 ± 44 mg, 240 ± 48 mg, or 260 ± 52 mg of compound 1, or a pharmaceutically acceptable salt thereof). In particular embodiments, the subject has gastric cancer, endometrial cancer, bladder cancer, multiple myeloma, breast cancer, or any other cancer described herein. In other embodiments, the subject has chronic myelogenous leukemia, acute lymphoblastic leukemia, or acute myelogenous leukemia. In yet other embodiments, the subject has a myelodysplastic syndrome (e.g., refractory anemia with excess of blasts group 1 (RAEBI) or refractory anemia with excess of blasts group 2 (RAEBII)).

In another aspect, the invention features a method of inhibiting the proliferation of cancer cells in a subject in need thereof by orally administering daily to the subject from 30 to 300 mg of compound 1, or a pharmaceutically acceptable salt thereof. In certain embodiments, from 20 to 100 mg, 20 to 80 mg, 20 to 50 mg, 30 to 100 mg, 35 to 100 mg, 40 to 100 mg, 50 to 100 mg, 60 to 100 mg, 70 to 100 mg, 30 to 80 mg, 35 to 80 mg, 40 to 80 mg, 50 to 80 mg, 60 to 80 mg, 50 to 300 mg, 60 to 300 mg, 70 to 300 mg, 50 to 200 mg, 60 to 200 mg, 70 to 200 mg, 100 to 300 mg, 120 to 300 mg, 140 to 300 mg, or 100 to 200 mg of compound 1, or a pharmaceutically acceptable salt thereof, is administered orally to the subject each day. In particular embodiments 20 ± 4 mg, 25 ± 5 mg, 30 ± 6 mg, 40 ± 8 mg, 45 ± 9 mg, 55 ± 11 mg, 60 ± 12 mg, 65 ± 13 mg, 70 ± 14 mg, 75 ± 15 mg, 80 ± 16 mg, 90 ± 18 mg, 100 ± 20 mg, 120 ± 24 mg, 140 ± 28 mg, 160 ± 32 mg, 180 ± 36 mg, 200 ± 40 mg, 220 ± 44 mg, 240 ± 48 mg, or 260 ± 52 mg of compound 1, or a pharmaceutically acceptable salt thereof is administered orally to the subject each day. In particular embodiments the subject has gastric cancer, endometrial cancer, bladder cancer, multiple myeloma, breast cancer, or any other cancer described herein. In other embodiments, the subject has chronic myelogenous leukemia, acute lymphoblastic leukemia, or acute myelogenous leukemia. In yet other embodiments, the subject has a myelodysplastic syndrome (e.g., refractory anemia with excess of blasts group 1 (RAEBI) or refractory anemia with excess of blasts group 2 (RAEBII)).

In another aspect, the invention features a method of inhibiting angiogenesis in a subject by administering to the subject compound 1, or a pharmaceutically acceptable salt thereof, in an amount, dosing frequency, and for a period of time which produces a mean steady state trough concentration for compound 1 of from 40 to 600 nM. In certain embodiments, the mean steady state trough concentration for compound 1 is from 40 to 200 nM, 50 to 200 nM, 60 to 200 nM, 70 to 200 nM, 80 to 200 nM, 90 to 200 nM, 40 to 120 nM, 50 to 120 nM, 60 to 120 nM, 70 to 120 nM, 80 to 120 nM, 200 to 600 nM, 220 to 600 nM, 240 to 600 nM, 250 to 600 nM, 270 to 600 nM, 280 to 600 nM, 200 to 400 nM, 200 to 300 nM, 250 to 400 nM, 300 to 500 nM, 350 to 550 nM, 400 to 600 nM, or 450 to 600 nM. Compound 1, or a pharmaceutically acceptable salt thereof, can be administered to the subject on average 4 to 7 times every 7 day period (e.g., 4 times a week, 5 times a week, 6 times a week, or 7 times a week). In certain embodiments, compound 1, or a pharmaceutically acceptable salt thereof, is administered to the subject daily. In particular embodiments, an average daily dose of from 30 to 300 mg of compound 1, or a pharmaceutically acceptable salt thereof, is orally administered to the subject in a unit dosage form (e.g., an average daily dose of from 20 to 100 mg, 20 to 80 mg, 20 to 50 mg, 30 to 100 mg, 35 to 100 mg, 40 to 100 mg, 50 to 100 mg, 60 to 100 mg, 70 to 100 mg, 30 to 80 mg, 35 to 80 mg, 40 to 80 mg, 50 to 80 mg, 60 to 80 mg, 50 to 300 mg, 60 to 300 mg, 70 to 300 mg, 50 to 200 mg, 60 to 200 mg, 70 to 200 mg, 100 to 300 mg, 120 to 300 mg, 140 to 300 mg, or 100 to 200 mg of compound 1, or a pharmaceutically acceptable salt thereof; or an average daily dose of 20 ± 4 mg, 25 ± 5 mg, 30 ± 6 mg, 40 ± 8 mg, 45 ± 9 mg, 55 ± 11 mg, 60 ± 12 mg, 65 ± 13 mg, 70 ± 14 mg, 75 ± 15 mg, 80 ± 16 mg, 90 ± 18 mg, 100 ± 20 mg, 120 ± 24 mg, 140 ± 28 mg, 160 ± 32 mg, 180 ± 36 mg, 200 ± 40 mg, 220 ± 44 mg, 240 ± 48 mg, or 260 ± 52 mg of compound 1, or a pharmaceutically acceptable salt thereof). In particular embodiments the subject has prostate cancer, lung cancer, breast cancer, colorectal cancer, renal cancer, or glioblastoma. In still other embodiments, the subject has a solid cancer that is refractory to treatment with a VEGF or VEGF-R inhibitor or antagonist (e.g., bevacizumab, sorafenib, or sunitinib). In yet other embodiments, the subject has a solid cancer that is intolerant to treatment with a VEGF or VEGF-R inhibitor or antagonist (e.g., bevacizumab, sorafenib, or sunitinib). In certain embodiments, the subject has a condition associated with aberrant angiogenesis, such as diabetic retinopathy, rheumatoid arthritis, psoriasis, atherosclerosis, chronic inflammation, obesity, macular degeneration, or a cardiovascular disease.

In another aspect, the invention also features a method of inhibiting angiogenesis in a subject in need thereof by orally administering daily to the subject from 30 to 300 mg of compound 1, or a pharmaceutically acceptable salt thereof. In certain embodiments, from 20 to 100 mg, 20 to 80 mg, 20 to 50 mg, 30 to 100 mg, 35 to 100 mg, 40 to 100 mg, 50 to 100 mg, 60 to 100 mg, 70 to 100 mg, 30 to 80 mg, 35 to 80 mg, 40 to 80 mg, 50 to 80 mg, 60 to 80 mg, 50 to 300 mg, 60 to 300 mg, 70 to 300 mg, 50 to 200 mg, 60 to 200 mg, 70 to 200 mg, 100 to 300 mg, 120 to 300 mg, 140 to 300 mg, or 100 to 200 mg of compound 1, or a pharmaceutically acceptable salt thereof, is administered orally to the subject each day. In particular embodiments 20 ± 4 mg, 25 ± 5 mg, 30 ± 6 mg, 40 ± 8 mg, 45 ± 9 mg, 55 ± 11 mg, 60 ± 12 mg, 65 ± 13 mg, 70 ± 14 mg, 75 ± 15 mg, 80 ± 16 mg, 90 ± 18 mg, 100 ± 20 mg, 120 ± 24 mg, 140 ± 28 mg, 160 ± 32 mg, 180 ± 36 mg, 200 ± 40 mg, 220 ± 44 mg, 240 ± 48 mg, or 260 ± 52 mg of compound 1, or a pharmaceutically acceptable salt thereof is administered orally to the subject each day. In particular embodiments the subject has prostate cancer, lung cancer, breast cancer, colorectal cancer, renal cancer, or glioblastoma. In still other embodiments, the subject has a solid cancer that is refractory to treatment with a VEGF or VEGF-R inhibitor or antagonist (e.g., bevacizumab, sorafenib, or sunitinib). In yet other embodiments, the subject has a solid cancer that is intolerant to treatment with a VEGF or VEGF-R inhibitor or antagonist (e.g., bevacizumab, sorafenib, or sunitinib). In certain embodiments, the subject has a condition associated with aberrant angiogenesis, such as diabetic retinopathy, rheumatoid arthritis, psoriasis, atherosclerosis, chronic inflammation, obesity, macular degeneration, or a cardiovascular disease.

In another aspect, the invention features a kit including (i) a pharmaceutical composition formulated for oral administration in unit dosage form including from 30 to 300 mg of compound 1, or a pharmaceutically acceptable salt thereof, and (ii) instruction for administering the pharmaceutical composition to a subject for the treatment of cancer or for the treatment of a condition associated with aberrant angiogenesis. In certain embodiments, the unit dosage form can contain from 20 to 100 mg, 20 to 80 mg, 20 to 50 mg, 30 to 100 mg, 35 to 100 mg, 40 to 100 mg, 50 to 100 mg, 60 to 100 mg, 70 to 100 mg, 30 to 80 mg, 35 to 80 mg, 40 to 80 mg, 50 to 80 mg, 60 to 80 mg, 50 to 300 mg, 60 to 300 mg, 70 to 300 mg, 50 to 200 mg, 60 to 200 mg, 70 to 200 mg, 100 to 300 mg, 120 to 300 mg, 140 to 300 mg, or 100 to 200 mg of compound 1, or a pharmaceutically acceptable salt thereof. In particular embodiments the unit dosage form can contain 20 ± 4 mg, 25 ± 5 mg, 30 ± 6 mg, 40 ± 9 mg, 45 ± 9 mg, 55 ± 11 mg, 60 ± 12 mg, 65 ± 13 mg, 70 ± 14 mg, 75 ± 15 mg, 80 ± 16 mg, 90 ± 18 mg, 100 ± 20 mg, 120 ± 24 mg, 140 ± 28 mg, 160 ± 32 mg, 180 ± 36 mg, 200 ± 40 mg, 220 ± 44 mg, 240 ± 48 mg, or 260 ± 52 mg of compound 1, or a pharmaceutically acceptable salt thereof. In particular embodiments the subject has gastric cancer, endometrial cancer, bladder cancer, multiple myeloma, breast cancer, chronic myelogenous leukemia, acute lymphoblastic leukemia, acute myelogenous leukemia, o a a myelodysplastic syndrome (e.g., refractory anemia with excess of blasts group 1 (RAEBI) or refractory anemia with excess of blasts group 2 (RAEBII)), or any other cancer described herein. In certain embodiments, the subject has diabetic retinopathy, rheumatoid arthritis, psoriasis, atherosclerosis, chronic inflammation, obesity, macular degeneration, or a cardiovascular disease.

In another aspect, the invention features a method of inhibiting the proliferation of BCR-ABI-expressing cells in a subject by administering to the subject compound 1, or a pharmaceutically acceptable salt thereof, in an amount, dosing frequency, and for a period of time which produces a mean steady state trough concentration for compound 1 of from 40 to 600 nM. In certain embodiments, the mean steady state trough concentration for compound 1 is from 40 to 200 nM, 50 to 200 nM, 60 to 200 nM, 70 to 200 nM, 80 to 200 nM, 90 to 200 nM, 40 to 120 nM, 50 to 120 nM, 60 to 120 nM, 70 to 120 nM, 80 to 120 nM, 200 to 600 nM, 220 to 600 nM, 240 to 600 nM, 250 to 600 nM, 270 to 600 nM, 280 to 600 nM, 200 to 400 nM, 200 to 300 nM, 250 to 400 nM, 300 to 500 nM, 350 to 550 nM, 400 to 600 nM, or 450 to 600 nM. Compound 1, or a pharmaceutically acceptable salt thereof, can be administered in an amount sufficient to suppress the emergence of resistant subclones or administered in an amount sufficient to suppress the emergence of compound mutants. Compound 1, or a pharmaceutically acceptable salt thereof, can be administered to the subject on average 4 to 7 times every 7 day period (e.g., 4 times a week, 5 times a week, 6 times a week, or 7 times a week), and for a period including 2 weeks, 1 month, 2 months, 4 months, 8 months, 1 year, or 18 months of uninterrupted therapy. In certain embodiments, compound 1, or a pharmaceutically acceptable salt thereof, is administered to the subject daily. In particular embodiments, an average daily dose of from 30 to 300 mg of compound 1, or a pharmaceutically acceptable salt thereof, is orally administered to the subject in a unit dosage form (e.g., an average daily dose of from 20 to 100 mg, 20 to 80 mg, 20 to 50 mg, 30 to 100 mg, 35 to 100 mg, 40 to 100 mg, 50 to 100 mg, 60 to 100 mg, 70 to 100 mg, 30 to 80 mg, 35 to 80 mg, 40 to 80 mg, 50 to 80 mg, 60 to 80 mg, 50 to 300 mg, 60 to 300 mg, 70 to 300 mg, 50 to 200 mg, 60 to 200 mg, 70 to 200 mg, 100 to 300 mg, 120 to 300 mg, 140 to 300 mg, or 100 to 200 mg of compound 1, or a pharmaceutically acceptable salt thereof; or an average daily dose of 20 ± 4 mg, 25 ± 5 mg, 30 ± 6 mg, 40 ± 8 mg, 45 ± 9 mg, 55 ± 11 mg, 60 ± 12 mg, 65 ± 13 mg, 70 ± 14 mg, 75 ± 15 mg, 80 ± 16 mg, 90 ± 18 mg, 100 ± 20 mg, 120 ± 24 mg, 140 ± 28 mg, 160 ± 32 mg, 180 ± 36 mg, 200 ± 40 mg, 220 ± 44 mg, 240 ± 48 mg, or 260 ± 52 mg of compound 1, or a pharmaceutically acceptable salt thereof). In particular embodiments the subject has a condition selected from chronic myelogenous leukemia, acute lymphoblastic leukemia, or acute myelogenous leukemia. In still other embodiments, the condition is refractory to treatment with a kinase inhibitor other than compound 1 (e.g., a condition is refractory to treatment with imatinib, nilotinib, or dasatinib). In yet other embodiments, the subject has a solid cancer that is intolerant to treatment with a VEGF or VEGFR inhibitor or antagonist (e.g., bevacizumab, sorafenib, or sunitinib).

In another aspect, the invention features a method of inhibiting the proliferation of BCR-ABL-expressing cells while suppressing the emergence of resistant subclones by contacting the cells with compound 1, or a pharmaceutically acceptable salt thereof, in an amount sufficient to suppress the emergence of resistant subclones. The cells can be contacted with from 20 nM to 320 nM, 30 nM to 320 nM, 20 nM to 220 nM, 30 nM to 220 nM, 20 nM to 120 nM, 30 nM to 120 nM, 40 nM to 320 nM, 40 nM to 220 nM, 40 nM to 120 nM, 50 nM to 320 nM, 50 nM to 220 nM, 50 nM to 120 nM, 70 nM to 320 nM, 70 nM to 220 nM, 90 nM to 320 nM, 90 nM to 220 nM, 110 nM to 320 nM, or 110 nM to 220 nM of compound 1, or a pharmaceutically acceptable salt thereof. The cells can be contacted with compound 1, or a pharmaceutically acceptable salt thereof, for a period including 2 weeks, 1 month, 2 months, 4 months, 8 months, 1 year, or 18 months of uninterrupted exposure.

In another aspect, the invention further features a method of inhibiting the proliferation of BCR-ABL-expressing cells while suppressing the emergence of compound mutants, the method including contacting the cells with compound 1, or a pharmaceutically acceptable salt thereof, in an amount sufficient to suppress the emergence of compound mutants. The cells can be contacted with from 160 nM to 1 µM, 260 nM to 1 µM, 360 nM to 1 µM, 160 nM to 800 nM, 260 nM to 800 nM, 360 nM to 800 nM, 160 nM to 600 nM, 260 nM to 600 nM, 360 nM to 600 nM, 160 nM to 400 nM, 260 nM to 400 nM, 360 nM to 500 nM, or 460 nM to 600 nM of compound 1, or a pharmaceutically acceptable salt thereof. The cells can be contacted with compound 1, or a pharmaceutically acceptable salt thereof, for a period including 2 weeks, 1 month, 2 months, 4 months, 8 months, 1 year, or 18 months of uninterrupted exposure.

In any of the above methods, the cells can be refractory to treatment with a kinase inhibitor other than compound 1 (e.g., refractory to treatment with imatinib, nilotinib, or dasatinib). In any of the above methods, the cells can be intolerant to treatment with a VEGF or VEGF-R inhibitor or antagonist (e.g., bevacizumab, sorafenib, or sunitinib).

In another aspect, the invention also features a method of inhibiting the proliferation of BCR-ABL-expressing cells or a mutant thereof in a subject in need thereof by orally administering daily to the subject from 30 to 300 mg of compound 1, or a pharmaceutically acceptable salt thereof. In certain embodiments, from 20 to 100 mg, 20 to 80 mg, 20 to 50 mg, 30 to 100 mg, 35 to 100 mg, 40 to 100 mg, 50 to 100 mg, 60 to 100 mg, 70 to 100 mg, 30 to 80 mg, 35 to 80 mg, 40 to 80 mg, 50 to 80 mg, 60 to 80 mg, 50 to 300 mg, 60 to 300 mg, 70 to 300 mg, 50 to 200 mg, 60 to 200 mg, 70 to 200 mg, 100 to 300 mg, 120 to 300 mg, 140 to 300 mg, or 100 to 200 mg of compound 1, or a pharmaceutically acceptable salt thereof, is administered orally to the subject each day. In particular embodiments 20 ± 4 mg, 25 ± 5 mg, 30 ± 6 mg, 40 ± 8 mg, 45 ± 9 mg, 55 ± 11 mg, 60 ± 12 mg, 65 ± 13 mg, 70 ± 14 mg, 75 ± 15 mg, 80 ± 16 mg, 90 ± 18 mg, 100 ± 20 mg, 120 ± 24 mg, 140 ± 28 mg, 160 ± 32 mg, 180 ± 36 mg, 200 ± 40 mg, 220 ± 44 mg, 240 ± 48 mg, or 260 ± 52 mg of compound 1, or a pharmaceutically acceptable salt thereof is administered orally to the subject each day. Compound 1, or a pharmaceutically acceptable salt thereof, can be administered in an amount sufficient to suppress the emergence of resistant subclones or administered in an amount sufficient to suppress the emergence of compound mutants. In particular embodiments the subject has a condition selected from chronic myelogenous leukemia, acute lymphoblastic leukemia, or acute myelogenous leukemia. In still other embodiments, the condition is refractory to treatment with a kinase inhibitor other than compound 1 (e.g., a condition is refractory to treatment with imatinib, nilotinib, or dasatinib). In still other embodiments, the condition is intolerant to treatment with a kinase inhibitor other than compound 1 (e.g., a condition is intolerant to treatment with imatinib, nilotinib, or dasatinib). Compound 1, or a pharmaceutically acceptable salt thereof, can be administered to the subject on average 4 to 7 times every 7 day period (e.g., 4 times a week, 5 times a week, 6 times a week, or 7 times a week), and for a period including 2 weeks, 1 month, 2 months, 4 months, 8 months, 1 year, or 18 months of uninterrupted therapy. In certain embodiments, compound 1, or a pharmaceutically acceptable salt thereof, is administered to the subject daily.

In another aspect, the invention features a kit including (i) a pharmaceutical composition formulated for oral administration in unit dosage form including from 30 to 300 mg of compound 1, or a pharmaceutically acceptable salt thereof, and (ii) instruction for administering the pharmaceutical composition to a subject suffering from a condition associated with the proliferation of BCR-ABL-expressing cells. In certain embodiments, the unit dosage form can contain from 20 to 100 mg, 20 to 80 mg, 20 to 50 mg, 30 to 100 mg, 35 to 100 mg, 40 to 100 mg, 50 to 100 mg, 60 to 100 mg, 70 to 100 mg, 30 to 80 mg, 35 to 80 mg, 40 to 80 mg, 50 to 80 mg, 60 to 80 mg, 50 to 300 mg, 60 to 300 mg, 70 to 300 mg, 50 to 200 mg, 60 to 200 mg, 70 to 200 mg, 100 to 300 mg, 120 to 300 mg, 140 to 300 mg, or 100 to 200 mg of compound 1, or a pharmaceutically acceptable salt thereof. In particular embodiments the unit dosage form can contain 20 ± 4 mg, 25 ± 5 mg, 30 ± 6 mg, 40 ± 8 mg, 45 ± 9 mg, 55 ± 11 mg, 60 ± 12 mg, 65 ± 13 mg, 70 ± 14 mg, 75 ± 15 mg, 80 ± 16 mg, 90 ± 18 mg, 100 ± 20 mg, 124 ± 24 mg, 140 ± 28 mg, 160 ± 32 mg, 180 ± 36 mg, 200 ± 40 mg, 220 ± 44 mg, 240 ± 48 mg, or 260 ± 52 mg of compound 1, or a pharmaceutically acceptable salt thereof. The compound 1, or a pharmaceutically acceptable salt thereof, can be, for example, the hydrochloride salt. In particular embodiments the subject has a condition selected from chronic myelogenous leukemia, acute lymphoblastic leukemia, or acute myelogenous leukemia. In still other embodiments, the condition is refractory to treatment with a kinase inhibitor other than compound 1 (e.g., a condition is refractory to treatment with imatinib, nilotinib, or dasatinib). In still other embodiments, the condition is intolerant to treatment with a kinase inhibitor other than compound 1 (e.g., a condition is intolerant to treatment with imatinib, nilotinib, or dasatinib).

In another aspect, the invention features a method of inhibiting the proliferation of mutant-expressing cells in a subject in need thereof by orally administering daily to said subject from 30 to 300 mg of compound 1, or a pharmaceutically acceptable salt thereof. In certain embodiments, from 20 to 100 mg, 20 to 80 mg, 20 to 50 mg, 30 to 100 mg, 35 to 100 mg, 40 to 100 mg, 50 to 100 mg, 60 to 100 mg, 70 to 100 mg, 30 to 80 mg, 35 to 80 mg, 40 to 80 mg, 50 to 80 mg, 60 to 80 mg, 50 to 300 mg, 60 to 300 mg, 70 to 300 mg, 50 to 200 mg, 60 to 200 mg, 70 to 200 mg, 100 to 300 mg, 120 to 300 mg, 140 to 300 mg, or 100 to 200 mg of compound 1, or a pharmaceutically acceptable salt thereof, is administered orally to the subject each day. In particular embodiments, 20 ± 4 mg, 25 ± 5 mg, 30 ± 6 mg, 40 ± 8 mg, 45 ± 9 mg, 55 ± 11 mg, 60 ± 12 mg, 65 ± 13 mg, 70 ± 14 mg, 75 ± 15 mg, 80 ± 16 mg, 90 ± 18 mg, 100 ± 20 mg, 120 ± 24 mg, 140 ± 28 mg, 160 ± 32 mg, 180 ± 36 mg, 200 ± 40 mg, 220 ± 44 mg, 240 ± 48 mg, or 260 ± 52 mg of compound 1, or a pharmaceutically acceptable salt thereof is administered orally to the subject each day. In certain embodiments, the mutant is a FLT3 mutant (e.g., FLT3-ITD), a KIT mutant (e.g., c-KIT or N822K), a FGFR mutant (e.g., FGFR1OP2-FGFR1), a PDGFRα mutant (e.g., F1P1L1-PDGFRα), or any mutant described herein. In other embodiments, the subject has acute myelogenous leukemia or a myelodysplastic syndrome (e.g., refractory anemia with excess of blasts group 1 (RAEBI) or refractory anemia with excess of blasts group 2 (RAEBII)). In still other embodiments, the condition is refractory to treatment with a kinase inhibitor other than compound 1 (e.g., a condition is refractory to treatment with imatinib, nilotinib, or dasatinib). In still other embodiments, the condition is intolerant to treatment with a kinase inhibitor other than compound 1 (e.g., a condition is intolerant to treatment with imatinib, nilotinib, or dasatinib). Compound 1, or a pharmaceutically acceptable salt thereof, can be administered to the subject on average 4 to 7 times every 7 day period (e.g., 4 times a week, 5 times a week, 6 times a week, or 7 times a week), and for a period including 2 weeks, 1 month, 2 months, 4 months, 8 months, 1 year, or 18 months of uninterrupted therapy. In certain embodiments, compound 1, or a pharmaceutically acceptable salt thereof, is administered to the subject daily.

In one aspect, the invention features a method of treating a cancer in a subject in need thereof by administering to the subject compound 1, or a pharmaceutically acceptable salt thereof, together or concurrently with an mTOR inhibitor each in an amount that together is effective to treat the cancer. In another aspect, the invention also features a method of treating a neoplasm in a subject in need thereof by administering to the subject compound 1, or a pharmaceutically acceptable salt thereof, together or concurrently with an mTOR inhibitor each in an amount that together is effective to treat the neoplasm. In another aspect, the invention further features a method of inhibiting angiogenesis in a subject in need thereof by administering to the subject compound 1, or a pharmaceutically acceptable salt thereof, together or concurrently with an mTOR inhibitor each in an amount that together is effective to inhibit the angiogenesis. In another aspect, the invention features a method of inhibiting the proliferation of cells by contacting the cells with compound 1, or a pharmaceutically acceptable salt thereof, together or concurrently with an mTOR inhibitor each in an amount that together is sufficient to inhibit the proliferation.

In any of the above aspects, the mTOR inhibitor is a rapamycin macrolide selected from sirolimus, everolimus, temsirolimus, ridaforolimus, biolimus, zotarolimus, and pharmaceutically acceptable salts thereof. Desirably, the mTOR inhibitor is ridaforolimus or a pharmaceutically acceptable salt thereof In other embodiments, the mTOR inhibitor is a non-rapamycin analog selected from LY294002, Pp242, WYE-354, Ku-0063794, XL765, AZD8055, NVP-BEZ235, OSI-027, wortmannin, quercetin, myricentin, staurosporine, and pharmaceutically acceptable salts thereof.

The combination therapy can include a regimen in which compound 1, or a pharmaceutically acceptable salt thereof, and the mTOR inhibitor are administered concurrently within 12 days, 8 days, 5 days, 4 days, 3 days, or 2 days of each other; compound 1, or a pharmaceutically acceptable salt thereof, and the mTOR inhibitor are administered concurrently within 24 hours of each other; or compound 1, or a pharmaceutically acceptable salt thereof, and the mTOR inhibitor are administered together. Compound 1 and the mTOR inhibitor can be administered as a combination therapy of the invention using any regimen described herein.

In certain embodiments, compound 1, or a pharmaceutically acceptable salt thereof, is administered at a low dose; the mTOR inhibitor is administered at a low dose; or both compound 1 and the mTOR inhibitor are administered at a low dose.

In particular embodiments, the combination therapy includes administering to the subject compound 1, or a pharmaceutically acceptable salt thereof, in an amount, dosing frequency, and for a period of time which produces a mean steady state trough concentration for compound 1 of from 40 to 600 nM. For example, the mean steady state trough concentration for compound 1 can be from 10 to 100 nM, 10 to 60 nM, 15 to 100 nM, 15 to 70 nM, 20 to 100 nM, 40 to 200 nM, 50 to 200 nM, 60 to 200 nM, 70 to 200 nM, 80 to 200 nM, 90 to 200 nM, 40 to 120 nM, 50 to 120 nM, 60 to 120 nM, 70 to 120 nM, 80 to 120 nM, 200 to 600 nM, 220 to 600 nM, 240 to 600 nM, 250 to 600 nM, 270 to 600 nM, 280 to 600 nM, 200 to 400 nM, 200 to 300 nM, 250 to 400 nM, 300 to 500 nM, 350 to 550 nM, 400 to 600 nM, or 450 to 600 nM. Compound 1, or a pharmaceutically acceptable salt thereof, can be administered to the subject on average 4 to 7 times every 7 day period (e.g., 4 times a week, 5 times a week, 6 times a week, or 7 times a week). In certain embodiments, compound 1, or a pharmaceutically acceptable salt thereof, is administered to the subject daily. In particular embodiments, an average daily dose of from 30 to 300 mg of compound 1, or a pharmaceutically acceptable salt thereof, is orally administered to the subject in a unit dosage form (e.g., an average daily dose of from 10 to 70 mg, 10 to 50 mg, 10 to 30 mg, 20 to 100 mg, 20 to 80 mg, 20 to 50 mg, 30 to 100 mg, 35 to 100 mg, 40 to 100 mg, 50 to 100 mg, 60 to 100 mg, 70 to 100 mg, 30 to 80 mg, 35 to 80 mg, 40 to 80 mg, 50 to 80 mg, 60 to 80 mg, 50 to 300 mg, 60 to 300 mg, 70 to 300 mg, 50 to 200 mg, 60 to 200 mg, 70 to 200 mg, 100 to 300 mg, 120 to 300 mg, 140 to 300 mg, or 100 to 200 mg of compound 1, or a pharmaceutically acceptable salt thereof; or an average daily dose of 7 ± 1.5 mg, 10 ± 2 mg, 15 ± 3 mg, 20 ± 4 mg, 25 ± 5 mg, 30 ± 6 mg, 40 ± 8 mg, 45 ± 9 mg, 55 ± 11 mg, 60 ± 12 mg, 65 ± 13 mg, 70 ± 14 mg, 75 ± 15 mg, 80 ± 16 mg, 90 ± 18 mg, 100 ± 20 mg, 120 ± 24 mg, 140 ± 28 mg, 160 ± 32 mg, 180 ± 36 mg, 200 ± 40 mg, 220 ± 44 mg, 240 ± 48 mg, or 260 ± 52 mg of compound 1, or a pharmaceutically acceptable salt thereof).

The combination therapy of the invention can be used to treat a subject with a carcinoma of the bladder, breast, colon, kidney, liver, lung, head and neck, gall-bladder, ovary, pancreas, stomach, cervix, thyroid, prostate, or skin; squamous cell carcinoma; cndomctrial cancer; multiple myeloma; a hematopoietic tumor of lymphoid lineage (e.g., leukemia, acute lymphocytic leukemia, acute lymphoblastic leukemia, B-cell lymphoma, T-cell-lymphoma, Hodgkin's lymphoma, non-Hodgkin's lymphoma, hairy cell lymphoma, or Burkitt's lymphoma); a hematopoietic tumor of myelogenous lineage (e.g., acute myelogenous leukemia, chronic myelogenous leukemia, multiple myelogenous leukemia, myelodysplastic syndrome, or promyelocytic leukemia); a tumor of mesenchymal origin (e.g., fibrosarcoma or rhabdomyosarcoma); a tumor of the central or peripheral nervous system (e.g., astrocytoma, neuroblastoma, glioma, or schwannomas); melanoma; seminoma; teratocarcinoma; osteosarcoma; or Kaposi's sarcoma. In certain embodiments, the subject has non-small-cell lung cancer, breast cancer, ovarian cancer, bladder cancer, prostate cancer, salivary gland cancer, pancreatic cancer, endometrial cancer, colorectal cancer, kidney cancer, head and neck cancer, stomach cancer, multiple myeloma, thyroid follicular cancer, or glioblastoma multiforme.

The combination therapy of the invention can be used to treat a subject having a condition associated with aberrant angiogenesis. The condition associated with aberrant angiogenesis can be a solid tumor (e.g., prostate cancer, lung cancer, breast cancer, colorectal cancer, renal cancer, glioblastoma, or any solid tumor described herein), diabetic retinopathy, rheumatoid arthritis, psoriasis, atherosclerosis, chronic inflammation, obesity, macular degeneration, or a cardiovascular disease.

In a related aspect, the invention features a pharmaceutical composition including compound 1, or a pharmaceutically acceptable salt thereof, an mTOR inhibitor, and a pharmaceutically acceptable carrier or diluent. In certain embodiments, the mTOR inhibitor is a rapamycin macrolide selected from sirolimus, everolimus, temsirolimus, ridaforolimus, biolimus, zotarolimus, and pharmaceutically acceptable salts thereof. Desirably, the mTOR inhibitor is ridaforolimus or a pharmaceutically acceptable salt thereof. In other embodiments, the mTOR inhibitor is a non-rapamycin analog selected from LY294002, Pp242, WYE-354, Ku-0063794, XL765, AZD8055, NVP-BEZ235, OSI-027, wortmannin, quercetin, myricentin, staurosporine, and pharmaceutically acceptable salts thereof.

The invention further features a kit including (i) a first pharmaceutical composition formulated for oral administration in unit dosage form including from 30 to 300 mg of compound 1, or a pharmaceutically acceptable salt thereof, and (ii) a second pharmaceutical composition including an mTOR inhibitor, wherein the first pharmaceutical composition and the second pharmaceutical composition are formulated separately in individual dosage amounts.

The invention also features a kit including a pharmaceutical composition formulated for oral administration in unit dosage form including from 30 to 300 mg of compound 1, or a pharmaceutically acceptable salt thereof, and an mTOR inhibitor.

In certain embodiments of the above kits, the unit dosage form can contain from 10 to 70 mg, 10 to 50 mg, 10 to 30 mg, 20 to 100 mg, 20 to 80 mg, 20 to 50 mg, 30 to 100 mg, 35 to 100 mg, 40 to 100 mg, 50 to 100 mg, 60 to 100 mg, 70 to 100 mg, 30 to 80 mg, 35 to 80 mg, 40 to 80 mg, 50 to 80 mg, 60 to 80 mg, 50 to 300 mg, 60 to 300 mg, 70 to 300 mg, 50 to 200 mg, 60 to 200 mg, 70 to 200 mg, 100 to 300 mg, 120 to 300 mg, 140 to 300 mg, or 100 to 200 mg of compound 1, or a pharmaceutically acceptable salt thereof. In particular embodiments the unit dosage form can contain 7 ± 1.5 mg, 2 mg, 15 ± 3 mg, 20 ± 4 mg, 25 ± 5 mg, 30 ± 6 mg, 40 ± 8 mg, 45 ± 9 mg, 55 ± 11 mg, 60 ± 12 mg, 65 ± 13 mg, 70 ± 14 mg, 75 ± 15 mg, 80 ± 16 mg, 90 ± 18 mg, 100 ± 20 mg, 120 ± 24 mg, 140 ± 28 mg, 160 ± 32 mg, 180 ± 36 mg, 200 ± 40 mg, 220 ± 44 mg, 240 ± 48 mg, or 260 ± 52 mg of compound 1, or a pharmaceutically acceptable salt thereof.

In certain embodiments of the above kits, the mTOR inhibitor is a rapamycin macrolide selected from sirolimus, everolimus, temsirolimus, ridaforolimus, biolimus, zotarolimus, and pharmaceutically acceptable salts thereof. In other embodiments of the above kits, the mTOR inhibitor is a non-rapamycin analog selected from LY294002, Pp242, WYE-354, Ku-0063794, XL765, AZD8055, NVP-BEZ235, OSI-027, wortmannin, quercetin, myricentin, staurosporine, and pharmaceutically acceptable salts thereof.

The kits of the invention can further include instructions for administering compound 1, or a pharmaceutically acceptable salt thereof, and the mTOR inhibitor to a subject for the treatment of cancer (e.g., a subject that has carcinoma of the bladder, breast, colon, kidney, liver, lung, head and neck, gall-bladder, ovary, pancreas, stomach, cervix, thyroid, prostate, or skin; squamous cell carcinoma; endometrial cancer; multiple myeloma; a hematopoietic tumor of lymphoid lineage (e.g., leukemia, acute lymphocytic leukemia, acute lymphoblastic leukemia, B-cell lymphoma, T-cell-lymphoma, Hodgkin's lymphoma, non-Hodgkin's lymphoma, hairy cell lymphoma, or Burkitt's lymphoma); a hematopoietic tumor of myelogenous lineage (e.g., acute myelogenous leukemia, chronic myelogenous leukemia, multiple myelogenous leukemia, myelodysplastic syndrome, or promyelocytic leukemia); a tumor of mesenchymal origin (e.g., fibrosarcoma or rhabdomyosarcoma); a tumor of the central or peripheral nervous system (e.g., astrocytoma, neuroblastoma, glioma, or schwannomas); melanoma; seminoma; teratocarcinoma; osteosarcoma; or Kaposi's sarcoma) or a subject that has a condition associated with aberrant angiogenesis (e.g., a solid tumor, diabetic retinopathy, rheumatoid arthritis, psoriasis, atherosclerosis, chronic inflammation, obesity, or macular degeneration).

In the methods of the present invention, the dosage and frequency of administration of compound 1 and the mTOR inhibitor can be controlled independently. For example, one compound may be administered orally each day, while the second compound may be administered intravenously once per day. The compounds may also be formulated together such that one administration delivers both of the compounds.

The exemplary dosage of mTOR and compound 1 to be administered will depend on such variables as the type and extent of the disorder, the overall health status of the subject, the therapeutic index of the selected mTOR inhibitor, and their route of administration. Standard clinical trials maybe used to optimize the dose and dosing frequency for any particular combination of the invention.

Compounds useful in the present invention include those described herein in any of their pharmaceutically acceptable forms, including isomers, such as diastereomers and enantiomers, mixtures of isomers, and salts thereof.

### Definitions

As used herein, the term "BCR-ABL-expressing cells" refers cells expressing either native BCR-ABL, resistant subclones, or compound mutants of BCR-ABL.

As used herein, the term "mean steady state trough concentration" refers to the average plasma concentration of compound 1 observed for a group of subjects as part of a dosing regimen for a therapy of the invention administered over a period of time sufficient to produce steady state pharmacokinetics (i.e., a period of 23 days of daily dosing), wherein the mean trough concentration is the average circulating concentration over all of the subjects at a time just prior to (i.e., within 1 hour of) the next scheduled administration in the regimen (e.g., for a daily regimen the trough concentration is measured about 24 hours after an administration of compound 1 and just prior to the subsequent daily administration).

By "an amount sufficient to suppress the emergence of compound mutants" is meant an amount of compound 1 which measurably reduces the emergence of compound mutants in vitro or in vivo in comparison to the rate of emergence of compound mutants which occurs at the minimal concentration of compound 1 required to inhibit the proliferation of BCR-ABL-expressing cells.

By "an amount sufficient to suppress the emergence of resistant subclones" is meant an amount of compound 1 which measurably reduces the emergence of resistant subclones in vitro or in vivo in comparison to the rate of emergence of resistant subclones which occurs at the minimal concentration of compound 1 required to inhibit the proliferation of BCR-ABL-expressing cells.

By "inhibiting the proliferation of BCR-ABL-expressing cells" is meant measurably slows, stops, or reverses the growth rate of the BCR-ABL-expressing cells cells in vitro or in vivo. Desirably, a slowing of the growth rate is by at least 20%, 30%, 50%, or even 70%, as determined using a suitable assay for determination of cell growth rates (e.g., a cell growth assay described herein).

By "inhibiting the proliferation of cancer cells" is meant measurably slows, stops, or reverses the growth rate of the cancer cells in vitro or in vivo. Desirably, a slowing of the growth rate is by at least 20%, 30%, 50%, or even 70%, as determined using a suitable assay for determination of cell growth rates (e.g., a cell growth assay described herein).

By "inhibiting the proliferation of cells" is meant measurably slows, stops, or reverses the growth rate of the cells in vitro or in vivo. Desirably, a slowing of the growth rate is by at least 20%, 30%, 50%, or even 70%, as determined using a suitable assay for determination of cell growth rates (e.g., a cell growth assay described herein).

The term "administration" or "administering" refers to a method of giving a dosage of a pharmaceutical composition to a mammal, where the method is, e.g., oral, intravenous, intraperitoneal, intraarterial, or intramuscular. The preferred method of administration can vary depending on various factors, e.g., the components of the pharmaceutical composition, site of the potential or actual disease and severity of disease. While compound 1 will generally be administered per orally, other routes of administration can be useful in carrying out the methods of the invention.

The term "unit dosage form" refers to physically discrete units suitable as unitary dosages, such as a pill, tablet, caplet, hard capsule or soft capsule, each unit containing a predetermined quantity of compound 1.

As used herein, the term "pharmaceutically acceptable salt" refers to any pharmaceutically acceptable salt, such as a non-toxic acid addition salt or metal complex, commonly used in the pharmaceutical industry. Examples of acid addition salts include organic acids, such as acetic, lactic, pamoic, maleic, citric, malic, ascorbic, succinic, benzoic, palmitic, suberic, salicylic, tartaric, methanesulfonic, toluenesulfonic, or trifluoroacetic acids, and inorganic acids, such as hydrochloric acid, hydrobromic acid, sulfuric acid, and phosphoric acid.

As used herein, the term "treating" refers to administering a pharmaceutical composition for prophylactic and/or therapeutic purposes. To "prevent disease" refers to prophylactic treatment of a subject who is not yet ill, but who is susceptible to, or otherwise at risk of, a particular disease. To "treat disease" or use for "therapeutic treatment" refers to administering treatment to a subject already suffering from a disease to improve or stabilize the subject's condition. Thus, in the claims and embodiments, treating is the administration to a subject either for therapeutic or prophylactic purposes.

By administration of mTOR inhibitor and compound 1 "concurrently" is meant that the mTOR inhibitor and compound 1 are formulated separately and administered separately within 2, 3, 4, 5, 6, or 7 days of each other.

By administration of mTOR inhibitor and compound 1 "together" is meant that the mTOR inhibitor and compound 1 are formulated together in a single pharmaceutical composition and administered together.

As used herein "an amount effective to treat" a neoplasm, cancer, or hyperproliferative disorder refers to an amount of compound 1 that slows the growth, slows the spreading of cells from a site of origin to other parts of the body, or relieves symptoms caused by the neoplasm, cancer, or hyperproliferative disorder. The symptoms relieved when a neoplasm, cancer, or hyperproliferative disorder responds to the therapies described herein include pain, and other types of discomfort.

When referring to combination therapy, as used herein "an amount effective to treat" a neoplasm or cancer refers to an amount of compound 1 and an mTOR inhibitor that together slows the growth, slows the spreading of cells from a site of origin to other parts of the body, or relieves symptoms caused by the neoplasm or cancer. The symptoms relieved when a neoplasm or cancer responds to the combination therapies described herein include pain, and other types of discomfort.

The terms "subject" and "patient" are used herein interchangeably. They refer to a human or another mammal (e.g., mouse, rat, rabbit, dog, cat, cattle, swine, sheep, horse or primate) that can be afflicted with or is susceptible to a disease or disorder (e.g., cancer, a neoplasm, or aberrant angiogenesis) but may or may not have the disease or disorder. In certain embodiments, the subject is a human being.

The term "cancer" refers to the physiological condition in mammals that is typically characterized by unregulated cell growth. Examples include, but are not limited to, carcinoma, lymphoma, blastoma, sarcoma, and leukemia. More particularly, examples of such cancers include squamous cell carcinoma, small-cell lung cancer, non-small cell lung cancer, pancreatic cancer, glioblastoma multiforme, esophageal/oral cancer, cervical cancer, ovarian cancer, endometrial cancer, prostate cancer, bladder cancer, hepatoma, breast cancer, colon or colorectal cancer, head and neck cancer, gastric cancer, multiple myeloma, renal cancer, chronic myelogenous leukemia, acute lymphoblastic leukemia, acute myelogenous leukemia, a myelodysplastic syndrome, and any other cancer described herein.

The term "neoplasm" refers to the physiological condition in mammals that is typically characterized by abnormal cellular proliferation. Non-limiting examples of neoplasms include any tumor described herein, such as solid tumors. More particularly, examples of neoplasms include solid tumors from gastric or gastrointestinal cancer, endometrial cancer, bladder cancer, multiple myeloma, breast cancer, prostate cancer, lung cancer, colorectal cancer, renal cancer, and glioblastoma multiforme.

The term "hyperproliferative disorder" refers to disorders associated with pathological cellular proliferation or pathological angiogenesis. Non-limiting examples of conditions associated with aberrant angiogenesis include solid tumors, diabetic retinopathy, rheumatoid arthritis, psoriasis, atherosclerosis, chronic inflammation, obesity, macular degeneration, and a cardiovascular disease.

By "low dose" is meant a dose that is less than a dose of an agent that would typically be given to a subject in a monotherapy for treatment of a neoplasm, cancer, or a condition associated with aberrant angiogenesis (e.g., less than 70%, 60%, 50%, 40%, or 30% of the amount administered as a monotherapy). The combinations of the invention can be used to reduce the dosage of the individual components of the combination therapy substantially to a point significantly below the dosages which would be required to achieve the same effects by administering an mTOR inhibitor or compound 1 alone as a monotherapy. Exemplary low doses of compound 1 and mTOR inhibitors are as follows: compound 1 at 7-42 mg orally daily (e.g., 7 ± 1.5 mg, 2 mg, 15 ± 3 mg, 20 ± 4 mg, 25 ± 5 mg, 30 ± 6 mg, or 3 5 ± 7 mg orally daily); ridaforolimus at 7-28 mg orally qdx5/week (e.g., 7 ± 1.5 mg, 2 mg, 15 ± 3 mg, 20 ± 4 mg, or 25 ± 3 mg orally qdx5/week); everolimus at 2-7 mg orally daily (e.g., 2 ± 0.4 mg, 3 ± 0.6 mg, 4 ± 0.8 mg, 5 ± 0.9 mg, or 6 ± 1.2 mg orally daily); temsirolimus 3-21 mg i.v. infusion weekly (e.g., 3 ± 0.6 mg, 5 ± 1 mg, 7.5 ± 1.5 mg, 2 mg, 15 ± 3 mg, or 18 ± 3.5 mg i.v. infusion weekly); sirolimus at 0.5-12 mg orally daily (e.g., 0.5 ± 0.1 mg, 1 ± 0.2 mg, 2 ± 0.4 mg, 3 ± 0.6 mg, 4 ± 0.8 mg, 5 ± 0.9 mg, 6 ± 1.2 mg, 8 ± 1.5 mg, or 10 ± 2 mg orally daily); biolimus at 100-600 µg i.v. infusion daily (e.g., 100 ± 20 µg, 150 ± 30 µg, 200 ± 40 µg, 300 ± 50 µg, 400 ± 50 µg, or 500 ± 50 µg i.v. infusion daily); zotarolimus at 100-600 µg i.v. infusion daily (e.g., 100 ± 20 µg, 150 ± 30 µg, 200 ± 40 µg, 300 ± 50 µg, 400 ± 50 µg, or 500 ± 50 µg i.v. infusion daily); NVP-BEZ235 at 5-50 mg orally daily (e.g., 5 ± 1 mg, 1.5 mg, 15 ± 3 mg, 20 ± 4 mg, 25 ± 5 mg, 30 ± 6 mg, 35 ± 7 mg, 40 ± 8 mg, 45 ± 9 mg, or 50 ± 10 mg orally daily); wortmannin at 10-70 mg orally daily (e.g., 2 mg, 15 ± 5 mg, 20 ± 6 mg, 30 ± 7 mg, 40 ± 8 mg, 50 ± 9 mg, 70 ± 10 mg orally daily); quercetin at 1-5 g orally daily (e.g., 1 ± 0.1 mg, 2 ± 0.2 mg, 3 ± 0.3 mg, 4 ± 0.5 mg, or 5 ± 1 mg orally daily); myricentin at 15-100 mg orally daily (e.g., 15 ± 5 mg, 20 ± 6 mg, 30 ± 7 mg, 40 ± 8 mg, 50 ± 9 mg, 75 ± 10 mg, or 100 ± 25 mg orally daily); and staurosporine at 10-50 mg orally daily (e.g., 10 ± 1.5 mg, 15 ± 3 mg, 20 ± 4 mg, 25 ± 5 mg, 30 ± 6 mg, 35 ± 7 mg, 40 ± 8 mg, 45 ± 9 mg, or 50 ± 10 mg orally daily). The following compounds can be administered in doses that are lower than those currently described for a monotherapy: LY294002, Pp242, WYE-354, Ku-0063794, XL765, AZD8055, and OSI-027.

Other features and advantages of the invention will be apparent from the following detailed description, the drawings, and the claims.

### Brief Description of the Drawings

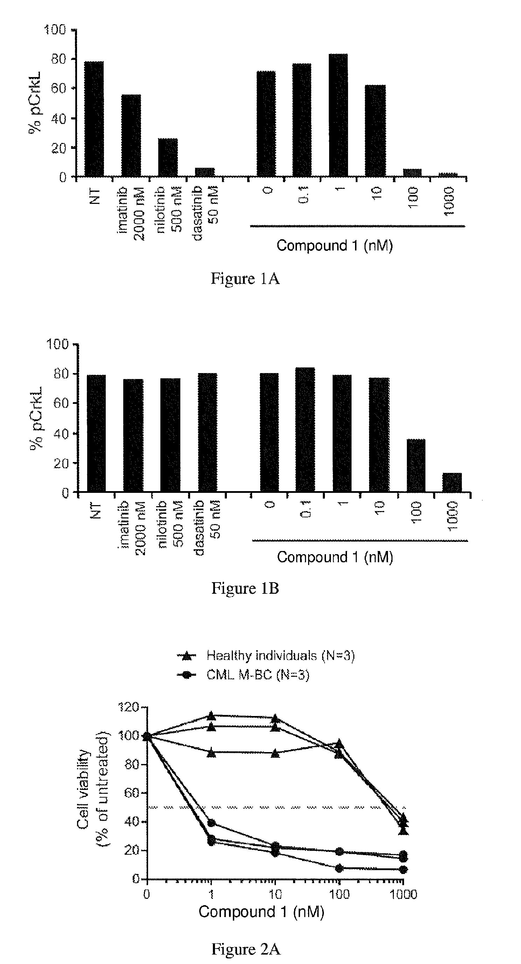

Figures 1A and 1B are graphs demonstrating that compound 1 inhibits BCR-ABL signaling in CML cell lines expressing native BCR-ABL or BCR-ABL^{T315I}. Figure 1A depicts an immunoblot analysis of CrkL phosphorylation in Ba/F3 cells expressing native BCR-ABL treated with imatinib, nilotinib, dasatinib, or compound 1. Cells were cultured for 4 hours in the presence of inhibitors, harvested, lysed, and analyzed by immunoblot using an antibody for CrkL, a substrate of BCR-ABL whose phosphorylation is an established clinical marker of BGR-ABL kinase activity. Both the phosphorylated and non-phosphorylated forms are resolved by electrophoretic mobility, and bands are quantitated by densitometry and expressed as a % phosphorylated CrkL. Figure 1B depicts an immunoblot analysis of CrkL phosphorylation in Ba/F3 BCR-ABL^{T315I}-expressing cells treated with imatinib, nilotinib, dasatinib, or compound 1. Assays and analysis were carried out as described above in panel (A). Abbreviations: NT, no treatment. Figures 1A and 1B demonstrate that compound 1 inhibits BCR-ABL signaling in CML cell lines expressing native BCR-ABL or BCR-ABL^{T315I}.

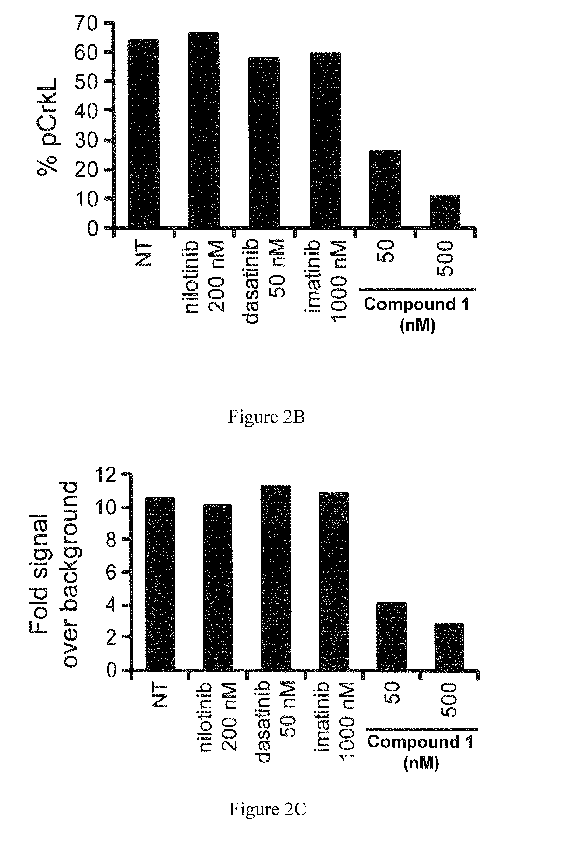

Figures 2A-C demonstrate that ex vivo treatment of CML primary cells with compound 1 inhibits cellular proliferation and BCR-ABL-mediated signaling. Figure 2A is a plot of cellular proliferation assays for ex vivo compound 1-treated mononuclear cells from CML myelogenous blast crisis (M-BC) patients harboring native BCR-ABL (N=3) and from healthy individuals (N=3). For reference, the dashed line indicates 50% cell viability relative to untreated cells. Figure 2B is a graph depicting the immunoblot analysis of CrkL phosphorylation in mononuclear cells from a CML lymphoid blast crisis (L-BC) patient harboring BCR-ABL^{T315I} following ex vivo exposure to compound 1, imatinib, nilotinib, or dasatinib. Cells were cultured for overnight in the presence of inhibitors, harvested, lysed, and analyzed by CrkL immunoblot. Both the phosphorylated and non-phosphorylated forms were resolved by electrophoretic mobility, and bands were quantitated by densitometry and expressed as a % phosphorylated CrkL. Figure 2C is a graph depicting FACS analysis of global tyrosine phosphorylation in mononuclear cells from the CML L-BC BCR-ABL^{T315I} patient in Figure 2B. After overnight culture in the presence of inhibitors, cells were fixed and permeabilized, incubated with a FITC-labeled antibody for phosphorylated tyrosine, and analyzed by FACS. Values reported are as fold increase in mean fluorescence intensity relative to unstained controls. Abbreviations: NT, no treatment.

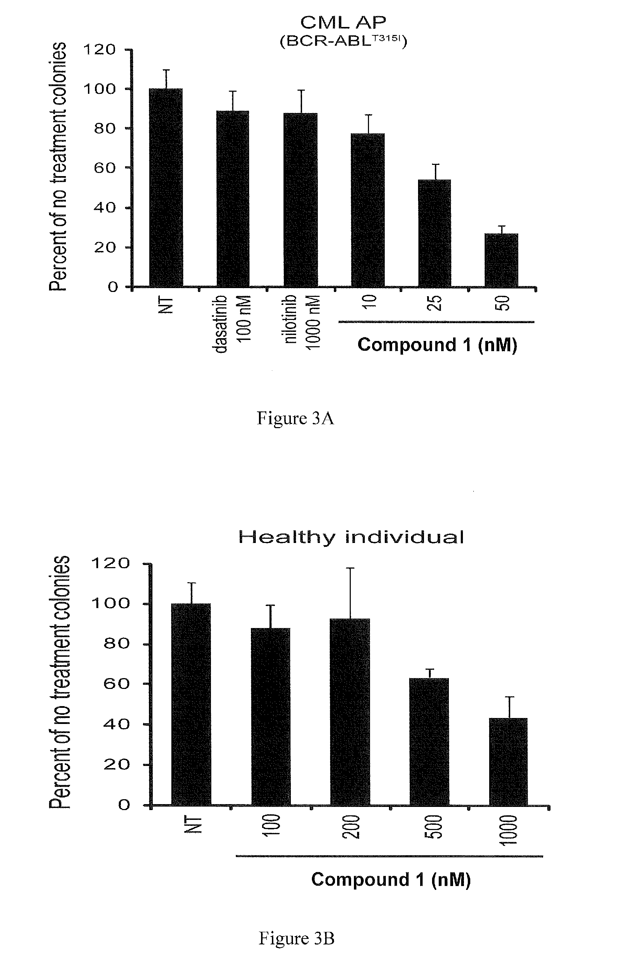

Figures 3A and 3B are graphs of colony formation assays for against compound 1. Figure 3A is a graph of colony formation assays in the presence of compound 1, nilotinib, and dasatinib using mononuclear cells from a CML AP patient harboring BCR-ABL^{T315I}. Figure 3B is a graph of colony formation assays in the presence of compound 1 using mononuclear cells from a healthy individual. Mononuclear cells from a CML accelerated phase (AP) patient harboring BCR-ABL^{T315I} and from a healthy individual were plated in methylcellulose containing nilotinib, dasatinib, or compound 1 and cultured for 14-18 days. Colonies were counted under an inverted microscope, and results were expressed as the mean of three replicates (error bars represent S.E.M.).

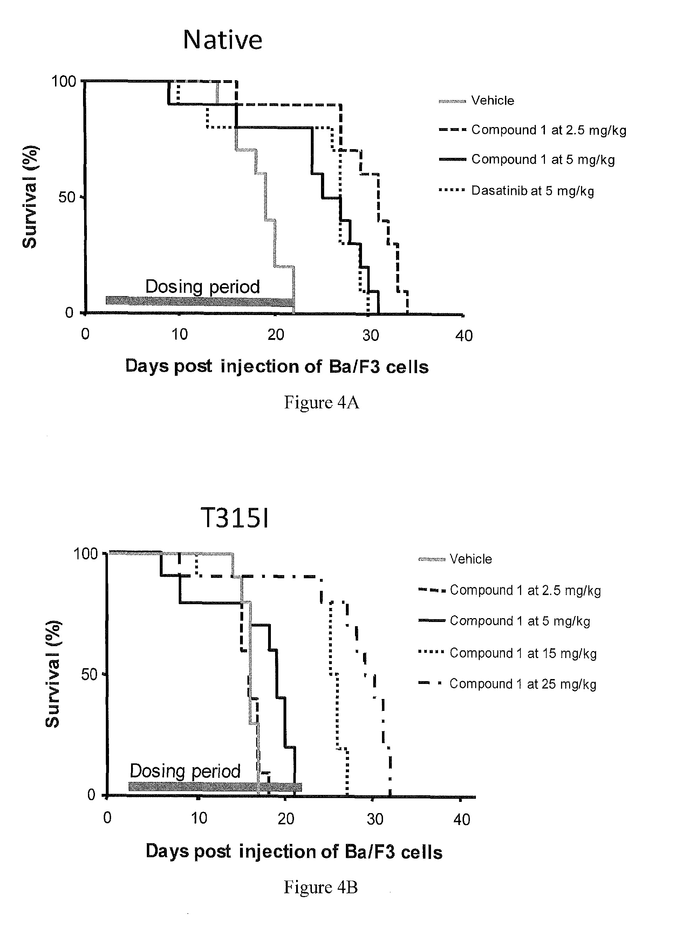

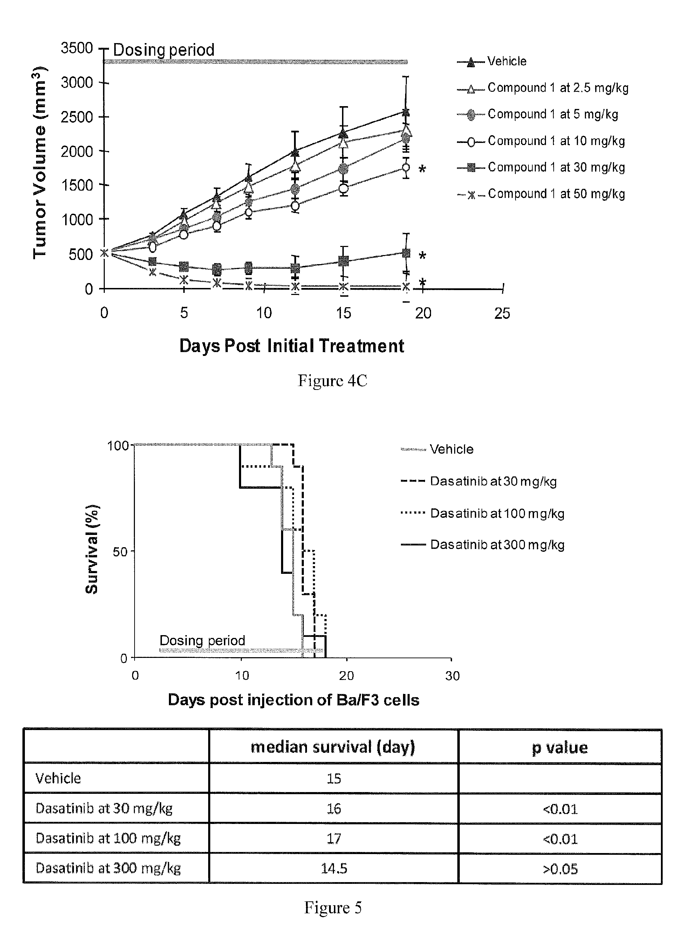

Figures 4A-4C demonstrate that compound 1 is effective in mouse xenograft models of BCR-ABL-Driven and BCR-ABL^{T315I}-driven tumor growth. Figures 4A and 4B are graphs showing the effect of compound 1 on survival of SCID mice after intravenous injection of Ba/F3 cells expressing either native BCR-ABL (Figure 4A) or BCR-ABL^{T315I} (Figure 4B). Ba/F3 cells expressing native BCR-ABL or BCR-ABL^{T315I} were injected into the tail vein of SCID mice, and animals were treated once daily by oral gavage with vehicle, compound 1, or dasatinib for the indicated dosing period (days 3-21). Figure 4C shows the in vivo efficacy of and suppression of BCR-ABL phosphorylation by compound 1 in a subcutaneous xenograft model using Ba/F3 cells expressing BCR-ABL^{T315I}. Cells were implanted subcutaneously into the right flank of nude mice, and when the average tumor volume reached approximately 500 mm³, and animals were treated once daily by oral gavage with vehicle or compound 1 for 19 consecutive days (dosing period indicated). Each compound 1 treatment group was compared to the vehicle group using Dunnett's test, and statistical significance (p<0.05) is indicated by an asterisk. BCR-ABL phosphorylation was evaluated in animals treated with a single dose of either vehicle or 30 mg/kg compound 1 by oral gavage (N=3 per group). Six hours after dosing, mice were sacrificed and tumor samples were analyzed by immunoblot analysis with antibodies against pBCR-ABL and eIF4E (loading control).
Figure 5 is a graph showing the effect of dasatinib in mouse models using Ba/F3 cells expressing BCR-ABLT315I. Survival curves are shown for mice treated during the indicated dosing period with vehicle or dasatinib. Median survival was calculated using the Kaplan-Meier method and statistical significance values are indicated for each group.

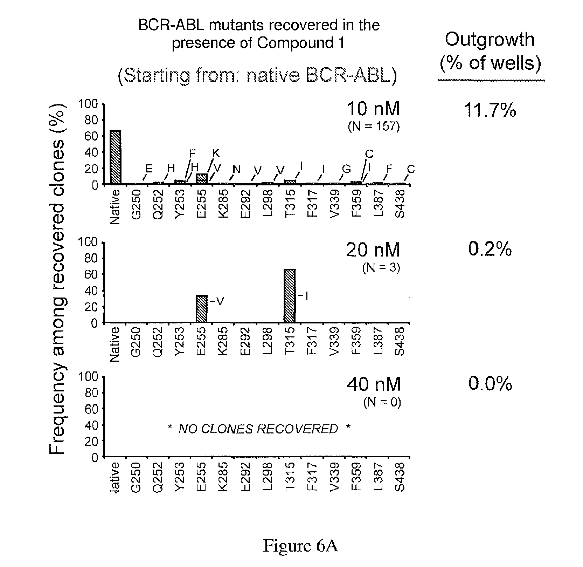

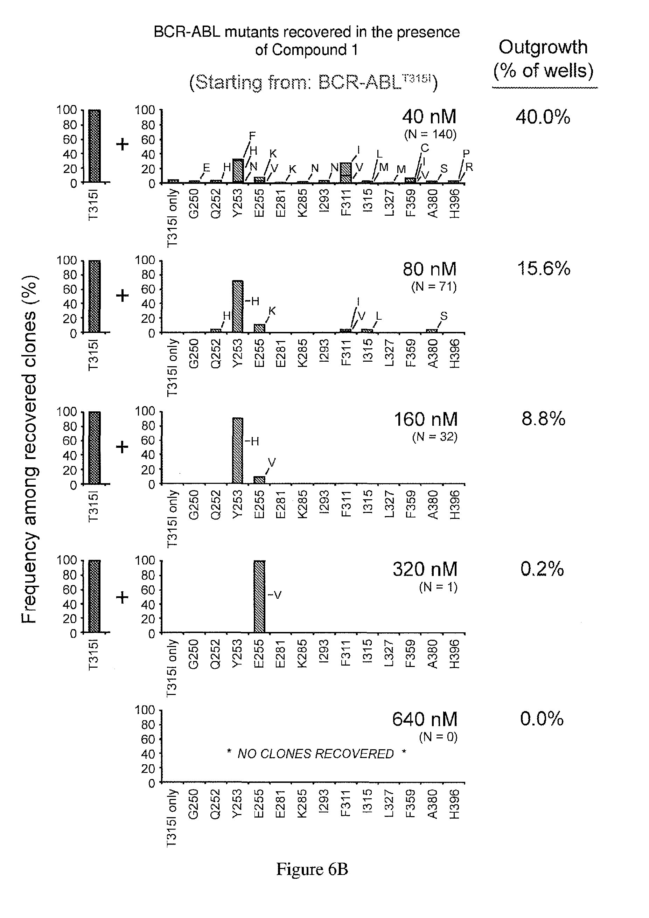

Figures 6A and 6B are graphs depicting the BCR-ABL mutants recovered in the presence of various concentrations of compound 1. Figure 6A shows the resistant subclones recovered from ENU-treated Ba/F3 cells starting from native BCR-ABL cultured in the presence of graded concentrations of compound 1 (10, 20, 40 nM). Each bar represents the relative percentage of the indicated BCR-ABL kinase domain mutant among recovered subclones. Since the percentage of surviving resistant subclones and the concentration of compound 1 are inversely related, a different number of sequenced subclones are represented in the graph for each concentration of compound 1 (see Table 2). The percent of wells surveyed that contained outgrowth is indicated to the right of each graph. Figure 6B shows the resistant subclones recovered from ENU-treated Ba/F3 cells expressing BCR-ABL^{T315I} cultured in the presence of graded concentrations of compound 1 (40, 80, 160, 320, 640 nM). A this assay started from cells expressing BCR-ABL^{T315I}, all recovered subclones contain the T315I mutation in addition to the specific secondary mutation indicated on each graph. The data demonstrates that compound 1, as a single agent, can suppress resistant outgrowth in cell-based mutagenesis screens.
Figures 7A-7D are graphs of pharmacokinetic data for compound 1. Figure 7A shows Cmax for various doses of compound 1 at cycle 1, day 1 (C1D1) and cycle 2, day 1 (C2D1). Figure 7B shows AUC for various doses of compound 1 at cycle 1, day 1 (C1D1) and cycle 2, day 1 (C2D1). Figure 7C shows concentration time profiles C1D1 following a single oral dose. Figure 7D shows concentration time profiles C2D1 following multiple oral doses.
Figures 8A-8E show pharmacodynamics data for compound 1. Figure 8A is a graph showing pharmacodynamics data for compound 1 in all patients in the clinical study and in patients having the T315I mutation. Figures 8B-8E are graphs showing pharmacodynamics data for compound 1 at 15 mg in patient having the F359C mutation (Figure 8B), for compound 1 at 30 mg in patient having no mutation (Figure 8C), for compound 1 at 45 mg in patient having the F359C mutation (Figure 8D), and for compound 1 at 60 mg in patient having the T315I mutation (Figure 8E).
Figure 9 is a graph showing inhibition of receptor phosphorylation of activated tyrosine kinases in AML cell lines. AML cell were incubated with increasing concentrations of compound 1 for 72 hours, and cell viability assessed using an MTS assay. MV4-11, Kasumi-1 and EOL-1 data are presented as mean ± SD from 3 experiments and KG1 data is presented as means ± SD from 2 experiments.
Figure 10 is a graph showing inhibition of growth and induction of apoptosis in MV4-11 cells. MV4-11 cells were seeded in 96-well plates, treated with increasing concentrations of compound 1 and caspase 3/7 activity measured at the indicated times. Data is expressed as fold induction of caspase activity relative to vehicle treated cells and is presented as means ± SD from 3 individual experiments

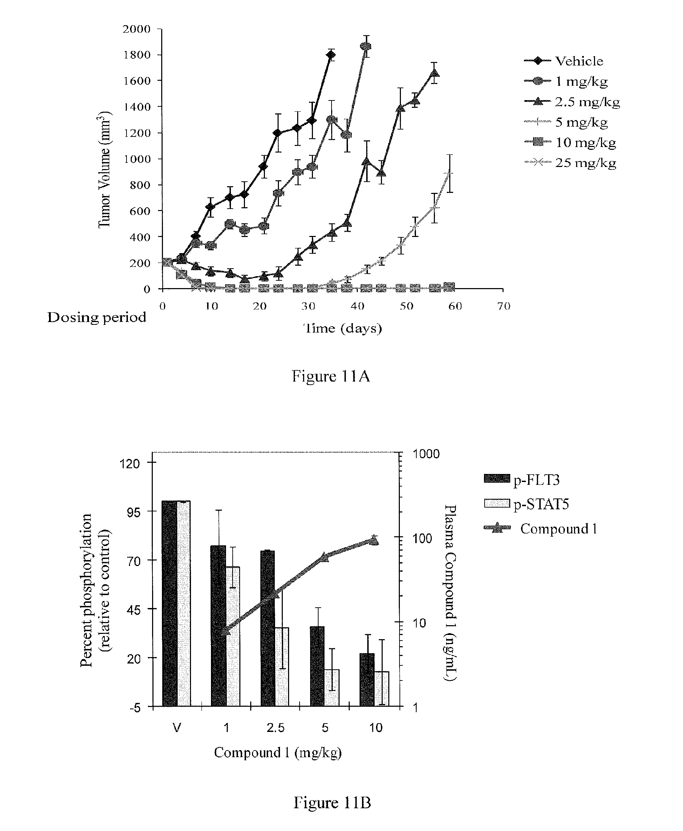

Figures 11A and 11B show efficacy and target inhibition of MV4-11 xenograft. Figure 11A is a graph of tumor growth for various doses of compound 1. Daily oral administration of vehicle or compound 1 for 4 weeks at doses of 1, 2.5, 5, 10 and 25 mg/kg/day was initiated when MV4-11 flank xenograft tumors reached approximately 200 mm3 (10 mice/group). Mean tumor volumes (± SEM) are plotted. Three of ten animals in the vehicle control group were sacrificed before the last treatment on day 28 due to tumor burden. Therefore tumor growth inhibition was calculated from day 0 to day 24 (as indicated by the asterisk), the next to last time point for tumor measurement during the dosing phase. Figure 11B is a graph showing inhibition of p-FLT3 and p-STAT5 for various doses of compound 1. Mice bearing established MV4-11 tumor xenografts were administered a single oral dose of compound 1 (4 mice/group) at the level indicated; control animals received vehicle alone (5 mice). Tumors were harvested 6 hours later, and analyzed for levels of phosphorylated and total FLT3 and STAT5 by immunoblotting. GAPDH was examined as a control. Quantification by densitometry of the relative phosphorylation of FLT3 and STAT5 as mean (±SEM) from two independent experiments are shown. FLT3 phosphorylation was normalized to GAPDH and STAT5 phosphorylation was normalized to total STAT5 protein.
Figure 12 is a graph showing ex vivo treatment of primary AML cells with compound 1 selectively inhibits FLT3-ITD cells. Primary leukemic blast cells were isolated from peripheral blood from 4 individual AML patients. FLT3-ITD status was determined by the pathology report and confirmed by PCR. Primary cell cultures were treated with the indicated concentrations of compound 1 for 72 hours, at which time viability was assessed using an MTS assay. All values were normalized to the viability of cells incubated in the absence of drug.
Figure 13 is a graph showing the effect of compound 1 on acute myelogenous leukemia (AML)-derived KG1 cells in a cell growth assay.
Figure 14 is a graph showing the effect of compound 1 on SNU16 gastric cancer cells with amplified FGFR2, compared to wtFGFR2 SNU1 cells, in a cell growth assay.
Figure 15 is a graph showing the effect of compound 1 on SNU16 gastric cancer cells in a soft agar colony formation assay.
Figure 16 is a graph showing the effect of compound 1 on AN3CA endometrial cancer cells with mutant FGFR2 (N549K), compared to wtFGFR2 Hec1B cells, in a cell growth assay.
Figure 17 is a graph showing the effect of compound 1 on MGH-U3 cells that express mutant FGFR3b (Y375C), compared to wtFGFR3 RT112 cells, in a cell growth assay.
Figure 18 is a graph showing the effect of compound I on OPM2 multiple myeloma ("MM") cells that carry t(4;14) translocation and express mutant FGFR3 (K650E), compared to wtFGFR3 NCI-H929 cells, in a cell growth assay.
Figure 19 is a graph showing the effect of compound 1 on MDA-MB-453 breast cancer cells that express mutant FGFR4 (Y367C) in a cell growth assay.
Figure 20 is a graph showing the effect of oral dosing of compound 1 on tumor growth in a xenograft model with FGFR2-driven AN3CA endometrial cancer cells.
Figure 21 is a graph showing in vivo pharmacodynamics and pharmacokinetics of oral dosing of compound 1 in a xenograft model with AN3CA endometrial cancer cells.
Figure 22 is a graph showing the results of a cell growth assay with endometrial cancer cell lines (AN3CA and MFE-296) and wild type FGFR2 cell lines (Hec-1-B and RL95-2) upon treatment with compound 1.
Figure 23 is a graph showing the effect of oral dosing of compound 1 in an AN3CA endometrial tumor xenograft on tumor growth.

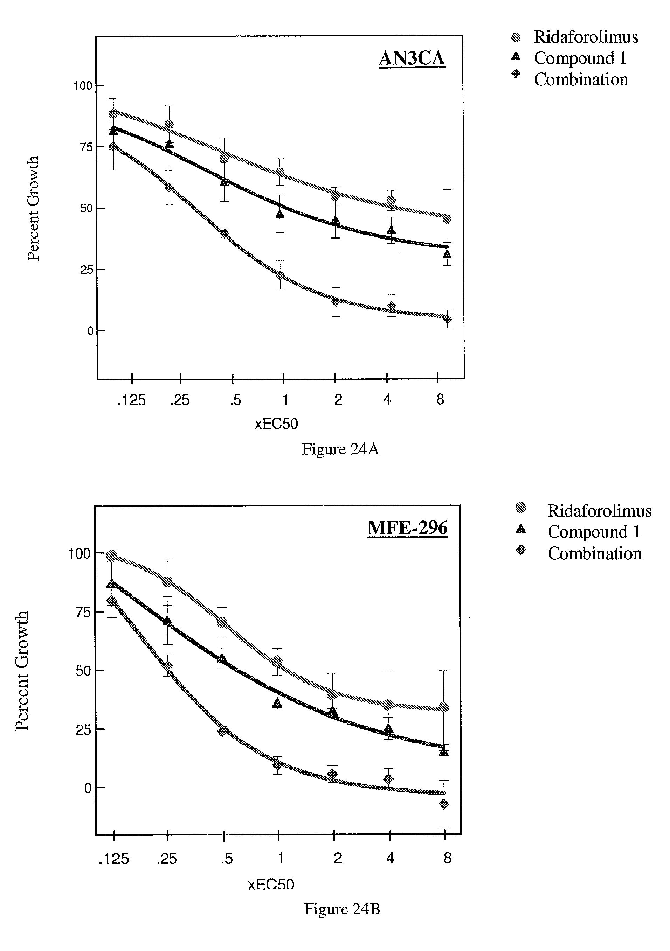

Figures 24A and 24B are graphs showing the effect of a combination of compound 1 with ridaforolimus on FGFR2-mutant endometrial cancer cells in a cell growth assay. Figure 24A shows the results of a cell growth assay with the AN3CA endometrial cancer cell line. The 1×EC50 concentration used to treat AN3CA cells for compound 1 is 30 nM and for ridaforolimus is 0.4 nM. Figure 24B shows the results of a cell growth assay with the MFE-296 endometrial cancer cell line. The 1×EC50 concentration used to treat MFE-296 cells for compound 1 is 100 nM and for ridaforolimus is 1 nM. Data are shown for ridaforolimus alone ("Ridaforolimus"), compound 1 alone ("Compound 1"), and a combination of compound 1 with ridaforolimus ("Combination").
Figures 25A and 25B are graphs showing median effect analyses of a combination of compound 1 with ridaforolimus. Data are shown for the AN3CA cell line (Figure 25A) and the MFE-296 cell line (Figure 25B).
Figure 26 is a graph showing cell cycle analysis in the AN3CA cell line following treatment. Data are shown cells with no treatment ("untreated") or cells treated with ridaforolimus alone, compound 1 alone, or a combination of compound 1 with ridaforolimus.
Figure 27 is a schematic showing a possible FGFR2/MAPK pathway and mTOR pathway (modified from Katoh M., J. Invest. Dermatol., 2009, 128: 1861-1867).
Figures 28A and 28B are graphs showing the effect of oral dosing of compound 1 with ridaforolimus in an AN3CA endometrial tumor xenograft. Figure 28A shows data for a low dose combination of 10 mg/kg compound 1 with ridaforolimus. Figure 28B shows data for a high dose combination of 30 mg/kg compound 1 with ridaforolimus. Data are shown for ridaforolimus alone ("Rid"), compound 1 alone ("Compound 1"), and a combination of compound 1 with ridaforolimus ("Compound 1, Rid"). Dosages are provided in parenthesis as units of mg/kg.
Figure 29 shows pharmacokinetics and pharmacodynamics data for oral dosing of ridaforolimus alone, compound 1 alone, and a combination of compound 1 with ridaforolimus. Data are shown for various concentrations of ridaforolimus ("Rid") and compound 1 ("Compound 1").

### Detailed Description

The invention provides methods for treating cancer, involving administration of a compound 1. Non-limiting examples of cancers include those that result in solid tumors, such as acute myelogenous leukemia, gastric or gastrointestinal cancer, endometrial cancer, bladder cancer, multiple myeloma, or breast cancer. Other examples of cancers include myelogenous leukemia, acute lymphoblastic leukemia, acute myelogenous leukemia, or a myelodysplastic syndrome (e.g., refractory anemia with excess of blasts group 1 (RAEBI) or refractory anemia with excess of blasts group 2 (RAEBII)).

In addition to the cancers mentioned above, the methods and compositions of the invention can be used to treat the following types of cancers, as well as others: skin (e.g., squamous cell carcinoma, basal cell carcinoma, or melanoma), prostate, brain and nervous system, head and neck, testicular, lung, liver (e.g., hepatoma), kidney, bone, endocrine system (e.g., thyroid and pituitary tumors), and lymphatic system (e.g., Hodgkin's and non-Hodgkin's lymphoma) cancers. Other types of cancers that can be treated using the methods of the invention include fibrosarcoma, neurectodermal tumor, mesothelioma, epidermoid carcinoma, and Kaposi's sarcoma.

Compound 1 has been found to possess strong antiangiogenic properties and, therefore, can be useful for the treatment of condition associated with aberrant angiogenesis, including solid cancers (e.g., prostate cancer, lung cancer, breast cancer, colorectal cancer, renal cancer, and glioblastoma), diabetic retinopathy, rheumatoid arthritis, psoriasis, atherosclerosis, chronic inflammation, obesity, macular degeneration, and a cardiovascular disease.

In particular, compound 1 is a pan-BCR-ABL inhibitor. Inhibition of the oncogenic BCR-ABL tyrosine kinase by imatinib induces durable responses in many patients with chronic phase chronic myelogenous leukemia (CML), while relapse is common in advanced CML and Ph+ acute lymphoblastic leukemia. Imatinib resistance is commonly attributed to BCR-ABL kinase domain mutations, and second-line BCR-ABL inhibitors nilotinib and dasatinib provide treatment alternatives for these patients. However, cross-resistance of the BCR-ABL^{T315I} mutation and multi-resistant compound mutants selected on sequential ABL kinase inhibitor therapy remain clinical concerns. Here, we describe the evaluation of compound 1, a potent inhibitor of BCR-ABL^{T315I} and other resistant mutants in vitro and in vivo. Compound 1 was found to inhibit the inactive form of BCR-ABL^{T31SI}. In cell-based mutagenesis screens, compound 1 completely suppressed resistance at certain concentrations, including the T315I mutant. The availability of an orally administered pan-BCR-ABL tyrosine kinase inhibitor, such as compound 1 offers important therapeutic advantages in a first-line capacity by minimizing the emergence of BCR-ABL kinase domain mutation-based drug resistance during treatment.

Furthermore, we have discovered that the combination of an mTOR and compound 1 is more effective than rapamycin macrolide monotherapy or compound 1 monotherapy for treating pathological cellular proliferation, inhibiting angiogenesis, and increasing the apoptosis of cancer cells. Non-limiting examples of cancers that can be treated using the compositions, methods, or kits of the invention include carcinoma of the bladder, breast, colon, kidney, liver, lung, head and neck, gall-bladder, ovary, pancreas, stomach, cervix, thyroid, prostate, or skin; squamous cell carcinoma; endometrial cancer; multiple myeloma; a hematopoietic tumor of lymphoid lineage (e.g., leukemia, acute lymphocytic leukemia, acute lymphoblastic leukemia, B-cell lymphoma, T-cell-lymphoma, Hodgkin's lymphoma, non-Hodgkin's lymphoma, hairy cell lymphoma, or Burkitt's lymphoma); a hematopoietic tumor of myelogenous lineage (e.g., acute myelogenous leukemia, chronic myelogenous leukemia, multiple myelogenous leukemia, myelodysplastic syndrome, or promyelocytic leukemia); a tumor of mesenchymal origin (e.g., fibrosarcoma or rhabdomyosarcoma); a tumor of the central or peripheral nervous system (e.g., astrocytoma, neuroblastoma, glioma, or schwannomas); melanoma; seminoma; teratocarcinoma; osteosarcoma; or Kaposi's sarcoma. Non-limiting examples of conditions associated with aberrant angiogenesis which can be treated using the compositions, methods, or kits of the invention include solid tumors (e.g., prostate cancer, lung cancer, breast cancer, colorectal cancer, renal cancer, or glioblastoma), diabetic retinopathy, rheumatoid arthritis, psoriasis, atherosclerosis, chronic inflammation, obesity, macular degeneration, and a cardiovascular disease.

### Synthesis and Formulation of Compound 1

Compound 1 can be synthesized at described in Scheme 1 and as described in PCT Publication No. WO 2007/075869. Alternatively, the acid chloride utilized in step can be replaced with a methyl ester as depicted in Scheme 2 which describes the modification of step 5.

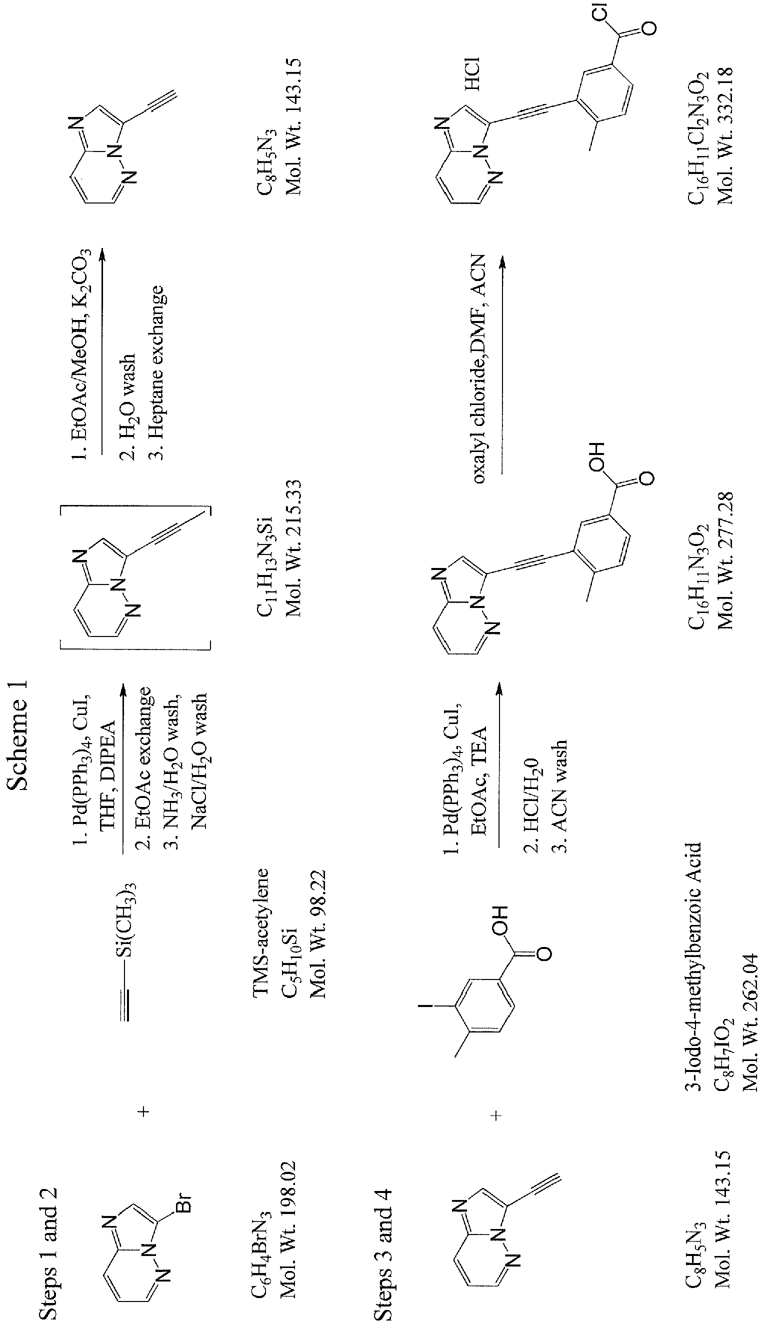

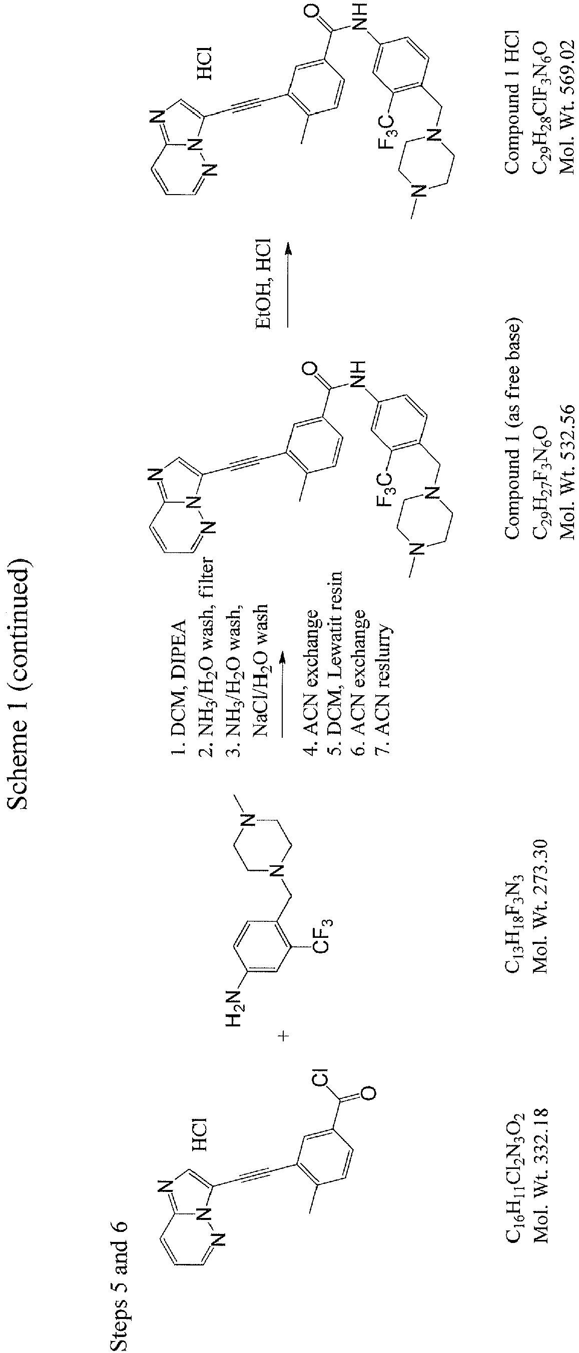

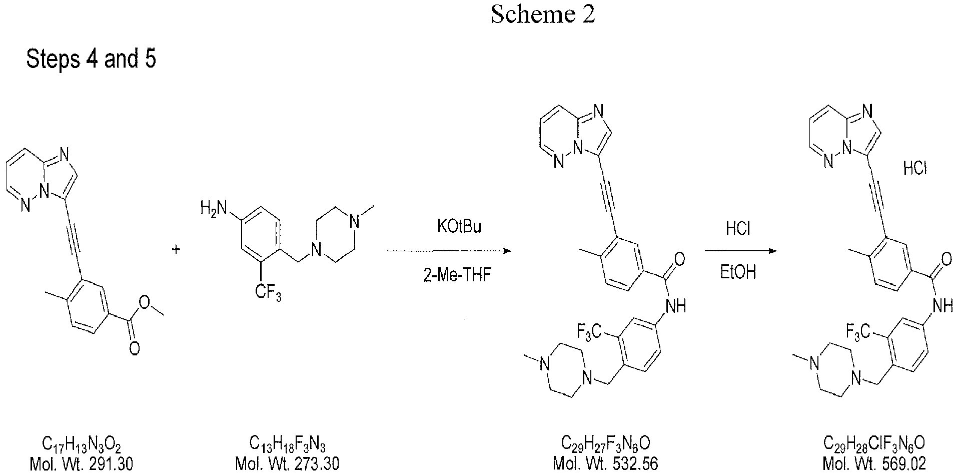

The mono-hydrochloride salt of compound 1 was used for carrying out clinical trials instead of the significantly less water soluble free base. The mono-HCl salt was found to be a crystalline, anhydrous solid formed from a range of solvents reproducibly. The hydrochloride salt of compound 1 has a thermodynamic solubility in unbuffered water of 1.7 mg/mL at pH 3.7.

Further identifying information for compound 1 includes:
Chemical name: 3-(Imidazo[1,2-b]pyridazin-3-ylethynyl)-4-methyl-N-(4-((4-methylpiperazin-1-yl)methyl)-3-(trifluoromethyl)phenyl)benzamide, hydrochloride salt;
USAN name: ponatinib (INN pending);
CAS Registry No.: 1114544-31-8 (HCl Salt) and 943319-70-8 (free base);
CAS Index name: Benzamide,3-(2-imidazo[1,2-b]pyridazin-3-ylethnyl)-4-methyl-N-[4-[(4-methyl-1-piperazinyl)methyl]-3-(trifluoromethyl)phenyl]-hydrochloride (1:1);
Molecular Formula: C₂₉H₂₈ClF₃N₆O (HCl salt) and C₂₉H₂₇F₃N₆O (free base) (no chiral centers); and
Molecular Weight: 569.02 g/mol (HCl salt) and 532.56 g/mol (free base).

Compound 1, or preferably a pharmaceutically acceptable salt thereof, such as the mono HCl salt, may be formulated for oral administration using any of the materials and methods useful for such purposes. Pharmaceutically acceptable compositions containing compound 1 suitable for oral administration may be formulated using conventional materials and methods, a wide variety of which are well known. While the composition may be in solution, suspension or emulsion form, solid dosage forms such as capsules, tablets, gel caps, caplets, etc. are of greatest current interest. Methods well known in the art for making formulations, including the foregoing unit dosage forms, are found, for example, in "Remington: The Science and Practice of Pharmacy" (20th ed., ed. A.R. Gennaro, 2000, Lippincott Williams & Wilkins). Compound 1 may be provided neat in capsules, or combined with one or more optional, pharmaceutically acceptable excipients such as fillers, binders, stabilizers, preservatives, glidants, disintegrants, colorants, film coating, etc., as illustrated below.

For example, white opaque capsules were prepared containing nominally 2 mg of compound 1 free base, provided as the hydrochloride salt, with no excipients. White opaque capsules were also prepared containing 5 mg, 15 mg, or 20 mg of compound 1 free base, provided as the hydrochloride salt, mixed with conventional excipients. Inactive ingredients used as excipients in an illustrative capsule blend include one or more of a filler, a flow enhancer, a lubricant, and a disintegrant. For instance, a capsule blend was prepared for the 5, 15 and 20 mg capsules, containing the compound 1 HCl salt plus colloidal silicon dioxide (ca. 0.3% w/w, a flow enhancer), lactose anhydrous (ca. 44.6% w/w, a filler), magnesium stearate (ca. 0.5% w/w, a lubricant), microcrystalline cellulose (ca. 44.6% w/w, a filler), and sodium starch glycolate (ca. 5% w/w, a disintegrant). The capsule shell contains gelatin and titanium dioxide.

The formulation process used conventional blending and encapsulation processes and machinery. The hydrochloride salt of compound 1 and all blend excipients except magnesium stearate were mixed in a V-blender and milled through a screening mill. Magnesium stearate was added and the material was mixed again. The V-blender was sampled to determine blend uniformity. The blend was tested for bulk density, tap density, flow, and particle size distribution. The blend was then encapsulated into size "3", size "4", or size "1" capsule shells, depending upon the strength of the unit dosage form.

Compound 1 was also formulated into tablets using conventional pharmaceutical excipients, including one or more of a filler or a mixture of fillers, a disintegrant, a glidant, a lubricant, a film coating, and a coating solvent in a blend similar to that used in the higher strength capsules. For example, tablets may be prepared using the following relative amounts and proportions (weight/weight): compound 1 (90 g provided as the HCl salt, 15.0% w/w), colloidal silicon dioxide (1.2 g, 0.2% w/w), lactose monohydrate (2409 g, 40.15% w/w), magnesium stearate (3 g, 0.5% w/w), microcrystalline cellulose (240.9 g, 40.15% w/w), and sodium starch glycolate (24 g, 4.0% w/w), with the amount of lactose monohydrate adjusted based on the amount of drug used.

Compound 1 and the excipients may be mixed using the same sort of machinery and operations as was used in the case of capsules. The resultant, uniform blend may then be compressed into tablets by conventional means, such as a rotary tablet press adjusted for target tablet weight, e.g. 300 mg for 45 mg tablets or 100 mg for 15 mg tablets; average hardness of e.g., 13 kp for 45 mg tablets and 3 kp for 15 mg tablets; and friability no more than 1%. The tablet cores so produced may be sprayed with a conventional film coating material, e.g., an aqueous suspension of Opadry® II White, yielding for example a ∼2.5% weight gain relative to the tablet core weight.

### mTOR Inhibitors

The mammalian target of rapamycin, commonly known as mTOR, is a serine/threonine protein kinase that regulates cell growth, cell proliferation, cell motility, cell survival, protein synthesis, and transcription. mTOR inhibitors, including rapamycin and its analogues, are a class of therapeutics that specifically inhibit signaling from mTOR or a combination of kinases including mTOR (e.g., such agents which act as inhibitors of both PI3K and mTOR). mTOR is a key intermediary in multiple mitogenic signaling pathways and plays a central role in modulating proliferation and angiogenesis in normal tissues and neoplastic processes. There are two classes of mTOR inhibiting compounds: rapamycin macrolides and non-rapamycin analogs.

Rapamycin (sirolimus) is an immunosuppressive lactam macrolide that is produced by *Streptomyces hygroscopicus*. See, for example, J.B. McAlpine et al., J. Antibiotics, 1991, 44: 688; S.L. Schreiber et al., J. Am. Chem. Soc., 1991, 113: 7433; and U.S. Patent No. 3,929,992, incorporated herein by reference.

Because there is more than one accepted convention for numbering the atoms of rapamycin and its analogs, the numbering convention used herein is depicted below:

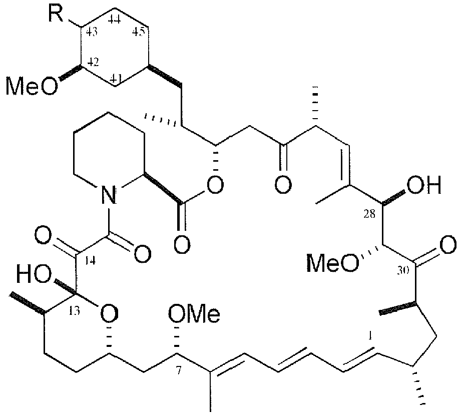

For reference, the R group for a number of compounds is set forth in the following table:

| **Compound** | **-R** |
|---|---|
| Rapamycin | -OH |
| AP23573 | -OP(O)(Me)₂ |
| Temsirolimus | -OC(O)C(CH₃)(CH₂OH)₂ |
| Everolimus | -OCH₂CH₂OH |
| Biolimus | -OCH₂CH₂OEt |
| ABT-578 | -Tetrazole |

Desirable rapamycin macrolides for use in the combination therapy of the invention include, but are not limited to, rapamycin (sirolimus or Rapamune (Wyeth)), temsirolimus or CCI-779 (Wyeth, see, U.S. Pat. Nos. 5,362,718 and 6,277,983, the contents of which are incorporated by reference herein in their entirety), everolimus or RAD001 (Novartis), ridaforolimus or AP23573 (Ariad), biolimus (Nobori), and zotarolimus or ABT 578 (Abbott Labs.).

Temsirolimus is a soluble ester prodrug of rapamycin, rapamycin 42-ester with 3-hydroxy-2-(hydroxymethyl)-2-methylpropionic acid, which is disclosed in U.S. Pat. No. 5,362,718. Temsirolimus has demonstrated significant inhibitory effects on tumor growth in both in vitro and in vivo models. Temsirolimus exhibits cytostatic, as opposed to cytotoxic properties, and may delay the time to progression of tumors or time to tumor recurrence. As disclosed in WO 00/240000, CCI-779 may be useful for the treatment of cancers of various origins, including renal, breast, cervical, uterine, head and neck, lung, prostate, pancreatic, ovarian, colon, lymphoma, and melanoma.

Everolimus is 40-O-(2-hydroxy)ethyl-rapamycin, the structure and synthesis of which is disclosed in WO 94/09010. Everolimus, which has been shown to be a potent immunosuppressive agent (U.S. Pat. No. 5,665,772), also exhibits evidence of antineoplastic properties (see, e.g., A. Boulay et al., Cancer Res., 2004, 64: 252-261). As a result of these properties, everolimus is currently marketed in certain countries as an immunosuppressant for prevention of allograft rejection (B. Nashan, Ther. Drug. Monit., 2002, 24: 53-58) and has undergone clinical testing as an anti-cancer agent (S. Huang and P.J. Houghton, Curr. Opin. Invest. Drugs, 2002, 3: 295-304; M.M. Mita et al., Clin. Breast Cancer, 2003, 4: 126-137; and M. Hidalgo and E.J. Rowinsky, Oncogene, 2000, 19: 6680-6686).

Zotarolimus is the 43-epi isomer thereof, e.g., as disclosed in WO 99/15530, or rapamycin analogs as disclosed in No. WO 98/02441 and WO 05/016252.

Ridaforolimus is a phosphorous-containing rapamycin derivative (see WO 03/064383, Example 9 therein). Like temsirolimus and everolimus, ridaforolimus has demonstrated antiproliferative activity in a variety of PTEN-deficient tumor cell lines, including glioblastoma, prostate, breast, pancreas, lung and colon (E.K. Rowinsky, Curr. Opin. Oncol., 2004, 16: 564-575). Ridaforolimus has been designated as a fast-track product by the U.S. Food and Drug Administration for the treatment of soft-tissue and bone sarcomas. Ridaforolimus has been tested in multiple clinical trials targeting hematologic malignancies (e.g., leukemias and lymphomas) and solid tumors (e.g., sarcomas, prostate cancer, and glioblastoma multiforme).

Many rapamycin macrolides are known in the art. Rapamycin macrolides which can be used in the methods, kits, and compositions of the invention include 42-desmethoxy derivatives of rapamycin and its various analogs, as disclosed, e.g., in WO 2006/095185 (in which such compounds are referred to as "39-desmethoxy" compounds based on their numbering system). The derivatives of rapamycin are of particular current interest in practicing this invention

Additionally, a large number of other structural variants of rapamycin have now been reported, typically arising as alternative fermentation products and/or from synthetic efforts. For example, the extensive literature on analogs, homologs, derivatives and other compounds related structurally to rapamycin ("rapalogs") include, among others, variants of rapamycin having one or more of the following modifications relative to rapamycin: demethylation, elimination or replacement of the methoxy at C7, C42 and/or C29; elimination, derivatization or replacement of the hydroxy at C 13, C43 and/or C28; reduction, elimination or derivatization of the ketone at C14, C24 and/or C30; replacement of the 6-membered pipecolate ring with a 5-membered prolyl ring; alternative substitution on the cyclohexyl ring or replacement of the cyclohexyl ring with a substituted cyclopentyl ring; epimerization of the C28 hydroxyl group; and substitution with phosphorous-containing moieties.

Thus, mTOR inhibitors include, for example, 43- and/or 28-esters, ethers, carbonates, carbamates, etc. of rapamycin including those described in the following patents, which are all hereby incorporated by reference: alkyl esters (U.S. Patent No. 4,316,885); aminoalkyl esters (U.S. Patent No. 4,650,803); fluorinated esters (U.S. Patent No. 5,100,883); amide esters (U.S. Patent No. 5,118,677); carbamate esters (U.S. Patent No. 5,118, 678); silyl esters (U.S. Patent No. 5,120,842); aminodiesters (U.S. Patent No. 5,162,333); sulfonate and sulfate esters (U.S. Patent No. 5,177,203); esters (U.S. Patent No. 5,221, 670); alkoxyesters (U.S. Patent No. 5,233,036); O-aryl, -alkyl, -alkenyl, and -alkynyl ethers (U.S. Patent No. 5,258,389); carbonate esters (U.S. Patent No. 5,260,300); arylcarbonyl and alkoxycarbonyl carbamates (U.S. Patent No. 5,262,423); carbamates (U.S. Patent No. 5,302,584); hydroxyesters (U.S. Patent No. 5,362,718); hindered esters (U.S. Patent No. 5,385,908); heterocyclic esters (U.S. Patent No. 5,385,909); gem- disubstituted esters (U.S. Patent No. 5,385,910); amino alkanoic esters (U.S. Patent No. 5,389,639); phosphorylcarbamate esters (U.S. Patent No. 5,391,730); carbamate esters (U.S. Patent No. 5,411,967); carbamate esters (U.S. Patent No. 5,434,260); amidino carbamate esters (U.S. Patent No. 5,463,048); carbamate esters (U.S. Patent No. 5,480,988); carbamate esters (U.S. Patent No. 5,480,989); carbamate esters (U.S. Patent No. 5,489,680); hindered N-oxide esters (U.S. Patent No. 5,491,231); biotin esters (U.S. Patent No. 5,504,091); O-alkyl ethers (U.S. Patent No. 5,665,772); and PEG esters of rapamycin (U.S. Patent No. 5,780,462). Also included are the reduced products, 24-dihydro-, 30-dihydro- and 24, 30-tetrahydro-rapamycin analogs and the 28-epi analogs (see, e.g., WO 01/14387) of rapamycin or of any of the foregoing compounds, as well as esters or ethers of any of the foregoing as well as oximes, hydrazones, and hydroxylamines of non-reduced compounds. See e.g. U.S. Patent Nos. 5,373,014, 5,378,836, 5,023,264, 5,563,145 and 5,023,263.

Non-rapamycin analog mTOR inhibiting compounds include, but are not limited to, LY294002, Pp242 (Chemdea Cat. No. CD0258), WYE-354 (Chemdea Cat. No. CD0270), Ku-0063794 (Chemdea Cat. No. CD0274), XL765 (Exelixis; J. Clin. Oncol., 2008,2008 ASCO Annual Meeting Proceedings 26:15S), AZD8055 (Astrazeneca), NVP-BEZ235 (Sauveur-Michel et al., Mol. Cancer Ther., 2008, 7:1851), OSI-027 (OSI Pharmaceuticals), wortmannin, quercetin, myricentin, staurosporine, and ATP competitive inhibitors (see U.S. patent application Ser. Nos. 11/361,213 and 11/361,599, each of which arc incorporated herein by reference in their entirety).

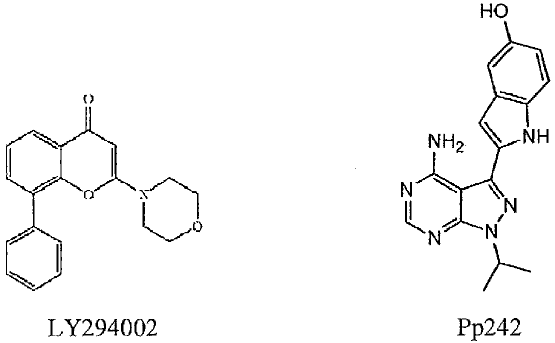

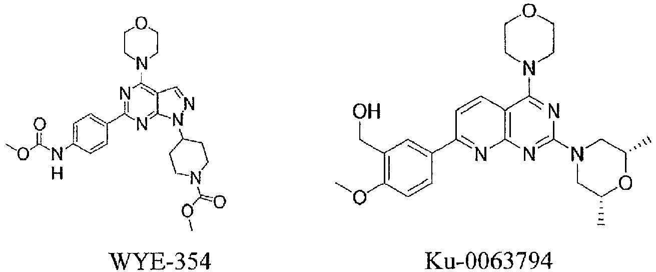

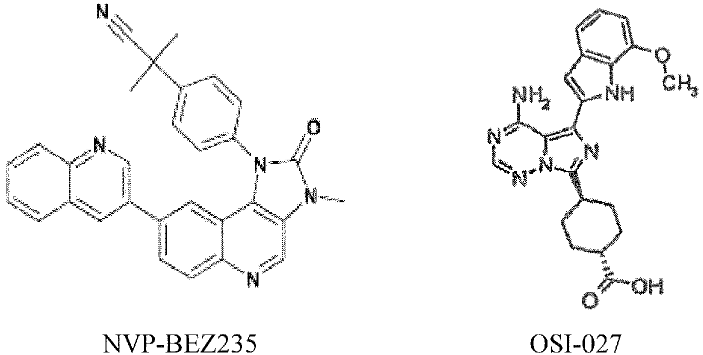

Other non-rapamycin analog mTOR inhibiting compounds which can be used in the methods, kits, and compositions of the invention include those described in PCT Publication Nos. WO2009008992; WO2009007750; WO2009007751; WO2009007749; WO2009007748; WO2008032060; WO2008032036; WO2008032033; WO2008032089; WO2008032091; WO2008032064; WO2008032077; WO2008032041; WO2008023159; WO2008023180; WO2007135398; WO2007129044; WO2007080382; and WO2006090169, each of which is incorporated herein by reference.

### Pharmaceutical Compositions

Formulations of mTOR inhibitors are very well well known in the art, including, e.g., solid dosage forms suitable for oral administration for sirolimus, temsirolimus, ridaforolimus, and everolilnus, as well as other compositions of temsirolimus and ridaforolimus for i.v. administration. Formulations of the non-macrolide mTOR inhibitors are disclosed in the patent documents referenced above. Compound 1 may be formulated together with the mTOR inhibitor, but more typically would be formulated separately to avoid complicating the formulation process and to permit independent scheduling of administration and dosing regiments of the two agents and to permit more convenient subsequent adjustments in dose of either agent.

### Dosage and Administration

In accordance with the methods, kits, and compositions of the invention a treatment may consist of a single dose or a plurality of doses over a period of time. Compound 1 may be administered alone or concurrently with administration of the mTOR inhibitor. Alternatively, compound 1 and the mTOR inhibitor may be administered sequentially. For example, compound 1 may be administered prior to or following administration of the mTOR inhibitor (e.g., one or more day(s) before and/or one or more day(s) after).

Administration may be one or multiple times daily, weekly (or at some other multiple day interval) or on an intermittent schedule, with that cycle repeated a given number of times (e.g., 2-10 cycles) or indefinitely.

Depending on the route of administration, effective doses may be calculated according to the body weight, body surface area, or organ size of the subject to be treated. Optimization of the appropriate dosages can readily be made by one skilled in the art in light of pharmacokinetic data observed in human clinical trials. The final dosage regimen will be determined by the attending physician, considering various factors which modify the action of the drugs, e.g., the drug's specific activity, the severity of the damage and the responsiveness of the subject, the age, condition, body weight, sex and diet of the subject, the severity of any present infection, time of administration, the use (or not) of concomitant therapies, and other clinical factors. As studies are conducted using the inventive combinations, further information will emerge regarding the appropriate dosage levels and duration of treatment.

In the combination therapy of the invention compound 1 is typically administered in a repeating cycle of total daily doses of 10 - 500 mg of compound 1 orally each day. The mTOR inhibitor can be given before, after or simultaneously with the compound 1, and on the same or different dosing schedules and by the same or different routes of administration. Dose levels for the mTOR inhibitor in this combination therapy are generally in the range of 10 - 800 mg overall per week of treatment, e.g., in some cases 35 - 250 mg/week. Such overall weekly dosage levels may be achieved using a variety of routes of administration and dosing schedules. The dosing schedule may be intermittent. "Intermittent" dosing refers to schedules providing intervening periods between doses, e.g. every second day dosing, every third day dosing, or more generally, schedules containing "holidays" of one or more days or weeks between periods of dosing. Non-limiting examples of such intermittent dosing including dosing on fewer than seven days per week as well as dosing cycles of one week of QDx4, QDx5, QDx6 or daily dosing followed by a period without drug, e.g., one, two or three weeks, then resuming with another week of drug treatment followed by a week (or weeks) without drug treatment, and so on. To illustrate further, administration of 60 mg QDx6 every other week provides a weekly dose of 360 mg of drug on an intermittent basis (i.e., every other week).

For example, in the case of oral administration, 2 -- 160 mg of the drug can be given one or more days per week, e.g. every day (QDx7), six days per week (QDx6), five days per week (QDx5), etc. Thus, everolimus may be given QDx7 at doses of 3 - 20 mg/day, e.g., 5mg or 10 mg. Ridaforolimus may be given QDx7 p.o. at doses of 10 - 25 mg/day, e.g., 10, 12.5 or 15 mg/day; or sirolimus at 2 or 4 mg p.o. QDx7, in some cases with a 6, 8, or 10 mg loading dose. The dosing schedule may be intermittent, as illustrated by QDx4, QDx5, and QDx6 schedules. Examples include oral administration of the mTOR inhibitor at 30 -100mg QDx5 or QDx6. For instance, in the practice of this invention, ridaforolimus, everolimus, temsirolimus or sirolimus is administered orally at levels of 10 - 50 mg QDx5. For certain indications, it may be desirable to administer ridaforolimus QDx5 at dose levels of 30 - 50 mg orally.

The desired overall level of exposure to the mTOR inhibitor can alternatively be achieved by various schedules of parenteral delivery. In such cases, 10 - 250 mg of the mTOR inhibitor is administered, for example, by i.v: infusion over 15 - 60 minutes, often 30 - 60 minutes, one or more times per 1-to 4-week period. In one such approach, the mTOR inhibitor is administered in a 30 - 60 minute i.v. infusion once each week for three or four weeks every 4-week cycle. Such i.v. delivery is of particular interest in the case of ridaforolimus, sirolimus and temsirolimus, which can be provided, for example, in weekly doses of 10 - 250 mg, e.g., 25, 50, 75, 100, 150, 200, or 250 mg/week, for three or four weeks of each 4-week cycle. Dose levels of 50 and 75 mg are of particular current interest. In another approach, the mTOR inhibitor is administered by i.v. infusion of 5 - 25 mg of the drug QDx5 every two weeks (e.g., with i.v. infusions Monday through Friday, every 2d week). Doses of 10, 12.5, 15, 17.5, and 20 mg are of particular current interest.

Of interest are dose levels and dosing schedules already approved or under study for the mTOR inhibitor in a monotherapy administered as part of a combination therapy with compound 1 as described herein.

Also of interest are combination therapies in which dose levels and/or dosing schedules result in a low dose (i.e., less than those amounts used for monotherapy) of mTOR inhibitor and/or compound 1 being administered to the subject.

### Indications

The methods, kits, and compositions of the invention can be used to treat disorders associated with pathological cellular proliferation, such as neoplasms, cancer, and conditions associated with pathological angiogenesis. Non-limiting examples of conditions associated with aberrant angiogenesis which can be treated using the compositions, methods, or kits of the invention include solid tumors, diabetic retinopathy, rheumatoid arthritis, psoriasis, atherosclerosis, chronic inflammation, obesity, and macular degeneration.

The methods, kits, and compositions of the invention can be used to treat primary and/or metastatic cancers, and other cancerous conditions. For example, the inventive compositions and methods should be useful for reducing size of solid tumors, inhibiting tumor growth or metastasis, treating various lymphatic cancers, and/or prolonging the survival time of mammals (including humans) suffering from these diseases.

Particular examples of conditions associated with proliferation of BCR-ABL expressing cells include cancer, such as any described herein. Additional cancers include chronic myelogenous leukemia, acute lymphoblastic leukemia, and acute myelogenous leukemia.

Particular examples of conditions associated with proliferation of FLT-3 mutant expressing cells include cancer and conditions associated with cancer, such as any cancer described herein. Activating mutations in FLT3 are the most common type of genetic alteration in acute myelogenous leukemia (AML). A majority of these mutations arise from an internal tandem duplication (ITD) in the juxtamembrane region of the receptor. Activating point mutations in the kinase activation loop also occur but with lower frequency. FLT3-ITD mutations have been associated with a worse prognosis for AML patients, both in terms of relapse and overall survival, when treated with standard therapy. Additional conditions include myelodysplastic syndromes (MDS), such as refractory anemia, refractory anemia with excess of blasts (RAEB) (e.g., RAEBI having 5-9% blasts and RAEBII having 10-19% blasts), refractory anemia with ringed sideroblasts, chronic myelomonocytic leukemia (CMML), and atypical chronic myelogenous leukemia (a-CML).

Other examples of conditions include those associated with FGFR1, PDGFRα, and KIT. Translocations affecting the activity of FGFR1 and PDGFRα are found in a subset of rare myeloproliferative neoplasms (MPNs). Translocations involving the FGFR1 gene and a range of other chromosome partners such as the FGFR1OP2 gene are characteristic of 8p11 myeloproliferative syndrome (EMS), a disease in which most patients ultimately and rapidly progress to AML. The FIP1L1-PDGFRα fusion protein is found in approximately 10-20% of patients with chronic cosinophilic leukemia/idiopathic hypereosinophilia (CEL/HEL) and it has been reported that these patients respond well to PDGFR inhibition. Also, the T674I mutant of PDGFRα is mutated at the position analogous to the T315I gatekeeper reside of BCR-ABL. Activating mutations in KIT (e.g., cKIT or N822K) are also found in AML. KIT mutations are less common and are found in specific cytogenetic subsets of AML with an overall frequency 2-8%.

Other examples of conditions include those associated with proliferation of cancer cells, such as cancers cells that result in solid tumors. Exemplary solid tumors include gastric or gastrointestinal cancer, endometrial cancer, bladder cancer, multiple myeloma, breast cancer, prostate cancer, lung cancer, colorectal cancer, renal cancer, and glioblastoma multiforme.

Examples of cancers and cancer conditions that can be treated include, but are not limited to, tumors of the brain and central nervous system (e.g., tumors of the meninges, brain, spinal cord, cranial nerves, and other parts of the CNS, such as glioblastomas or medulla blastomas); head and/or neck cancer; breast tumors; tumors of the circulatory system (e.g., heart, mediastinum and pleura, and other intrathoracic organs, vascular tumors, and tumor-associated vascular tissue); tumors of the blood and lymphatic system (e.g., Hodgkin's disease, Non-Hodgkin's disease lymphoma, Burkitt's lymphoma, AIDS-related lymphomas, malignant immunoproliferative diseases, multiple myeloma, malignant plasma cell neoplasms, lymphoid leukemia, myelogenous leukemia, acute or chronic lymphocytic leukemia, monocytic leukemia, other leukemias of specific cell type, leukemia of unspecified cell type, unspecified malignant neoplasms of lymphoid, hematopoietic and related tissues, such as diffuse large cell lymphoma, T-cell lymphoma, or cutaneous T-cell lymphoma); tumors of the excretory system (e.g., kidney, renal pelvis, ureter, bladder, and other urinary organs); tumors of the gastrointestinal tract (e.g., esophagus, stomach, small intestine, colon, colorectal, rectosigmoid junction, rectum, anus, and anal canal); tumors involving the liver and intrahepatic bile ducts, gall bladder, and other parts of the biliary tract, pancreas, and other digestive organs; tumors of the oral cavity (e.g., lip, tongue, gum, floor of mouth, palate, parotid gland, salivary glands, tonsil, oropharynx, nasopharynx, pyriform sinus, hypopharynx, and other sites of the oral cavity); tumors of the reproductive system (e.g., vulva, vagina, Cervix uteri, uterus, ovary, and other sites associated with female genital organs, placenta, penis, prostate, testis, and other sites associated with male genital organs); tumors of the respiratory tract (e.g., nasal cavity, middle ear, accessory sinuses, larynx, trachea, bronchus, and lung, such as small cell lung cancer and non-small cell lung cancer); tumors of the skeletal system (e.g., bone and articular cartilage of limbs, bone articular cartilage, and other sites); tumors of the skin (e.g., malignant melanoma of the skin, non-melanoma skin cancer, basal cell carcinoma of skin, squamous cell carcinoma of skin, mesothelioma, and Kaposi's sarcoma); and tumors involving other tissues, including peripheral nerves and autonomic nervous system, connective and soft tissue, retroperitoneum and peritoneum, eye and adnexa, thyroid, adrenal gland, and other endocrine glands and related structures, secondary and unspecified malignant neoplasms of lymph nodes, secondary malignant neoplasm of respiratory and digestive systems and secondary malignant neoplasms of other sites.

More specifically, the kits, compositions, and methods of the invention can be used to treat sarcomas. In some embodiments, the compositions and methods of the present invention are used in the treatment of bladder cancer, breast cancer, chronic lymphoma leukemia, head and neck cancer, endometrial cancer, non-flodgkin's lymphoma, non-small cell lung cancer, ovarian cancer, pancreatic cancer, and prostate cancer.

Tumors that can be advantageously treated using compositions and methods of the present invention include PTEN-deficient tumors (see, for example, M.S. Neshat et al., PNAS, 2001, 98: 10314-10319; K. Podsypanina et al., PNAS, 2001, 98: 101320-10325; G.B. Mills et al., PNAS, 2001, 98: 10031-10033; and M. Hidalgo and E.K. Rowinski, Oncogene, 2000, 19: 6680-6686). As already mentioned above, the FRAP/mTOR kinase is located downstream of the phosphatidyl inositol 3-kinase/Akt-signaling pathway, which is up-regulated in multiple cancers because of loss the PTEN tumor suppressor gene. PTEN-deficient tumors may be identified, using genotype analysis and/or in vitro culture and study ofbiopsied tumor samples. Non-limiting examples of cancers involving abnormalities in the phosphatidyl-inositol 3 kinase/Akt-mTOR pathway include, but are not limited to, glioma, lymphoma and tumors of the lung, bladder, ovary, endometrium, prostate, or cervix, which are associated with abnormal growth factor receptors (e.g., EGFR, PDGFR, IGF-R and IL-2); ovarian tumors which are associated with abnormalities in P13 kinase; melanoma and tumors of the breast, prostate, or endometrium which are associated with abnormalities in PTEN; breast, gastric, ovarian, pancreatic, and prostate cancers associated with abnormalities with Akt; lymphoma, cancers of the breast or bladder, and head and neck carcinoma associated with abnormalities in elF-4E; mantle cell lymphoma, breast cancer, and head and neck carcinomas associated with abnormalities in Cyclin D; and familial melanoma and pancreas carcinomas associated with abnormalities in P16.

The kits, compositions, and methods of the invention can also be used to treat diseases with aberrant angiogenesis, such as diabetic retinopathy, rheumatoid arthritis, psoriasis, atherosclerosis, chronic inflammation, obesity, macular degeneration, and a cardiovascular disease.

### Pharmaceutical Kits

A wide variety of other packaging choices are available for practicing the invention. The pharmaceutical kits of the invention include one or more containers (e.g., vials, ampoules, test tubes, flasks, or bottles) containing one or more of the ingredients of a pharmaceutical composition including compound 1 and/or an mTOR inhibit, allowing for the administration of the compound 1 alone or mTOR inhibitor and compound 1 together or concurrently. The kits optionally include instructions for the dosing, administration, and/or patient population being treated.

The different ingredients of a pharmaceutical package may be supplied in a solid (e.g., lyophilized) or liquid form. Each ingredient will generally be suitable as aliquoted in its respective container or provided in a concentrated form. Pharmaceutical packs or kits may include media for the reconstitution of lyophilized ingredients. The individual containers of the kit will preferably be maintained in close confinement for commercial sale.

Alternatively, compound 1 and the mTOR inhibitor are both formulated to be administered orally (e.g., kits containing compound 1 in unit dosage form for oral delivery and either ridaforolimus, sirolimus, or everolimus also in unit dosage form for oral delivery). Products formulated for oral administration, e.g., capsules, tablets, etc., may be packaged in blister packs, which can laid out and/or labeled in accordance with a selected dosing schedule.

The following examples are put forth so as to provide those of ordinary skill in the art with a complete disclosure and description of how the methods and compounds claimed herein are performed, made, and evaluated, and are intended to be purely exemplary of the invention and are not intended to limit the scope of what the inventors regard as their invention.

### Example 1: Inhibition of BCR-ABL and Mutants for Chronic

### Myelogenous Leukemia

### EXPERIMENTAL PROCEDURES

**Inhibitors:** Imatinib was dissolved in PBS to generate a 10.0 mM stock solution, distributed into 10 µL aliquots, and stored at-20 °C. Compound 1, nilotinib, and dasatinib were dissolved in DMSO to generate 10.0 mM stock solutions, distributed into 10 µL aliquots, and stored at -20 °C. Serial dilutions of 10.0 mM stock solutions were carried out just prior to use in each experiment. Compound 1 (3-(imidazo[1,2b]pyridazin-3-ylethynyl)-4-methyl-N-(4-((4-methylpiperazin-1-yl)methyl)-3-(trifluoromethyl)phenyl)benzamide) can be prepared as described herein.

**Crystallization and Structural Determination of ABL^{T315I}:Compound 1 Complex:** the kinase domain of murine ABL^{T315I} (residues 229-515) was co-expressed with YopH protein tyrosine phosphatase in E. coli and purified as previously reported (Ref). Purification of ABL^{T315I} was carried out in the presence of compound 1 to near homogeneity (> 95%) using a combination of metal affmity, Mono Q, and size exclusion chromatography columns. The typical yield of final purified ABL^{T315I} bound with compound 1 was about 1 mg/L. Co-crystals of ABL^{T315I} and compound 1 were grown by the hanging drop vapor diffusion method at 4 °C by mixing equal volumes of the compound 1:ABL^{T315I} complex (25 mg/mL) and well solution (30% w/v polyethylene 4000, 0.2 M sodium acetate, 0.1 M Tris-HCl, pH 8.5). After 1-2 days, crystals reached a typical size of 50 × 50 × 300 µm³ and were harvested in mother liquor supplemented with 30% v/v glycerol as cyroprotectant. X-ray diffraction data were collected at 100K at beamline 19 BM (Advanced Photon Service, Argonne, IL). The data were indexed and scaled in space group P21 by using HKL2000 package. The structure of compound 1 in complex with ABL^{T315I} was determined by molecular replacement by AMoRe using the structure of native ABL bound with imatinib (PDB code: 1IEP). There were two ABL^{T315I} molecules in the asymmetric unit. The structure was refined with CNX combined with manual rebuilding in Quanta (Accelrys Inc., San Diego, CA), and compound 1 was built into the density after several cycles of refinement and model building. Further refinement and model building were carried out until convergence was reached. The final model, refined to 1.95 A, consists of residues 228 through 511, except 386-397 in the activation loop, which are disordered. The electron density for the bound inhibitor compound 1 as well as the side chain of I315 was well resolved in both complexes, leaving no ambiguities for the binding mode of the inhibitor.

**Autophosphorylation Assays For ABL^{T315I}:** Kinase autophosphorylation assays with full length, tyrosine-dephosphorylated ABL and ABL^{T315I} (Invitrogen; San Diego, CA) were performed as previously described (O'Hare et al., Blood 104:2532 (2004)) in the presence of imatinib, nilotinib, dasatinib, or compound 1. The concentrations of inhibitor used were: 0, 0.1, 1, 10, 100, 1000 nM.

**Cell Lines:** Ba/F3 transfectants (expressing full-length, native BCR-ABL or BCR-ABL with a single kinase domain mutation) were maintained in RPMI 1640 supplemented with 10% FCS, 1 unit/mL penicillin G, and 1 mg/mL streptomycin (complete media) at 37 °C and 5% CO₂. The Ba/F3 cell line expressing BCR-ABL^{T315A} was a kind gift of Dr. Neil Shah, UCSF. Parental Ba/F3 cells were supplemented with IL-3, provided by WEHI-conditioned media. Prior to cell proliferation assays, RNA was isolated from each Ba/F3 cell line, and kinase domain mutations were confirmed by RT-PCR followed by DNA sequence analysis using Mutation Surveyor software (SoftGenetics, State College, PA).

**Cell Proliferation Assays:** Ba/F3 cell lines were distributed in 96-well plates (4 × 10³ cells/well) and incubated with escalating concentrations of compound 1 for 72 h. The concentrations of inhibitor used for IC₅₀ determinations in lines expressing either native or mutant BCR-ABL were: 0, 0.04, 0.2, 1, 5, 25, 125, and 625 nM. The concentrations of inhibitor used for IC₅₀ determinations in parental Ba/F3 cells were: 0,1, 5, 25, 125, 625, 3125, and 10,000 nM. Proliferation was measured using a methanethiosulfonate (MTS)-based viability assay (CellTiter96 Aqueous One Solution Reagent; Promega, Madison, WI). IC₅₀ values are reported as the mean of three independent experiments performed in quadruplicate. For cell proliferation experiments with CML or normal primary cells, mononuclear cells were isolated on Ficoll gradients (GE Healthcare) from peripheral blood of CML myelogenous blast crisis (M-BC) patients or from healthy individuals. Cells were plated in 96-well plates (5 × 10⁴ cells/well) over graded concentrations of compound 1 (0-1000 nM) in RPMT supplemented with 10% FBS, L-glutamine, penicillin/streptomycin, and 100 µM β-mercaptoethanol. Following a 72 h incubation, cell viability was assessed by subjecting cells to an MTS assay. All values were normalized to the control wells with no drug.

**CrkL Phosphorylation in Ba/F3 Cell Lines:** Ba/F3 cells expressing either native BCR-ABL or BCR-ABL^{T315I} (5 × 10⁶ per well) were cultured 4 h in RPMI supplemented with 10% FBS, L-glutamine, and penicillin/streptomycin in the absence of inhibitor or in the presence of imatinib (2000 nM), dasatinib (50 nM), nilotinib (500 nM), or compound 1 (0.1-1000 nM). Cells were lysed directly into boiling SDS-PAGE loading buffer supplemented with protease and phosphatase inhibitors. Lysates were subjected to SDS-PAGE and immunoblotted with anti-CrkL antibody C-20 (Santa Cruz). Phosphorylated and non-phosphorylated CrkL were distinguished based on differential band migration, and band signal intensities were quantified by densitometry on a Lumi Imager (Roche) and expressed as a % phosphorylated CrkL.

**Ex Vivo Exposure of DCR-ABL^{T315I} Patient Samples to Compound 1:** After obtaining informed consent, peripheral blood mononuclear cells from a patient with CML in lymphoid blast crisis (CML L-BC) with a BCR-ABL^{T315I} mutation were isolated by Ficoll centrifugation. RT-PCR and sequencing analysis confirmed that the sample predominantly contained the BCR-ABL^{T315I} mutant. Mononuclear cells (5 × 10⁶ cells/well) were cultured overnight in serum-free IMDM media (Invitrogen) supplemented with 20% BIT (StemCell), 40 µg/mL human low-density lipoprotein, and 100 µM β-mercaptoethanol in the absence of inhibitor or in the presence of imatinib (1000 nM), dasatinib (50 nM), nilotinib (200 nM), or compound 1 (50 nM, 500 nM). Cells were lysed directly into boiling SDS-PAGE loading buffer supplemented with protease and phosphatase inhibitors. Lysates were subjected to SDS-PAGE and immunoblotted with anti-CrkL antibody C-20 (Santa Cruz). Phosphorylated and non-phosphorylated CrkL were distinguished based on differential band migration. Band signal intensities were quantified by densitometry on a Lumi Imager (Roche).

**Global Tyrosine Phosphorylation by FACS:** Mononuclear cells (2 × 10⁵) were cultured overnight in serum-free media in absence of inhibitor or in the presence of imatinib (1000 nM), dasatinib (50 nM), nilotinib (200 nM), or graded concentrations of compound 1 (50, 500 nM). Cells were fixed and permeabilized according to the manufacturer's instructions (Caltag; San Diego, CA), incubated with 2 µg of anti-phosphotyrosine 4G10-FITC antibody (BD Biosciences, San Jose, CA) for 1 hr, washed twice with PBS supplemented with 1% BSA and 0.1 % sodium azide, and fixed in 1% formaldehyde. FITC signal intensity was analyzed on a FACSAria instrument (BD) and mean fluorescence intensity (MFI) was calculated. Values are reported as fold increase in MFI relative to unstained controls.

**Hematopoietic Colony Forming Assays of Primary CML Cells and Normal Bone Marrow:** To assess the effect of compound 1 against primary CML cells harboring BCR-ABL^{T315I} and normal hematopoietic progenitors, bone marrow mononuclear cells isolated by Ficoll density centrifugation were cultured with graded concentrations of compound 1 (CML patient: 0, 10, 25, 50 nM; healthy individual: 0, 100, 200, 500, 1000 nM). Cells were plated in triplicate (5 × 10⁴ cells/plate) in 1 mL of IMDM:methylcellulose media (1:9 v/v) containing 50 ng/mL SCF, 10 ng/mL GM-CSF, and 10 ng/mL IL-3 (Methocult GF H4534; Stem Cell Technologies, Vancouver, British Columbia, Canada) to assess granulocyte/macrophage colony formation (CFU-GM). Cells were cultured at 37°C in a humidified incubator for 14-18 days. Colonies were counted with >50 cells/colony as the criterion for positive colony scoring. Results are reported as the percentage of colonies relative to untreated control ± SEM.

**Pharmacokinetics:** The pharmacokinetic profile of compound 1 (in citrate buffer, pH 2.74) was assessed in CD-1 female mice after a single dose administered by oral gavage. Blood samples were collected at various time points and compound 1 concentrations in plasma determined by an internal standard LC/MS/MS method using protein precipitation and calibration standards prepared in blank mouse plasma. Reported concentrations are average values from 3-mice/time point/dose group.

**Ba/F3 survival model:** Ba/F3 cells expressing native BCR-ABL or BCR-ABL^{T315I} were injected into the tail vein of female SCID mice (100 µL of a 1 × 10⁷ cells/mL suspension in serum-free medium). Beginning 72 hours later mice were treated once daily by oral gavage with vehicle (25 mM citrate buffer, pH 2.75), compound 1, or dasatinib for up to 19 consecutive days. Animals were sacrificed when they became moribund as per IACUC guidelines, and evaluation of mice at necropsy was consistent with death due to splenomegaly caused by tumor cell infiltration. The survival data was analyzed using Kaplan-Meier method, and statistical significance was evaluated with a Log-rank test (GraphPad PRISM) by comparing the survival time of each treatment group with the vehicle group. A value of p<0.05 was considered to be statistically significant and p<0.01 1 to be highly statistically significant.

**Ba/F3 Tumor Model:** Ba/F3 cells expressing BCR-ABL^{T315I} were implanted subcutaneously into the right flank of female nude mice (100 µL of a 1 × 10⁷ cells/mL cell suspension in serum-free medium). For analysis of efficacy, mice were randomly assigned to different treatment groups when the average tumor volume reached approximately 500 mm³. Mice were treated once daily by oral gavage with vehicle (25 mM citrate buffer, pH 2.75) or compound 1 for up to 19 consecutive days. Tumor volume (mm³) was calculated using the following formula: tumor volume = L x W² x 0.5. To determine tumor growth inhibition when the treatment period was finished, the percent change in tumor volume was calculated for all animals using the formula ΔV = (T_{final} - Tᵢₙᵢₜᵢₐₗ) / Tᵢₙᵢₜᵢₐₗ × 100, where Iᵢₙᵢₜᵢₐₗ was the tumor volume at the start of treatment and T_{final} was the volume at the time the animal was sacrificed. The mean tumor volume change of each treatment group was compared to all other groups using a one-way ANOVA test (GraphPad PRISM) and to that of vehicle-treated mice for statistical significance using Dunnett's test, where a value ofp<0.05 was considered to be statistically significant and p<0.01 to be highly statistically significant. For analysis of tyrosine-phosphorylated BCR-ABL and CrkL levels, tumor-bearing animals (average tumor size: 500 mm³) were treated with a single dose of either vehicle or 30 mg/kg compound 1 by oral gavage. Six hours after dosing mice (N=3/group), animals were sacrificed and tumor samples collected for Western blot analysis with antibodies against pBCR-ABL and eIF4E (Cell Signaling Technology) and total CrkL (C-20; Santa Cruz).

**Accelerated cell-based mutagenesis screen with Single-agent Compound 1:** Ba/F3 cells expressing native BCR-ABL were treated overnight with N-ethyl-N-nitrosourea (ENU; 50 µg/mL), pelleted, resuspended in fresh media, and distributed into 96-well plates at a density of 1 × 10⁵ cells/well in 200 µL complete media supplemented with graded concentrations of compound 1. The wells were observed for cell growth by visual inspection under an inverted microscope and media color change every two days throughout the course of the 28-day experiment. The contents of wells in which cell outgrowth was observed were transferred to a 24-well plate containing 2 mL complete media supplemented with compound 1 at the same concentration as in the initial 96-well plate. If growth was simultaneously observed in all wells of a given condition, 24 representative wells were expanded for further analysis. At confluency, cells in 24-well plates were collected by centrifugation. DNA was extracted from the cell pellets using a DNEasy Tissue kit (QIAGEN, Inc., Valencia, CA). The BCR-ABL kinase domain was amplified using primers B2A (5' TTCAGAAGCTTCTCCCTGACAT 3') and ABL4317R (5' AGCTCTCCTGGAGGTCCTC 3'), PCR products were bi-directionally sequenced by a commercial contractor (Agencourt Bioscience Corporation, Beverly, MA) using primers ABL3335F (5 ' ACCACGCTCCATTATCCAGCC 3') and ABL4275R (5' CCTGCAGCAAGGTAGTCA 3'), and the chromatograms were analyzed for mutations using Mutation Surveyor software (SoftGenetics, State College, PA). Results from this screen are reported as the cumulative data from three independent experiments (see Table 2). The mutagenesis screen was also conducted as described above for single-agent compound 1 starting with Ba/F3 cells expressing BCR-ABL^{T315I} (see Table 3) or BCR-ABL^{E255V} (see Table 4) in single independent experiments.

### RESULTS

### (1) X-ray Crystallographic Analysis of Compound 1 in Complex with ABL^{T315I}.

Recent X-ray crystallographic studies have revealed that the T315I mutation in the kinase domain of ABL mutant acts as a simple point mutant preventing imatinib, nilotinib, and dasatinib each from forming the hydrogen bond otherwise made with the side chain of T315 in native ABL. Compound 1's DFG-out mode of binding and an overall network of protein contacts is similar to that of imatinib, except for at least one important distinction: the ethynyl linkage in compound 1 positions the molecule to avoid the steric clash seen with the other inhibitors and permits productive van der Waals interactions with I315.

### (2) Compound 1 Inhibits the Catalytic Activity of ABL^{T315I}.

We tested the activity of compound 1 in comparison with imatinib, nilotinib and dasatinib in biochemical assays with purified, dephosphorylated, full-length native ABL kinase and ABL^{T315I} kinase proteins. While each of the inhibitors diminished the enzymatic activity of native ABL, only compound 1 was effective against the ABL^{T315I} mutant, as measured by vitro [γ-³²P]-ATP autophosphorylation of full-length ABL^{T315I} kinase. Similar potent inhibition by compound 1 was observed for additional clinically relevant imatinib-resistant ABL mutants tested, including ABL^{G250E}, ABL^{Y253F}, and ABL^{E255K}. These results establish that compound 1 directly targets native and kinase domain mutant ABL kinase, including the ABL^{T315I} kinase mutant.

### (3) Compound 1 Inhibits the Growth of Ba/F3 Cells Expressing Native or Mutant BCR-ABL, Including BCR-ABL^{T315I}.

Cellular proliferation assays were performed with parental Ba/F3 cells and Ba/F3 cells expressing native BCR-ABL or BCR-ABL with a single mutation in the kinase domain (M244V, G250E, Q252H, Y253F, Y253H, E255K, E255V, T315A, T315I, F317L, F317V, M351T, F359V, or H396P). Compound 1 potently inhibited proliferation of Ba/F3 cells expressing native BCR-ABL (IC₅₀: 0.5 nM). Notably, all BCR-ABL mutants tested remained sensitive to compound 1 (IC₅₀: 0.5-36 nM; Table 1) including the BCR-ABL^{T315I} mutant (IC₅₀: 11 nM). Staining with Annexin V showed that inhibition of proliferation by compound 1 was correlated with induction of apoptosis (data not shown). Growth inhibition of parental Ba/F3 cells did not reach an IC₅₀ until a concentration of compound 1 of 1713 nM, indicating that inhibitory effects are linked to BCR-ABL inhibition.

We also tested compound 1 against a panel of patient-derived BCR-ABL-positive and -negative cell lines. While we observed potent growth inhibition of K562, KY01, and LAMA cells (derived from CML patients in blast crisis), there was no significant activity against three different BCR-ABL-negative leukemia cell lines, where the IC₅₀ was comparable to or greater than that of Ba/F3 parental cells (Table 1).

**Table 1. IC₅₀ values for Compound 1 in cellular proliferation assays.**

| Cell lines | | AP24534 IC₅₀ (nM) |
|---|---|---|
| Ba/F3 cells | | |
| | Native BCR-ABL | 0.5 |
| | M244V | 2.2 |
| | G250E | 4.1 |
| | Q252H | 2.2 |
| | Y253F | 2.8 |
| | Y253H | 6.2 |
| | E255K | 14 |
| | E255V | 36 |
| | T315A | 1.6 |
| | T315I | 11 |
| | F317L | 1.1 |
| | F317V | 10 |
| | M351T | 1.5 |
| | F359V | 10 |
| | H396P | 1.1 |
| | Parental | 1713 |

| CML leukemia cells | | |
|---|---|---|
| | K562 | 3.9 |
| | KYO1 | 0.4 |
| | LAMA | 0.3 |

| Non-CML leukemia cells | | |
|---|---|---|
| | Marimo | 2215 |
| | HEL | 2522 |
| | CMK | 1652 |

### (4) Compound 1 Inhibits BCR-ABL-Mediated Signaling in Cells Expressing BCR-ABL^{T315I}.

To confirm target inhibition in Ba/F3 cells expressing native BCR-ABL or BCR-ABL^{T315I}, we examined the tyrosine phosphorylation status of BCR-ABL and the direct BCR-ABL substrate CrkL (Figure 1). Monitoring CrkL tyrosine phosphorylation status provides a convenient means of assessing BCR-ABL kinase activity in primary human cells, and is the preferred pharmacodynamic assay in CML clinical trials involving new BCR-ABL (Druker et al., N Engl J Med 344:1031 (2001); Talpaz et al., N Engl J Med 354:2531 (2006)), since direct measurement of phosphorylated BCR-ABL tyrosine phosphorylation status is not feasible in primary cell lysates due to proteolytic lability. For comparison, the clinical ABL inhibitors imatinib, nilotinib, and dasatinib were included. In the CrkL gel shift assay, the percentage of tyrosine-phosphorylated CrkL decreases in direct response to inhibition of BCR-ABL. While all of the tested inhibitors were effective against Ba/F3 cells expressing native BCR-ABL (Figure 1A), only compound 1 demonstrated activity against the T315I mutant (Figure 1B). Inhibition of BCR-ABL phosphorylation was observed in parallel experiments in Ba/F3 cells expressing native BCR-ABL or BCR-ABL^{T315I}. BCR-ABL phosphorylation was evaluated in Ba/F3 cells expressing either native BCR-ABL or BCR-ABL^{T315I} treated overnight with imatinib, nilotinib, dasatinib, or compound 1. Samples were analyzed by immunoblot analysis with antibodies against pBCR-ABL and eIF4E (loading control).

### (5) Treatment of CML Primary Cells with Compound 1 Inhibits Cellular Proliferation.

To assess the efficacy of compound 1 on primary cells derived from patients with BCR-ABL-driven leukemia, we exposed mononuclear cells from CML myelogenous blast crisis patients, or from healthy individuals, to graded concentrations of compound 1 and assayed viable cells after 72 hours. Consistent with biochemical and cell line viability data, compound 1 induced a selective reduction of viable cell numbers with IC₅₀ values approximately 500-fold lower in primary CML cells compared with normal cells (Figure 2A).

### (6) Compound 1 Inhibits BCR-ABL^{T315I} Kinase Activity and Colony Formation in Primary CML Cells With Minimal Toxicity to Normal Cells.

To assess target inhibition following ex vivo exposure to compound 1 of mononuclear cells obtained from a CML lymphoid blast crisis patient with a T315I mutation, we carried out an assay similar to the one described for Ba/F3 cell lines, wherein cells were incubated overnight in the presence of inhibitors, harvested and lysed, and analyzed for CrkL phosphorylation by immunoblot. Exposure to compound 1 resulted in a reduction in phosphorylated CrkL signal while none of the other three clinical ABL inhibitors showed any effect (Figure 2B), and similar results were obtained upon analysis of cells from this patient for global tyrosine phosphorylation by FACS (Figure 2C).

We also evaluated the efficacy of compound 1 in myelogenous colony formation assays using mononuclear cells from a CML accelerated phase patient harboring BCR-ABL^{T315I} and from a healthy individual. Cells were plated in methylcellulose in the presence of inhibitor, cultured for approximately 14-18 days, and counted under an inverted microscope. Whereas neither nilotinib nor dasatinib showed any effect against cells from the T315I patient, compound 1 inhibited the formation of colonies in a concentration-dependent manner (Figure 3A). By contrast, compound 1 showed no toxicity to normal hematopoetic cells at concentrations below 500 nM (Figure 3B), which was consistent with cellular proliferation assays performed using normal cells (Figure 2A).

### (7) Oral Compound 1 Prolongs Survival and Reduces Tumor Burden in Mice with BCR-ABL^{T3I5I}-Dependent Disease.

To examine the in vivo pharmacokinetic profile of compound 1, CD-1 mice were administered a single dose of compound 1 (either 2.5 or 30 mg/kg) by oral gavage, and plasma concentrations of compound 1 were measured by LC/MS/MS at 2, 6, and 24 h post-dose. Compound 1 was orally bioavailable, with mice treated with a dose of 2.5 mg/kg achieving mean plasma levels of 89.6, 58.2, and 1.9 nM at 2, 6, and 24 h, respectively. At an increased dose of 30 mg/kg, mean plasma levels reached 781.7, 561.3, and 7.9 nM at 2, 6, and 24 h, respectively. Dose-exposure proportionality was observed between the 2.5 mg/kg (AUC₀₋₂₄ₕ: 767 nmol·h/mL) and 30 mg/kg (AUC₀₋₂₄ₕ: 7452 nmol·h/mL) doses. Together, these data demonstrate that compound 1 blood levels exceeding the in vitro IC₅₀ values for all tested BCR-ABL mutants can be sustained for several hours with modest oral doses.

We next evaluated the in vivo activity of compound 1 in several well-established mouse models of CML. First, activity was examined in a survival model in which Ba/F3 cells expressing native BCR-ABL were injected intravenously into the tail veins of mice. As shown in Figure 4A , treatment with either compound 1 or dasatinib prolonged survival compared to a median survival of 19 days for vehicle-treated mice. A daily oral dose of 5-mg/kg dasatinib, which has been reported to be an efficacious regimen (Lombardo et al., J Med Chem 47:6658 (2004)), prolonged median survival to 27 days (p<0.01). Similarly, daily oral doses of 2.5 and 5 mg/kg compound 1 prolonged median survival time to 27.5 and 30 days, respectively (p<0.01 for both dose levels).

The activity of compound 1 was subsequently evaluated in this same survival model using Ba/F3 cells expressing BCR-ABL^{T315I}. In comparison to vehicle-treated mice, which had a median survival of 16 days, compound 1 treatment (for up to 19 days) prolonged survival in a dose-dependent manner (Figure 4B). While daily oral dosing of 2.5 mg/kg compound 1 increased median survival by only 0.5 days (p>0.05), compound 1 dosed at 5, 15, and 25 mg/kg significantly prolonged median survival to 19.5, 26, and 30 days, respectively (p<0.01 for all three dose levels). By contrast, an independent parallel study using this T315I survival model confirmed no difference in median survival between mice treated with vehicle or dasatinib (Figure 5).

The anti-tumor activity of compound 1 was further assessed in a xenograft model in which Ba/F3 cells expressing BCR-ABL^{T315I} were injected subcutaneously into mice. Tumor growth was inhibited by compound 1 in a dose-dependent manner (Figure 4C) compared to vehicle treated mice, with significant suppression of tumor growth upon daily oral dosing at 10 and 30 mg/kg (%T/C = 68% and 20%, respectively; p<0.01 for both dose levels). Daily oral dosing of 50 mg/kg compound 1 caused significant tumor regression (%T/C = 0.9%, p<0.01), with a 96% reduction in mean tumor volume at the final measurement compared to the start of treatment. To confirm target inhibition, levels of phosphorylated BCR-ABL^{T315I} and phosphorylated CrkL were assessed in tumors from mice harvested 6 hr after one-time dosing with vehicle or compound 1. As shown in Figure 4C, a single oral dose of 30 mg/kg markedly decreased levels of phosphorylated BCR-ABL and phosphorylated CrkL.

### (8) Single-Agent Compound 1 is Sufficient to Completely Suppress Outgrowth of Resistant Subclones.

To survey for potential sites of vulnerability to resistance not probed in the cell proliferation Ba/F3 panel, especially compound 1-specific mutations (for example, at inhibitor-enzyme contact residues) and to assess whether compound 1 offers an advantage over other single-agent inhibitors, we tested this compound in our established accelerated mutagenesis assay, which we have previously validated for imatinib, nilotinib, and dasatinib.

In a set of experiments starting from Ba/F3 cells expressing native BCR-ABL, we established the resistance profile at several concentrations of compound 1 (5-40 nM) and found a concentration-dependent reduction in both the percentage of wells with outgrowth and in the scope of mutations observed (Figure 6A). At 5 nM compound 1, all wells (576/576) exhibited outgrowth and 90% of the sequenced representative subclones expressed native BCR-ABL (Table 2). Raising the concentration of compound 1 to 10 nM resulted in both a marked reduction in outgrowth (168/1440 wells; 11.7%) and an increased frequency of mutated subclones (33.1%; Table 2). Mutations recovered included occurrences at several P-loop residues (G250, Q252, Y253, and E255), a cluster at or near the C-helix (K285, E292, and L298), and T315 (T315I), F317, V339, F359, L387, and S438. Among the recovered mutations, nearly all have been previously encountered in imatinib resistance (or nilotinib or dasatinib resistance) (reviewed in O'Hare et al., Blood 110:2242 (2007)). No novel mutations were encountered that were specific for compound 1. Positions Y253, T315, and F317 are contact residues, and K285 is adjacent to a key hydrogen-bond contributor, E286.

Since mutations persisting at a concentration that completely suppresses T315I are likely to represent the key concerns for resistance to compound 1, we next investigated 20 nM compound 1 and found that outgrowth was sharply curtailed (3/1440 wells; 0.2%; Figure 6A, Table 2), with only two mutations, E255V and T315I persisting. Thus, within our extensive survey, no previously undiscovered mutations capable of conferring high-level resistance to compound 1 were identified. At 40 nM compound 1, which is more than 40-fold lower than the IC₅₀ for parental BaF/3 cells, complete suppression of in vitro resistance was achieved. This absence of resistant outgrowth was further confirmed at higher concentrations of compound 1 (80, 160, 320 nM; data not shown). To our knowledge, no other single-agent BCR-ABL inhibitor has been shown to have this capability.

**Table 2. Compound 1 cell-based mutagenesis assay starting from native BCR-ABL**

| Ba/F3 cells expressing native BCR-ABL | | | | | | | | | | |
|---|---|---|---|---|---|---|---|---|---|---|
| | | | | By specific mutation | | | | By residue | | |
| Concentration | Wells surveyed | Wells with outgrowth | Clones sequenced (N) | Mutant(s) | Occurrences (n) | Frequency among clones (%) | Frequency among mutants (%) | Residue | Occurrences (n) | Frequency by residue (%) |
| 5 nM | 576 | 576 | 51 | Native BCR-ABL | 46 | 90.2 | --- | --- | --- | --- |
| | | | | G250E | 1 | 2.0 | 20.0 | G250 | 1 | 20.0 |
| | | | | Y253H | 1 | 2.0 | 20.0 | Y253 | 1 | 20.0 |
| | | | | E255K | 1 | 2.0 | 20.0 | E255 | 1 | 20.0 |
| | | | | T315I | 1 | 2.0 | 20.0 | T315 | 1 | 20.0 |
| | | | | F3171 | 1 | 2.0 | 20.0 | F317 | 1 | 20.0 |
| 10 nM | 1440 | 168 | 157 | Native BCR-ABL | 105 | 66.9 | --- | --- | --- | --- |
| | | | | G250E | 1 | 0.6 | 1.9 | G250 | 1 | 1.9 |
| | | | | Q252H | 4 | 2.5 | 7.7 | Q252 | 4 | 7.7 |
| | | | | Y253F | 1 | 0.6 | 1.9 | Y253 | 7 | 13.5 |
| | | | | Y253H | 6 | 3.8 | 11.5 | | | |
| | | | | E255K | 12 | 7.6 | 23.1 | E255 19 E255 | 19 | 36.5 |
| | | | | E255V | 7 | 4.5 | 13.5 | | | |
| | | | | K285N | 1 | 0.6 | 1.9 | K285 | 1 | 1.9 |
| | | | | E292V | 1 | 0.6 | 1.9 | E292 | 1 | 1.9 |
| | | | | L298V | 2 | 1.3 | 3.8 | L298 | 2 | 3.8 |
| | | | | T315I | 7 | 4.5 | 13.5 | T315 | 7 | 13.5 |
| | | | | F317I | 1 | 0.6 | 1.9 | F317 | 1 | 1.9 |
| | | | | V339G | 1 | 0.6 | 1.9 | V339 | 1 | 1.9 |
| | | | | F359C | 2 | 1.3 | 3.8 | F359 | 5 | 9.6 |
| | | | | F359I | 3 | 1.9 | 5.8 | | | |
| | | | | L387F | 2 | 1.3 | 3.8 | L387 | 2 | 3.8 |
| | | | | S438C | 1 | 0.6 | 1.9 | S438 | 1 | 1.9 |
| | | | | | | | | | | |
| 20 nM | 1440 | 3 | 3 | E255V | 1 | 33.3 | 33.3 | E255 | 1 | 33.3 |
| | | | | T315I | 2 | 66.7 | 100.0 | T315 | 2 | 66.7 |
| | | | | | | | | | | |
| 40 nM | 1440 | 0 | 0 | --- | --- | --- | --- | --- | --- | --- |

### (9) Effects of Compound 1 on Compound Mutants.

As compound 1 therapy is likely to be tested in the setting of failure of imatinib and at least one salvage therapy (such as one of the FDA-approved second-line ABL kinase inhibitors), there is considerable potential for pre-existence of a T315I or other resistance-conferring mutation. Although so far rare and documented only in a small number of cases, patients can also fail with a compound BCR-ABL mutation involving a secondary kinase domain mutation in conjunction with a pre-existing mutation in the same allele (Khorashad et al., Blood 111:2378 (2008); Shah et al., J Clin Invest 117:2562 (2007); Stagno et al., Leuk Res 32:673 (2008)). Having found a very limited resistance susceptibility profile at the level of single kinase domain mutations, we wanted to investigate vulnerability of compound 1 to compound mutations.

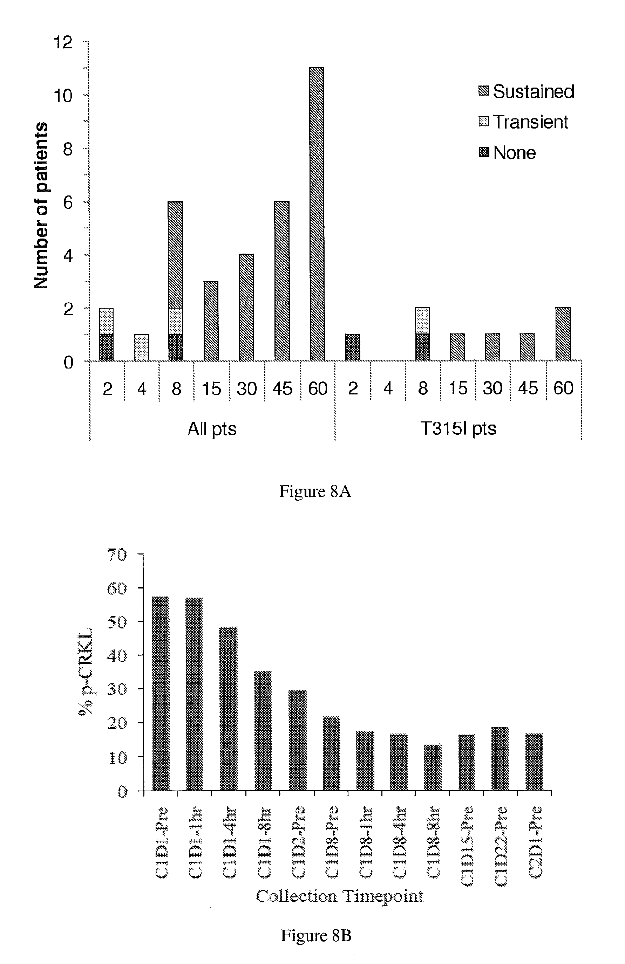

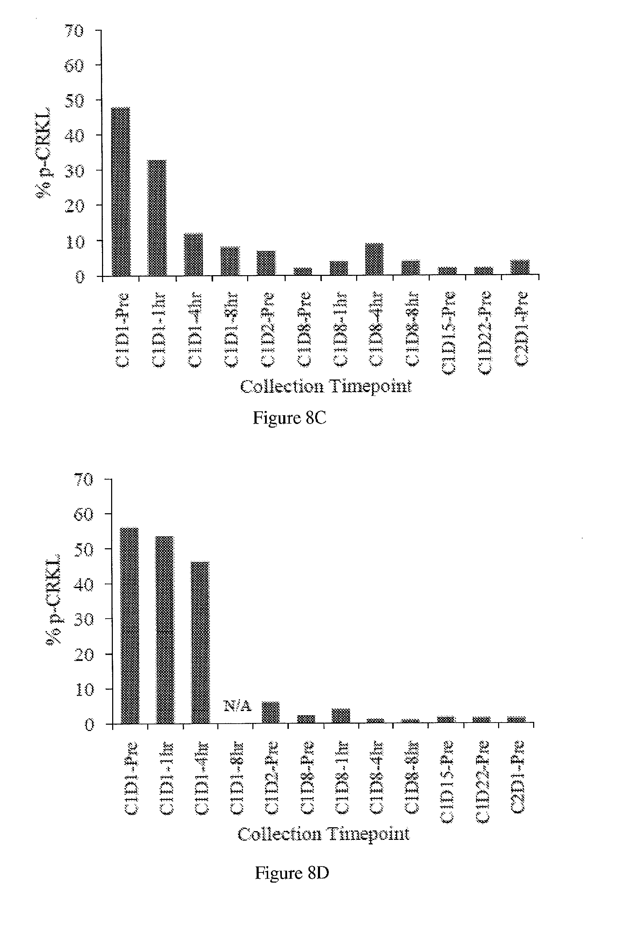

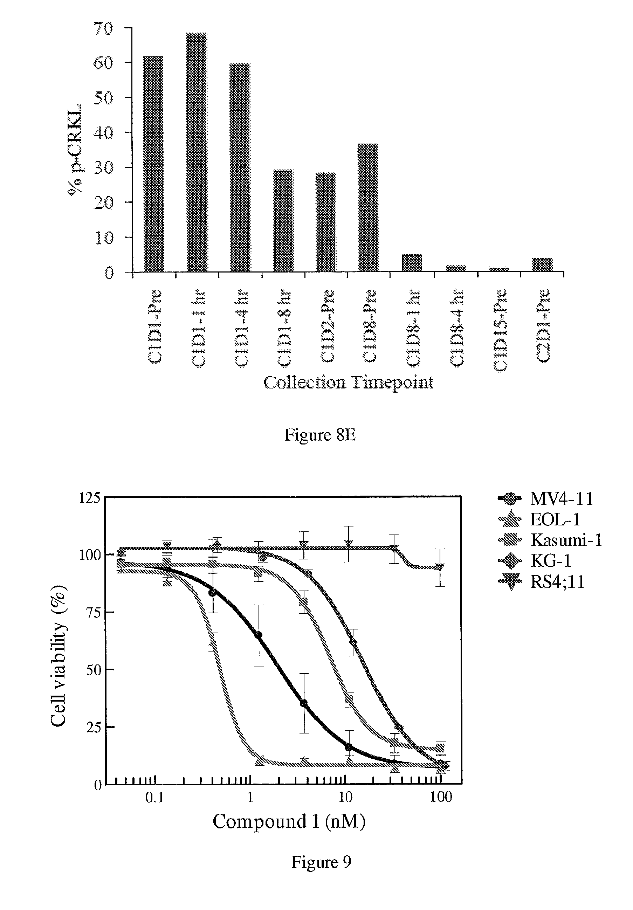

To simulate a situation in which compound 1 is used to treat a patient with a predominant T315I subclone, we again conducted the accelerated mutagenesis assay, this time starting on the background of an existing T315I mutation (Figure 8B and Table 3). We found that there was still a concentration-dependent hierarchy and that the inhibitor could still control all tested compound mutants. All compound mutants except Y253H/T315I and E255V/T315I were eliminated at a concentration of 160 nM compound 1. At 320 nM, the only remaining compound mutant was E255V/T315I, which couples the two most resistant single mutants, and outgrowth was completely suppressed at the highest tested concentration (640 nM), still almost 3-fold below the IC₅₀ for parental Ba/F3 cells. This resistance profile was confirmed in a subsequent screen starting from a background of BCR-ABL^{E255V}, the most resistant single BCR-ABL kinase domain mutation to compound 1, with the E255V/T315I compound mutant persisting to 320 nM and eliminated at 640 nM (Table 4).

**Table 3. Compound 1 cell-based mutagenesis assay starting from BCR-ABL^{T315I}**

| Ba/F3 cells expressing BCR-ABL^{T315I} | | | | | | | | | | |
|---|---|---|---|---|---|---|---|---|---|---|
| | | | | By specific compound mutation (with T315I) | | | | By residue | | |
| Concentration | Wells surveyed | Wells with outgrowth | Clones sequenced (N) | Mutant(s) | Occurrences (n) | Frequency among clones (%) | Frequency among mutants (%) | Residue | Occurrences (n) | Frequency by residue (%) |
| 10 nm | 480 | 480 | 10 | T315I only | 9 | 90.0 | --- | --- | --- | --- |
| | | | | A365V | 1 | 10.0 | 100.0 | A365 | 1 | 100.0 |
| | | | | | | | | | | |
| 20nM | 480 | 480 | 20 | T315I only | 20 | 100.0 | --- | --- | --- | --- |
| | | | | | | | | | | |
| 40nM | 480 | 192 | 140 | T3151 only | 6 | 4.3 | --- | --- | --- | --- |
| | | | | G250E | 3 | 2.1 | 2.2 | G250 | 3 | 2.2 |
| | | | | Q252H | 5 | 3.6 | 3.7 | Q252 | 5 | 3.7 |
| | | | | Y253F | 3 | 2.1 | 2.2 | Y253 | 46 | 34.3 |
| | | | | Y253H | 41 | 29.3 | 30.6 | | | |
| | | | | Y253N | 2 | 1.4 | 1.5 | | | |
| | | | | E255K | 7 | 5.0 | 5.2 | E255 | 12 | 9.0 |
| | | | | E255V | 5 | 3.6 | 3.7 | | | |
| | | | | E281K | 1 | 0.7 | 0.7 | E281 | 1 | 0.7 |
| | | | | K285N | 2 | 1.4 | 1.5 | K285 | 2 | 1.5 |
| | | | | I293N | 4 | 2.9 | 3.0 | N293 | 4 | 3.0 |
| | | | | F311I | 24 | 17.1 | 17.9 | F311 | 39 | 29.1 |
| | | | | F311V | 15 | 10.7 | 11.2 | | | |
| | | | | I315L | 3 | 2.1 | 2.2 | I315 | 4 | 3.0 |
| | | | | I315M | 1 | 0.7 | 0.7 | | | |
| | | | | L327M | 1 | 0.7 | 0.7 | L327 | 1 | 0.7 |
| | | | | F359C | 7 | 5.0 | 5.2 | F359 | 9 | 6.7 |
| | | | | F359I | 1 | 0.7 | 0.7 | | | |
| | | | | F359V | 1 | 0.7 | 0.7 | | | |
| | | | | A380S | 3 | 2.1 | 2.2 | A380 | 3 | 2.2 |
| | | | | H396P | 4 | 2.9 | 3.0 | H396 | 5 | 3.7 |
| | | | | H396R | 1 | 0.7 | 0.7 | | | |
| | | | | | | | | | | |
| 80nM | 480 | 75 | 71 | Q252H | 3 | 4.2 | 4.2 | Q252 | 3 | 4.2 |
| | | | | Y253H | 51 | 71.8 | 71.8 | Y253 | 51 | 71.8 |
| | | | | E255K | 8 | 11.3 | 11.3 | E255 | 8 | 11.3 |
| | | | | F311I | 2 | 2.8 | 2.8 | F311 | 3 | 4.2 |
| | | | | F311V | 1 | 1.4 | 1.4 | | | |
| | | | | 1315L | 3 | 4.2 | 4.2 | I315 | 3 | 4.2 |
| | | | | A380S | 3 | 4.2 | 4.2 | A380 | 3 | 4.2 |
| | | | | | | | | | | |
| 160 nM | 480 | 42 | 32 | Y253H | 29 | 90.6 | 90.6 | Y253 | 29 | 90.6 |
| | | | | E255V | 3 | 9.4 | 9.4 | E255 | 3 | 9.4 |
| | | | | | | | | | | |
| 320 nM | 480 | 1 | 1 | E255V | 1 | 100.0 | 100.0 | E255 | 1 | 100.0 |
| | | | | | | | | | | |
| 640 nM | 480 | 0 | 0 | --- | --- | --- | --- | --- | --- | -- |

**Table 4. Compound 1 cell-based mutagenesis assay starting from BCR-ABL^{E255V}**

| Ba/F3 cells expressing BCR-ABL^{E255V} | | | | | | | | | | |
|---|---|---|---|---|---|---|---|---|---|---|
| | | | | By specific compound mutation (with E255V) | | | | By residue | | |
| Concentration | Wells surveyed | Wells with outgrowth | Clones sequenced (N) | Mutant(s) | Occurrences (n) | Frequency among clones (%) | Frequency among mutants (%) | Residue | Occurrences (n) | Frequency by residue (%) |
| 80 nM | 480 | 152 | 123 | E255V only | 104 | 84.6 | --- | --- | --- | --- |
| | | | | G250E | 2 | 1.6 | 10.5 | G250 | 2 | 10.5 |
| | | | | Q252H | 1 | 0.8 | 5.3 | Q252 | 1 | 5.3 |
| | | | | Y253H | 5 | 4.1 | 26.3 | Y253 | 5 | 26.3 |
| | | | | E292V | 1 | 0.8 | 5.3 | E292 | 1 | 5.3 |
| | | | | F311I | 2 | 1.6 | 10.5 | F311 | 2 | 10.5 |
| | | | | T315I | 1 | 0.8 | 5.3 | T315 | 1 | 5.3 |
| | | | | E355G | 1 | 0.8 | 5.3 | E355 | 1 | 5.3 |
| | | | | F359C | 3 | 2.4 | 15.8 | F359 | 5 | 26.3 |
| | | | | F359I | 2 | 1.6 | 10.5 | | | |
| | | | | H396R | 1 | 0.8 | 5.3 | H396 | 1 | 5.3 |
| | | | | | | | | | | |
| 160 nM | 480 | 9 | 6 | Y253F | 1 | 16.7 | 16.7 | Y253 | 3 | 50.0 |
| | | | | Y253H | 2 | 33.3 | 33.3 | | | |
| | | | | T315I | 3 | 50.0 | 50.0 | T315 | 3 | 50.0 |
| | | | | | | | | | | |
| 320 nM | 480 | 1 | 1 | T315I | 1 | 100.0 | 100.0 | T315 | 1 | 100.0 |
| | | | | | | | | | | |
| 640 nM | 480 | 0 | 0 | --- | --- | --- | --- | --- | --- | --- |

### DISCUSSION

Compound I is an ABL kinase inhibitor that binds to the inactive, DFG-out conformation of the kinase domain of ABL and ABL^{T315I} and features a carbon-carbon triple bond linkage proximal to the T315I mutation. X-ray crystallographic studies confirmed that compound 1 binds to ABL^{T315I} in the DFG-out binding mode. Compound 1 maintained an extensive hydrogen-bonding network, and also occupied a region of the kinase that overlaps significantly with the binding site of imatinib. Compound 1 formed five hydrogen bonds to the kinase, together with numerous van der Waals contacts, resulting in potent inhibition of the kinase (ABL^{T315I} IC₅₀: 2.0 nM; native ABL IC₅₀: 0.37 nM). Additionally, the triple bond itself is optimally positioned to make productive hydrophobic contact with the side chain of I315, while its linear-shape and rigid geometry enforce a conformational constraint avoiding steric clashes and acting as an inflexible connector that positions the other two sectors of compound 1 into their established binding pockets.

Evaluation of compound 1 in cellular proliferation assays confirmed its potent pan-BCR-ABL inhibition against cells expressing native or kinase domain mutant BCR-ABL, including BCR-ABL^{T315I}, as well as a high degree of selectivity for Philadelphia chromosome (Ph)-positive cells over Ph-negative cells (Table 1). In Ba/F3 cells, this amounted to a greater than 3000-fold differential in sensitivity between cells expressing native BCR-ABL and parental cells (native IC₅₀: 0.5 nM; parental IC₅₀: 1713 nM). Findings were congruous for primary CML cells versus normal cells treated ex vivo with compound 1 in cellular assays (Figure 2A) as well as in hematopoetic colony formation assays (Figure 3). Among the BCR-ABL kinase domain mutants tested, the E255V mutant was most resistant to compound 1 (IC₅₀: 36 nM), and this mutation has been reported to confer high-level resistance to imatinib and intermediate-level resistance to both nilotinib and dasatinib (O'Hare et al., Blood 110:2242 (2007)). Notably, however, mutations at residues Y253 and F359 (which have been reported at the time of nilotinib failure (Kantarjian et al., Blood 110:3540 (2007)), as well as F317 (implicated in clinical resistance to dasatinib (Burgess et al., Proc Natl Acad Sci U S A 102:3395 (2005)), were potently inhibited by compound 1 at IC₅₀ values comparable to or below that of T315I cells (Table 1).

As reactivation of BCR-ABL signaling is a frequently observed feature of kinase domain mutation-mediated resistance to clinical ABL inhibitors, particularly in patients with chronic phase disease, we analyzed BCR-ABL^{T315I}-expressing cells by immunoblot analysis for CrkL phosphorylation, an established direct substrate of native and mutant BCR-ABL. In both Ba/F3 cells in vitro and primary CML BCR-ABL^{T315I} cells ex vivo, treatment with compound 1 resulted in a marked reduction in % pCrkL, while none of the three clinical ABL inhibitors showed any effect (Figure 1B and Figure 2B). Similar inhibition was observed when probing the levels of pBCR-ABL and pBCR-ABL^{T315I} in Ba/F3 cells, confirming the validity of the % pCrkL readout. This CrkL shift assay is a preferred means of examining pharmacodynamic efficacy of ABL kinase inhibitors, and will be employed for compound 1 in its phase 1 evaluation.

Compound 1 demonstrated potent activity after oral administration in a series of mouse models of CML driven by native BCR-ABL or BCR-ABL^{T315I}. In a survival model using Ba/F3 cells expressing native BCR-ABL, compound 1 significantly prolonged survival at low doses of 2.5 and 5 mg/kg (Figure 4A). Similar efficacy was observed using dasatinib at 5 mg/kg, suggesting that, at the same dose level, the in vivo activity of compound 1 in mice against native BCR-ABL is comparable to that of dasatinib. Importantly, in both survival and subcutaneous CML models using Ba/F3 cells expressing BCR-ABL^{T315I} compound 1 significantly extended survival of mice at 5, 15, and 25 mg/kg (Figure 4B). Tumor stasis or regression occurred at 30 and 50 mg/kg in the subcutaneous tumor model, and suppression BCR-ABL signaling was observed at a dose of 30 mg/kg (Figure 4C). Compound 1 was well tolerated at all dose levels used in these studies. These results have several implications. First, the fact that compound 1 is orally bioavailable provides an advantage over other T315I inhibitors that have been tried previously in the clinic. In particular, both the ABL/Aurora kinase inhibitors MK-0457 and PHA-739358 require intravenous administration to achieve doses sufficient to inhibit BCR-ABL activity (Giles et al., Blood 109:500 (2007); Gontarewicz et al., Blood 111:4355 (2008)). Additionally, both of these inhibitors inhibit both normal cells and BCR-ABL^{T315I} cells at comparable concentrations. By contrast, our in vivo data suggests that compound 1 has a wide therapeutic range between 5 and 50 mg/kg in CML animal models dependent on BCR-ABL^{T315I}.

We have previously used our accelerated cell-based mutagenesis screen to predict the spectrum of BCR-ABL kinase domain mutations conferring clinical resistance to imatinib, nilotinib, and dasatinib (Bradeen et al., Blood 108:2332 (2006)). As additional follow-up data on CML patients treated with each of the second-line ABL inhibitors are becoming available, several mutations have been reported in association with failure of either nilotinib (L248R, Y253H, E255K/V, T315I, F359I/V; (Kantarjian et al., Blood 110:3540 (2007)) or dasatinib (V299L, T315I, F317I/L; (Shah et al., J Clin Invest 117:2562 (2007)) which are largely consistent with our in vitro profiling. In our accelerated mutagenesis screens for compound 1, we found a concentration-dependent reduction in both the percentage of wells with outgrowth and in the range of mutations observed. Although at 10 nM compound 1 we observed 16 different substitutions across 13 different residues, increasing the concentration to 20 nM precipitously reduced both the total outgrowth observed (11.7% at 10 nM; 0.2% at 20 nM) and mutant types recovered (Figure 6A and Table 2). The only resistant subclones recovered at 20 nM harbored either a T315I or E255V mutation, and complete suppression of outgrowth at 40 nM compound 1 and above was observed (Figure 6A and Table 2). Our data suggest that compound 1, administered at the appropriate levels, may be exempt from susceptibility to single-mutation-based resistance. This result, using single-agent compound 1, has been previously achieved in this assay only in the presence of dual-combinations of either nilotinib or dasatinib with a pre-clinical T315I inhibitor (O'Hare et al., Proc Natl Acad Sci U S A 105:5507 (2008)).

To further explore the extent of compound I's ability to suppress resistant outgrowth, we carried out accelerated mutagenesis screens starting on a background of Ba/F3 cells expressing either of the two individually most resistant mutants, BCR-ABL^{T315I} or BCR-ABL^{E255V}. This predictive assay implicated certain compound mutations, especially those involving any two members of the set comprised of Y253H, E255V, and T315I in moderate to high-level resistance to compound 1 (Tables 3 and 4). Among these, Y253H/T315I and E255V/T315I are predicted to be the most resistant pairings with respect to compound 1 (Figure 6B and Tables 3 and 4). Notably, the presence of the T315I component implies that none of the currently approved clinical BCR-ABL inhibitors would be active against these mutants. Thus, compound 1 has the capability to eliminate compound mutations involving T315I and E255V that would be that would be predicted to be highly resistant to all other inhibitors. Currently, compound mutations within the kinase domain of BCR-ABL are rare (Table 5), but it is conceivable that their prevalence will increase with the prolonged survival of patients and with more patients undergoing sequential ABL kinase inhibitor treatment and at the present time, they present a formidable problem for those patients who have them. Although no mutagenesis screen can be completely exhaustive, our data suggest that mutations that would completely abrogate binding to compound 1 may not be compatible with preservation of sufficient kinase activity. In this scenario, escape from inhibition would come at the expense of a "functional suicide."

**Table 5. BCR-ABL compound mutants involving E255V or T315I conferring moderate to high level resistance to compound 1**

| Compound mutant | Compound 1 concentration at which recovered in screen | | | Reported clinically (refs.) |
|---|---|---|---|---|
| | 80 nM | 160 nM | 320 nM | |
| T315I / Q252H | ✔ | | | NR |
| T315I / Y253H | | ✔ | | (1), (2) |
| T315I / E255K | ✔ | | | (3) |
| T315I / E255V | ✔ | ✔ | ✔ | NR |
| T315I / F311I | ✔ | | | (2) |
| T315I / F311V | ✔ | | | NR |
| T315I / A380S | ✔ | | | NR |
| E255V / G250E | ✔ | | | NR |
| E255V / Q252H | ✔ | | | NR |
| E255V / Y253F | | ✔ | | NR |
| E255V / Y253H | ✔ | ✔ | | NR |
| E255V / E292V | ✔ | | | NR |
| E255V / F311I | ✔ | | | NR |
| E255V / E355G | ✔ | | | NR |
| E255V / F359C | ✔ | | | NR |
| E255V / F359I | ✔ | | | NR |
| E255V / H396H | ✔ | | | NR |

| | | | | |
|---|---|---|---|---|
| (1) Shah et al. (2007). JCI 117, 2562-2569. (2) Khorashad et al. (2008). Blood 111, 2378-2381. (3) Stagno et al. (2008). Leuk. Res. 32, 673-674. NOTE: The following clinically reported compound mutants were not detected in this screen: V299L / E255V. Abbreviations: NR, not reported. | | | | |

The combined results of our biochemical, cell-based, and in vivo studies suggest that compound 1, administered in appropriate amounts, exhibits sufficient activity against native BCR-ABL and all tested BCR-ABL mutants to warrant consideration for single-agent use as a pan-BCR-ABL inhibitor. Moreover, our results indicate that compound 1 holds promise for controlling compound mutants involving T315I, but also raise awareness that it is advantageous to eliminate resistant subclones at the single-mutation stage.

### Example 2: Clinical Study

Compound 1 is an orally available tyrosine kinase inhibitor that potently inhibits the enzymatic activity of BCR-ABL^{T315I}, the native enzyme and all other tested variants. It also inhibits survival of cell lines expressing these BCR-ABL variants with IC50s of < 40 nM.

A phase 1 clinical trial was conducted to assess the safety of compound 1 and provide preliminary assessments of clinical activity. The trial employed an open-label dose escalation design. Compound 1 was synthesized and formulated as described herein.

Patients with hematologic malignancies refractory to treatment (or relapsed or having no available standard therapy), ECOG status ≤ 2, QTcF < 450 ms, adequate hepatic and renal function, and normal cardiac function were eligible and received a single daily oral dose of compound 1. Hematological malignancies included CML (any phase), ALL, AML, MDS, MM, or CLL. Furthermore, patients must not have had chemotherapy ≥ 21 days or investigational agents ≥ 14 days prior to enrollment.

Fifty-seven patients (30 males) were enrolled and treated, median age 61 years (range 26-85) and median years from diagnosis 5.4 (0-21). Diagnoses included 50 CML (37 chronic [CP], 7 accelerated [AP], 6 blast phase [BP]), 3 Ph+ ALL, and 4 other malignancies (2 myelofibrosis, 1 myeloma, and 1 MDS). BCR-ABL mutation status in 48 Ph+ pts included 14 patients with no mutation and 34 patients with mutations (14 T315I, 5 F317L, 4 G250E, 3 with 2 or more mutations, and the remainder showed other mutations including F359C and F359V. Other specific mutations were M351T, L273M/F359V, G250E, E279K, F359C, L387F, and E453K). Prior therapies in 53 Ph+ pts (CML and ALL) included imatinib (96% of patients), dasatinib (87%), and nilotinib (57%), where 35 pts had ≥ 3 prior TKIs and 50 pts had ≥ 2 prior TKIs.

Patients were treated at the following dose levels: 2 mg (3 pts), 4 mg (6 pts), 8 mg (7 pts), 15 mg (8 pts), 30 mg (7 pts), 45 mg (13 pts), and 60 mg (13 pts). 45 mg was identified as the maximum tolerated dose (MTD) for further investigation. Intra-patient dose escalation was permitted.

Preliminary safety and efficacy data showed the following: for the 2 to 30 mg cohorts: no DLTs; for the 45 mg cohort: a reversible rash was seen with one patient; and for the 60 mg cohort: four patients developed reversible pancreatic related DLT (pancreatitis). The most common drug-related adverse events of any grade (AE) were thrombocytopenia (25%), anemia, lipase increase, nausea, and rash (12% each), and arthralgia, fatigue, and pancreatitis (11% each).

Pharmacokinetic and pharmacodynamic (PK/PD) studies included blood plasma analysis with deuterated compound 1 as an internal standard (PK) and measurement of phosphorylated levels of BCR-ABL substrate CRKL (p-CRKL) relative to total levels (PD). Sampling was conducted throughout the first 24 hours and prior to dosing on days (D) 8, 15, and 22 of cycle 1 (C1), and D1 of C2 (cycle = 28 days). Classification for PD effects included not evaluable (p-CRKL ≤ 20% at baseline or too few samples for analysis), transient (p-CRKL inhibition ≥ 50%* at 2 or more post-dose timepoints, but not sustained throughout cycle 1), sustained (p-CRKL inhibition ≥ 50%* at 2 or more post-dose timepoints that is sustained throughout cycle 1), or no effect (no p-CRKL inhibition by the above criteria). * indicates ≥ 25% inhibition is acceptable if baseline p-CRKL is too low (e.g., 35%) to reliably quantitate a > 50% decrease.

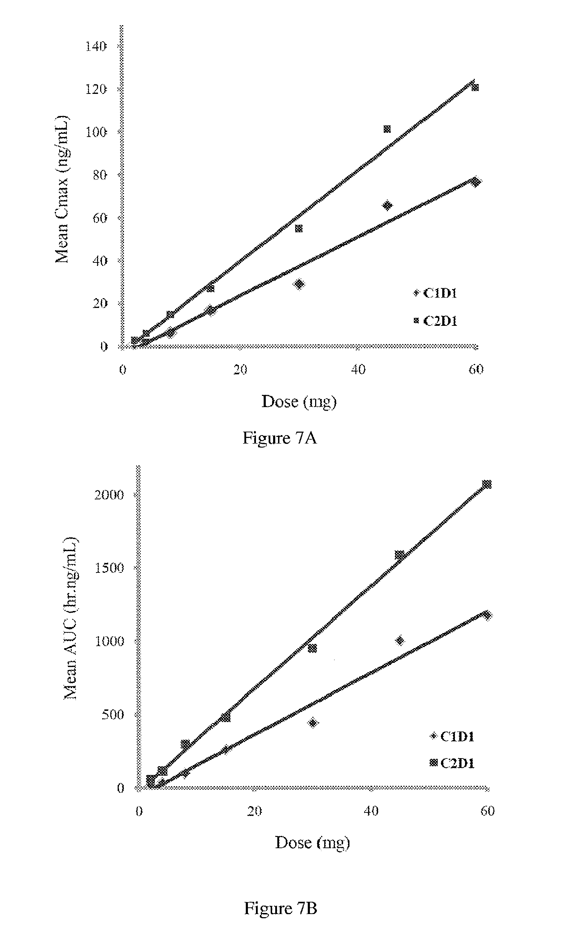

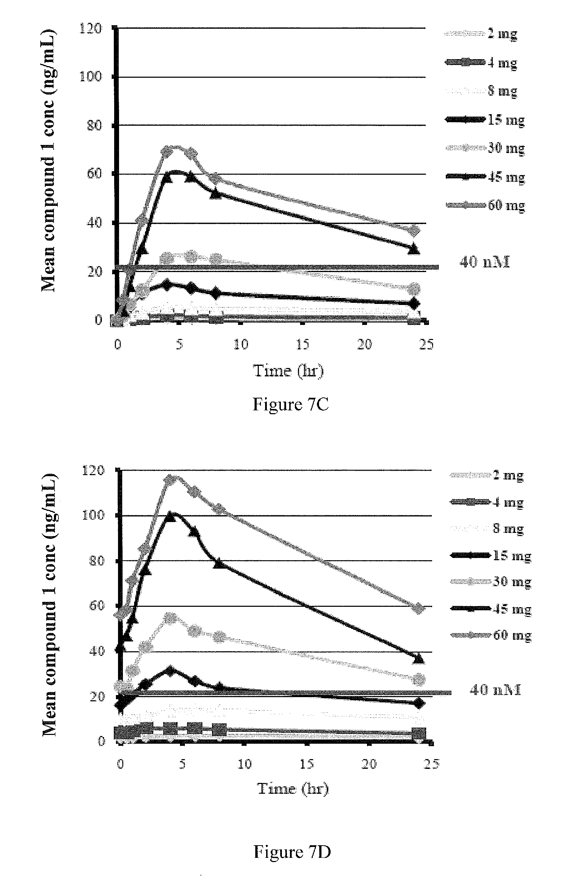

Pharmacokinetic data demonstrated that the half life of compound 1 is 19-45 hours. At doses ≥ 30 mg, the half life is 18 hours. Figures 7A and 7B show the linear relationship of Cmax and AUC to dose over the dosing range. Figure 7C and 7D show concentration time profiles. The Cmax on day 1 at the 30 mg dose was approximately 55 nM. After repeated dosing, 1.5 to 3-fold accumulation was observed in evaluable patients.

Pharmacokinetic data for patients receiving 60 mg of compound 1 daily is provided in Table 6.

**Table 6. Profile of compound 1 orally administered at 60mg (ng/mL)**

| Period(hr) | Subject | **0** | **0.5** | **1** | **2** | **4** | **6** | **8** | **24** |
|---|---|---|---|---|---|---|---|---|---|
| 1 | A | BQL | 0.31 | 6.21 | 15.6 | 46.9 | 71.4 | 80.2 | 43.1 |
| | B | BQL | 2.92 | 8.35 | 12.2 | 31 | 29.5 | 22.7 | 17.9 |
| | C | BQL | 3.95 | 27 | 48.5 | 73 | 60.6 | 41.8 | 33.2 |
| | D | BQL | 11.6 | 27.8 | 56 | 151 | 151 | 135 | 51.6 |
| | E | BQL | 3.25 | 13.8 | 63.3 | 79.2 | 78.5 | 67 | 28.2 |
| | F | BQL | 22.1 | 39.7 | 56.6 | 65.6 | 56.4 | 46.9 | 22.3 |
| | G | BQL | BQL | 0.59 | 4.94 | 35.4 | 52.7 | 49.8 | 26.2 |
| | **Mean** | **Missing** | **7.355** | **17.635** | **36.734** | **68.871** | **71.443** | **63.343** | **31.786** |
| 2 | A | 82 | 77.6 | 79.1 | 76.7 | 108 | 138 | 137 | 80.7 |
| | B | 30.1 | 36.8Missing | | 57.1 | 109 | 87.1 | 70.2 | 35.8 |
| | C | 61.5 | 67.4 | 78.5 | 94.8 | 94.7 | 85.3 | 72.1 | 47.6 |
| | D | 13.1 | 14.6 | 17.9 | 33.8 | 54.3 | 50.8 | 41 | 19.1 |
| | **Mean** | **46.675** | **49.1** | **58.5** | **65.6** | **91.5** | **90.3** | **80.075** | **45.8** |

The mean steady state trough level when dosing daily at 60 mg (the level at 24 hour post dosing following one 28-day cycle) is about 45 ng/mL, which corresponds to a circulating plasma concentration of about 90 nM, a circulating concentration that can be useful for suppressing the emergence of resistant subclones in these subjects. With doses of 30 mg or higher, trough levels surpassed 40 nM (21 ng/mL), the concentration in which the mutation assay demonstrated complete suppression of emergent clones (as in Figure 6A).

PD data demonstrate inhibition of CrkL phosphorylation at doses of 8 mg and higher. As shown in Figure 8A, sustained target inhibition was observed at doses ≥ 8 mg in the overall population and at doses ≥ 15 mg in T315I patients. Figures 8B-8E show pharmacodynamics data for different doses and in patients having different mutations. Overall best hematologic responses were complete hematologic response (CHR) in 22 of 22 CP patients (85%), including new and baseline CHR, and major hematologic response (MHR) in 5 of 12 AP, BP, or ALL patients. Cytogenetic responses were 8 complete cytogenetic responses (CCyR) and 12 MCyR. Best hematologic responses in the T315I subset were CHR in 8 of 9 CP pts (89%), including new and baseline CHR, and MHR in 3 of 8 AP, BP, or ALL patients. Nine of 12 T315I patients were evaluable for CyR: 5 CP and 1 AP, BP, or ALL patients achieved CCyR, and 6 CP and 3 AP, BP, or ALL achieved MCyR Molecular responses included 8 MMRs in 32 CP AML patients (4 in patients with T315I at baseline).

Conclusions: No DLTs have been observed at doses up to 30 mg compound 1, and reversible DLTs were observed at higher doses. PK and PD demonstrate that blood levels at 30 mg exceed those needed for in vitro inhibition of resistant mutant BCR-ABL isoforms, including T315I. Preliminary analysis revealed evidence of clinical antitumor activity in patients with resistance to approved second-line TKIs dasatinib and nilotinib, including patients with the T315I mutation of BCR-ABL. The results obtained thus far show (1) consistent sustained target inhibition observed at doses of 8 mg and higher in the overall population, (2) sustained target inhibition in T315I patients observed at 15 mg dose level or higher, (3) identification of 45 mg of compound 1 as the MTD, and (4) the higher doses needed to suppress the emergence of resistant subclones in patients undergoing therapy were tolerated without significant adverse events. Trough drug concentrations surpassed the threshold for pan-BCR-ABL activity of compound 1 are observed at doses ≥ 30 mg. At doses ≥ 15 mg, there was sustained inhibition of BCR-ABL in patients with a variety of mutations, including T315I. Together, these findings correlate well with clinical evidence of anti-leukemic activity in refractory Ph+ patients who have failed currently available TKIs.

### Example 3: Inhibition of FLT3 Mutants for Acute Myelogenous Leukemia EXPERIMENTAL PROCEDURES

**Cell lines, antibodies and reagents:** MV4-11, RS4;11, Kasumi-1 and KG1 cells were obtained from the American Type Culture Collection (Manassas, VA), and EOL1 cells obtained from DSMZ (Braunschweig, Germany). Cells were maintained and cultured according to standard techniques at 37 °C in 5% (v/v) CO₂ using RPMI 1640 supplemented with 10% FBS (20% FBS for Kasumi-1 cells). Compound 1 was synthesized at ARIAD Pharmaceuticals (Cambridge, MA), and sorafenib and sunitinib were purchased from American Custom Chemical Corporation (San Diego, CA). All compounds were prepared as 10 mM stock solutions in DMSO. The antibodies used included: phospho-PDGFRα, PDGFRα, FLT3, FGFR1 and GAPDH from Santa Cruz Biotechnology (Santa Cruz, CA); STAT5, KIT, phospho-KIT, phospho-FGFR and phospho-FLT3 from Cell Signaling Technology (Beverly, MA); phospho-STAT5 from BD Biosciences (San Jose CA).

**Cell viability assays:** Cell viability was assessed using the Cell Titer 96 Aqueous One Solution Cell Proliferation Assay (Promega, Madison WI). Exponentially growing cell lines were plated into 96-well plates and incubated overnight at 37°C. Twenty-four hours after plating, cells were treated with compound or vehicle (DMSO) for 72 hours. Fluorescence was measured using a Wallac Victor microplate reader (PerkinElmer, Waltham, MA). Data are plotted as percent viability relative to vehicle-treated cells and the IC₅₀ values (the concentration that causes 50% inhibition) are calculated using XLfit version 4.2.2 for Microsoft Excel. Data are shown as mean (± SD) from 3 separate experiments, each tested in triplicate.

**Immunoblot analysis:** To examine inhibition of receptor tyrosine kinase signaling, cells were treated with compound 1 over a range of concentrations (0.03-100 nM) for 1 hour. Cells were lysed in ice-cold SDS lysis buffer (0.06 M Tris-HCL. 1% SDS and 10% glycerol) and protein concentration was determined using a BCA Protein assay (Thermo Scientific, Rockford, IL). Cellular lysates (50 µg) were resolved by electrophoresis and transferred to nitrocellulose membranes using NuPage reagents (Invitrogen, Carlsbad, CA). Membranes were immunoblotted with phosphorylated antibodies and then stripped with Restore Western Blot Stripping Buffer (Thermo Scientific) and immunoblotted with total protein antibodies. The IC₅₀ values were calculated by plotting percent phosphorylated protein in compound I -treated cells relative to vehicle-treated cells.

**Apoptosis assays:** For measurement of caspase activity, MV4-11 cells were seeded into black-walled 96-well plates at 1 x 10⁴ cells/well for 24 hours and then treated with compound 1 for the indicated time-points. Apo-One Homogeneous Caspase 3/7 reagent (Promega, Madison, WI) was added according to the manufacturer's protocol, and fluorescence was measured in the Wallac Victor microplate reader. To measure PARP cleavage, MV4-11 cells were plated in 6-well plates and, the following day, were treated for 24 hours with compound 1. At the end of treatment cells were lysed with SDS buffer and immunoblotted to measure for both total PARP and cleaved PARP expression (Cell Signaling Technology).

**Subcutaneous xenograft model:** The MV4-11 human tumor xenograft efficacy study was performed by Piedmont Research Center (Morrisville, NC). Briefly, tumor xenografts were established by the subcutaneous implantation of MV4-11 cells (1 x 10⁷ in 50% matrigel) into the right flank of female CB.17 SCID mice and dosing was initiated when the average tumor volume reached ~200 mm³. Compound 1 was diluted in a vehicle of 25 mM citrate buffer (pH=2.75) and mice were dosed orally once daily for 4 weeks. The tumors were measured in two dimensions (length and width) with a caliper in millimeters. Tumor volume (mm³) was calculated with the following formula tumor volume = (length x width²)/2. Tumor growth inhibition (TGI) was calculated as follows: TGI = (1-ΔT / ΔC) x 100, where ΔT stands for mean tumor volume change of each treatment group and ΔC for mean tumor volume change of control group. The tumor volume data were collected and analyzed with a one-way ANOVA test (GraphPad Prism, San Diego, CA) to determine the overall difference among groups. Each compound 1 treatment group was further compared to the vehicle control group for statistical significance using Dunnett's Multiple Comparison Test. A p-value <0.05 was considered to be statistically significant and a p-value <0.01 to be highly statistically significant.

**Pharmacokinetics and pharmacodynamics**: Following MV4-11 xenograft tumor establishment, mice were administered a single oral dose of compound 1 and tumors harvested 6 hours later. Individual tumors were homogenized in ice-cold RIPA buffer containing protease and phosphatase inhibitors and clarified by centrifugation. Samples were resolved by SDS-PAGE, transferred to nitrocelluose membranes, and immunoblotted with antibodies against total and phosphorylated FLT3 and STAT5. Compound I concentrations in plasma were determined by an internal standard LC/MS/MS method using protein precipitation and calibration standards prepared in blank mouse plasma. Below quantitation limit (BQL) = <1.2 ng/ml compound 1. Reported concentrations are the mean values from four mice/group.

**Treatment of primary AML patient samples ex vivo:** All patient samples were de-identified and collected with informed consent with approval from the Institutional Review Board of Oregon Health & Science University. Mononuclear cells were isolated from peripheral blood from patients with acute myelogenous leukemia over a Ficoll gradient followed by red cell lysis. Cells were quantitated using Guava ViaCount reagent and a Guava Personal Cell Analysis flow cytometer (Guava Technologies, Hayward, CA). Cells were plated into 96-well plates (5 x 10⁴ per well) over graded concentrations of compound 1 (1-1000 nM) in RPMI supplemented with 10% FBS, penicillin/streptomycin, L-glutamine, fungizone, and 10⁻⁴ M 2-mercaptoethanol. After 72 hour incubation, cells were subjected to an MTS assay (Cell Titer Aqueous One Solution Cell Proliferation Assay. Promega) for assessment of cell viability. All values were normalized to the viability of cells plated without any drug and percent viability was used to determine the compound 1 IC₅₀ for each sample. FLT3 status was determined by PCR on genomic DNA from each patient.

### RESULTS

### (1) Compound 1 inhibited signaling and proliferation in hematopoietic cell lines driven by mutant, constitutively active FLT3, KIT, FGFR1 and PDGFRα.

Compound 1 inhibits the in vitro kinase activity of FLT3, KIT, FGFR1 and PDGFRα with IC₅₀ₛ of 13, 13, 2 and 1 nM, respectively. Here, the activity of compound 1 was evaluated in a panel of leukemic cell lines that harbor activating mutations in FLT3 (FLT3-ITD; MV4-11 cells) and KIT (N822K; Kasumi-1 cells), or activating fusions of FGFR1 (FGFR1OP2-FGFR1; KG-1 cells) and PDGFRα (FIP1L1-PDGFRα; EOL-1 cells). Compound 1 inhibited phosphorylation of all 4 RTKs in a dose-dependent manner, with IC₅₀ₛ between 0.3 - 20 nM (Table 7).

**Table 7. Inhibition of proliferation and signaling in AML cell lines**

| | | | **IC₅₀ (nM)** | | |
|---|---|---|---|---|---|
| **Cell line** | **RTK status** | **Assay** | **Compound 1** | **Sorafenib** | **Sunitinib** |
| MV4-11 | FLT3-ITD | RTK phosphorylation | 0.3 | | |
| | | Cell viability | 2 | 4 | 12 |
| Kasumi-1 | c-KIT (N822K) | RTK phosphorylation | 20 | | |
| | | Cell viability | 8 | 59 | 56 |
| KG1 | FGFR1OP2-FGFR1 | RTK phosphorylation | 3 | | |
| | | Cell viability | 17 | >100 | >100 |
| EOL1 | FIP1L1-PDGFRα | RTK phosphorylation | 0.6 | | |
| | | Cell viability | 0.5 | 0.5 | 3 |
| RS4;11 | wt | RTK phosphorylation | | | |
| | | Cell viability | >100 | >100 | >100 |

Consistent with these activated receptors being important in driving leukemogenesis (Chalandon et al., Haematologica. 90:949-968 (2005)), compound 1 also potently inhibited the viability of all 4 cell lines with IC₅₀ₛ of 0.5 -17 nM (Figure 9, Table 7). In contrast, the IC₅₀ for inhibition of RS4;11 cells, which lack activating mutations in these 4 receptors, was >100 nM. These data suggest that compound 1 selectively targets leukemic cells that express one of these aberrant RTKs.

The potency and activity profile of compound 1 was next compared to that of two other multi-targeted kinase inhibitors, sorafenib and sunitinib, by examining their effects on viability of the same panel of cell lines in parallel. While potent inhibitory activity of sorafenib and sunitinib was observed against FLT3 (IC₅₀, of 4 and 12 nM, respectively) and PDGFRα (0.5 and 3 nM), neither compound exhibited the high potency that compound 1 has against KIT (59 and 56 nM) or FGFR1 (>100 and >100 nM) (Table 7).

### (2) Potent apoptotic effects of compound 1 on MV4-11 cells

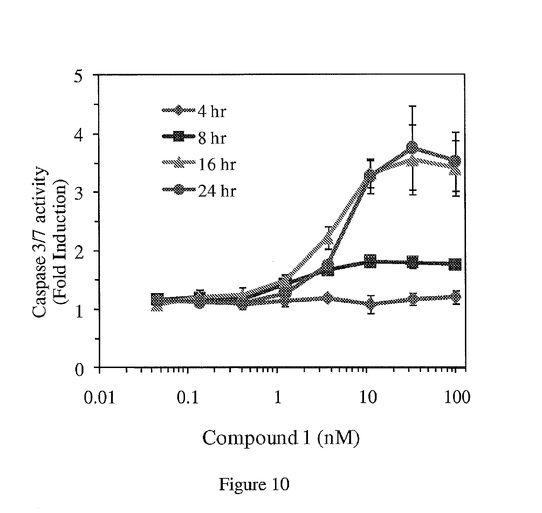

Given the major clinical relevance of the FLT3-ITD mutation in AML, subsequent studies focused on the characterization of compound 1's activity against this target. To examine the basis for compound 1's effect on viability of FLT3-ITD-driven MV4-11 cells, its effect on 2 markers of apoptosis was measured. A dose- and time-dependent increase in caspase 3/7 activity was observed, with maximal induction (up to 4-fold) seen with 10-30 nM compound 1 and within 16 hours of treatment (Figure 10). Similarly, at concentrations ≥10 nM, compound 1 showed near maximal induction of PARP cleavage and concomitant inhibition of phosphorylation of STAT5, a direct downstream substrate of the mutant FLT3-ITD kinase (Choudhary et al., Blood. 110:370-374 (2007)) and important regulator of cell survival. Taken together, these data support the conclusion that inhibition of FLT3-ITD by compound 1 inhibits MV4-11 cell viability through the induction of apoptosis.

### (3) In vivo efficacy and pharmacodynamic studies

To examine the effect of compound 1 on FLT3-ITD-driven tumor growth in vivo, compound 1 (1 - 25 mg/kg), or vehicle, was administered orally, once daily for 28 days, to mice bearing MV4-11 xenografts. As shown in Figure 11A, compound 1 potently inhibited tumor growth in a dose-dependent manner. Administration of 1 mg/kg, the lowest dose tested, led to significant inhibition of tumor growth (TGI=46%, p<0.01) and doses of 2.5 mg/kg or greater resulted in tumor regression. Notably, dosing with 10 or 25 mg/kg led to complete and durable tumor regression with no palpable tumors detected during a 31-day follow up.

To confirm target modulation in vivo, mice bearing MV4-11 xenografts were administered a single oral dose of vehicle or compound 1 at 1, 2.5, or 10 mg/kg. Tumors were harvested after 6 hours and levels of phosphorylated FLT3 and STAT5 were evaluated by immunoblot analysis. A single dose of 1 mg/kg compound 1 had a modest inhibitory effect on FLT3 signaling, decreasing levels of p-FLT3 and p-STAT5 by approximately 30%. Increased doses of compound 1 led to increased inhibition of signaling with 5 and 10 mg/kg doses inhibiting signaling by approximately 75 and 80%, respectively. Pharmacokinetic analysis demonstrated a positive association between the concentration of compound 1 in plasma and inhibition of FLT3-ITD signaling (Figure 11B). These data show that inhibition of signaling by compound 1 is associated with the degree of efficacy (Figure 11A) and support the conclusion that inhibition of FLT3-ITD signaling accounts for the anti-tumor activity of compound 1 in this model.

### (4) Activity of compound 1 in primary AML cells

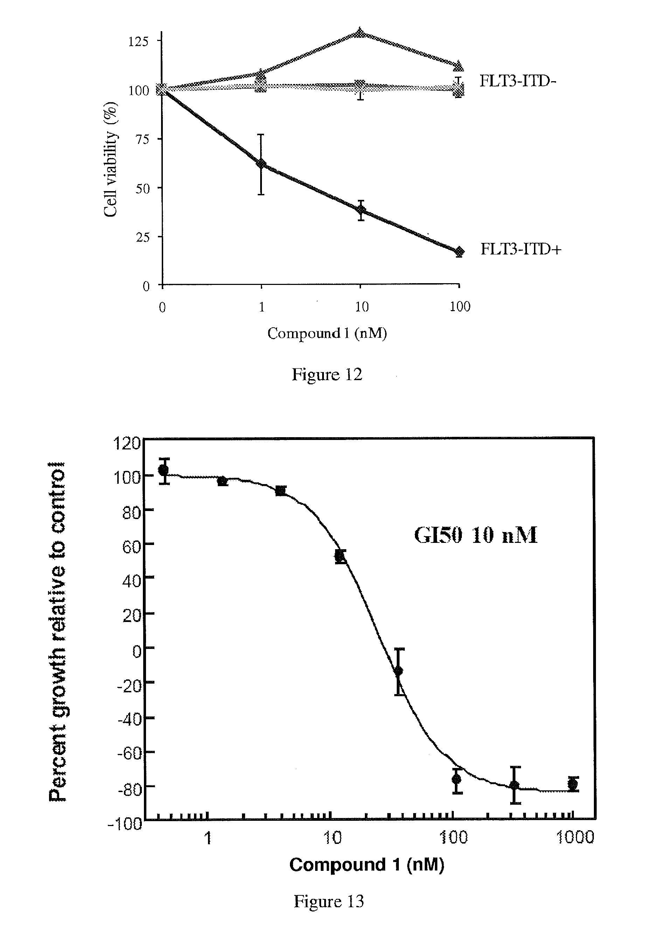

To assess the activity of compound 1 in primary cells from patients with AML, we obtained peripheral blood blasts from four patients; three that expressed wild-type FLT3 and one that harbored a FLT3-ITD mutation. FLT3 status was confirmed by PCR on genomic DNA from each patient. Cell viability was measured following exposure to compound 1 for 72 hours (Figure 12). Consistent with the results obtained in cell lines, compound 1 reduced viability of FLT3-ITD positive primary blasts with an IC₅₀ 4 nM, while wild-type blasts showed no reduction in viability at the concentrations tested (up to 100 nM). Taken together, these findings support the conclusion that compound 1 is selectively cytotoxic to leukemic cells harboring a FLT3-ITD mutant.

### Discussion

Here, using leukemic cell lines containing activated forms of each of these receptors, we show that compound 1 exhibits activity against kinases a discrete set of kinases, implicated in the pathogenesis of hematologic malignancies (FLT3, KIT, and members of the FGFR and PDGFR families) with potency similar to that observed for BCR-ABL, i.e., IC₅₀ₛ for inhibition of target protein phosphorylation and cell viability ranged from 0.3 - 20 nM and 0.5 - 17 nM, respectively. Other multitargeted kinase inhibitors, such as sorafenib and sunitinib, have previously been shown to have inhibitory activity against a subset of these kinases. However, we found that compound 1 was unique in its ability to inhibit activity of all four kinase with high potency. Since compound 1 exhibits comparably potency against FLT3, KIT, FGFR1 and PDGFRα in the models tested here, compound 1 can be useful for the treatment of diseases in which these kinases play a role.

MPNs with genetic rearrangements of FGFR1 and PDGFRα are considered to be rare; however, it has been demonstrated that the resulting fusion proteins play a major role in the pathogenesis of these diseases (Gotlib et al., Leukemia 22:1999-2010 (2008); Macdonald et al., Acta Haematol. 107:101-107 (2002)). EMS is an aggressive disease that can rapidly transform to AML in the absence of treatment. We have shown here that compound 1 potently inhibits viability of the AML KG1 cell line, which is driven by an FGFR1OP2-FGFR1 fusion protein, supporting the clinical applicability of compound 1 in this disease type. HEL/CEL patients with a PDGFRα fusion achieve dramatic hematological responses when treated with the PDGFR inhibitor imatinib (Gotlib et al., Leukemia 22:1999-2010 (2008)) and we have shown that compound 1 has potent activity against the FIP1L1-PDGFRα fusion protein as demonstrated in the leukemic EOL cell line. However, the T674I mutant of PDGFRα, which is mutated at the position analogous to the T315I gatekeeper residue in BCR-ABL, has been demonstrated to confer resistance to imatinib in patients (Gotlib et al., Leukemia 22:1999-2010 (2008)). Importantly, compound 1 has potent activity against the PDGFRα T674I mutant kinase, with an IC₅₀ of 3 nM, support the application of compound 1 for the treatment of patients who carry this fusion protein.

Both the incidence and prognostic significance of FLT3-ITD alterations in AML show that this kinase plays a critical role in the pathogenesis of the disease (Levis et al., Leukemia 17:1738-1752 (2003)) and, as such, represents a major target for therapeutic intervention. In the studies reported here, using the FLT3-ITD expressing cell line MV4-11, we show a close relationship between inhibition of FLT3 activity, both in vitro and in vivo, and inhibition of tumor cell viability. In vitro, low nM concentrations of compound 1 (i.e., <10 nM) led to a decrease in FLT3 phosphorylation, a decrease in viability and an increase in markers of apoptosis. In an in vivo xenograft model, a daily oral dose of 1 mg/kg compound 1 led to significant inhibition of tumor growth and a dose of 5 mg/kg or greater led to tumor regression. Consistent with the effects on tumor growth being due to inhibition of FLT3, a single dose of 1 mg/kg compound 1 led to a partial inhibition of FLT3-ITD and STAT5 phosphorylation, while doses of 5 and 10 mg/kg led to substantial inhibition. Finally, compound 1 potently inhibited viability of primary blasts isolated from a FLT3-ITD positive AML patient (IC₅₀ of 4 nM), but not those isolated from three FLT3 wild-type patients (IC₅₀ >100 nM).

Multiple compounds with FLT3 activity have been described and several have already been evaluated in patients, with relatively modest clinical activity reported to date (e.g., Stirewalt et al., Nat. Rev. Cancer 3:650-665 (2003); Chu et al., Drug Resist. Updat. 12:8-16 (2009); Weisberg et al., Oncogene Jul 12 2010). Based on preclinical studies that show that FLT3 inhibition needs to be sustained in order to effect killing of FLT3-dependent AML cells, a view has emerged that in order to achieve maximum therapeutic benefit continuous and near-complete inhibition of FLT3 kinase may be required (Pratz et al., Blood 113:3938-3946 (2009)). Our in vitro studies demonstrate that complete, i.e., sustained substantial, inhibition of FLT3 phosphorylation and function can be obtained at ≤10 nM concentrations. Importantly, preliminary analysis of the pharmacokinetic properties of compound 1, when dosed at tolerable levels, evidenced trough levels exceeding 40 nM (i.e., prior to the next daily dose). These data support the conclusion that the potency and pharmacologic properties of compound 1 permit continuous and near-complete inhibition of FLT3 in patients.

Compound 1 is a multi-targeted kinase inhibitor that displays potent inhibition of FLT3 and is cytotoxic to AML cells harboring the FLT3-ITD mutation. Importantly, this agent exhibits activity against additional RTKs, FGFR1, KIT and PDGFRα, which have also been shown to play roles in the pathogenesis of hematologic malignancies. Notably, the potency of compound 1 against these RTKs in vitro and plasma levels of compound 1 observed in humans support a clinical role for compound 1 against these targets. Taken together, these observations provide strong preclinical support for the development of compound 1 as a novel therapy for AML and other hematologic malignancies, such as those driven by KIT, FGFR1 or PDGFRα is warranted.

### Example 4: Preliminary Results in an AML Patient with a FLT3 mutation

Beyond the highly significant cell-based results discussed in Example 3, preliminary clinical trial results include a complete response in a refractory AML patient with a FLT3-ITD mutation following treatment with 45 mg of compound 1, given daily p.o. Overall, these results support the development of compound 1 in patients with FLT3-ITD driven AML and other hematologic malignancies. Moreover, in view of its inhibitory profile against other kinases, ponatinib may also have an important role against various cancers driven by KIT, FGFR1, PDGFRα or other kinases, native or mutant.

### Example 5: Kinase Selectivity Profile of Compound 1

**Reagents:** Compound 1 was synthesized, as described herein. The following compounds were purchased: PD173074 (Calbiochem, Gibbstown, NJ), BMS-540215 (American Custom Chemical, San Diego, CA), CHIR-258 and BIBF-1120 (Selleck Chemical Co, London ON, Canada).

**Kinase assay:** Kinase inhibition assays to determine IC50s were performed at Reaction Biology Corporation (RBC, Malvern, PA USA). Compounds were tested at 10 µM ATP using a 10-point curve with 3-fold serial dilutions starting at 1 µM. Average data from 2 assays are shown.

**Cell growth assay:** Cell growth was assessed using either Cell Titer 96 Aqueous One Solution Cell Proliferation Assay (Promega, Madison, WI) or CyQuant Cell proliferation Assay (Invitrogen, Carlsbad, CA). Cells were treated with compound 24 hours after plating and grown for 72 hours. The concentration that causes 50% growth inhibition (GI50) was determined by correcting for the cell count at time zero (time of treatment) and plotting data as percent growth relative to vehicle (dimethyl sulfoxide, DMSO) treated cells using XLfit version 4.2.2 for Microsoft Excel. Data are shown as mean (±SD) from 3 separate experiments tested in triplicate.

**Soft agar colony formation assay:** The soft agar assay was performed using the CytoSelect 96-Well Cell Transformation Assay (Cell Biolabs, San Diego, CA). Briefly, cells were resuspended in 0.08% agar and plated on 0.06% agar in 96-well plates. Cells were treated once with Compound 1 at the time of plating and incubated for 8-10 days. Cells were either stained with iodonitrotetrazoliumchloride (Sigma, St. Louis, MO) or solubilized and quantified with CyQuant Dye according to the manufacturer's protocol. "ND" indicated not determined.

**Western immunoblotting:** Cells were treated 24 hours after plating and incubated with inhibitor for 1 hour. Cells were lysed in either SDS buffer or Phospho-SafeTM buffer (Novagen, Gibbstown, NJ) and protein lysates were immunoprecipitated overnight and/or immunoblotted with the indicated antibodies. Protein expression was quantified using Quantity One software (BioRad, Hercules, CA). The IC50 values (the concentration that causes 50% inhibition) were calculated by plotting percent inhibition of the phospho-signal normalized to the total protein signal using XLfit4. Data shown in the table are average values from 2-3 assays.

**Subcutaneous tumor models:** AN3CA cells were implanted into the right flank of nude mice. For analysis of efficacy, when the average tumor volume reached ∼200 mm³, inhibitor was administered by daily oral dosing for 12 days. Mean tumor volumes (± SE; tumor volume= L x W² x 0.5) were calculated for each treatment group.

**Pharmacodynamics/Pharmacokinetics:** For pharmacodynamic analysis tumor samples were frozen upon collection, homogenized in Phospho-SafeTM buffer and analyzed by Western immunoblotting. Inhibitor concentrations in plasma were determined by an internal standard LC/MS/MS method using protein precipitation and calibration standards prepared in blank mouse plasma. Data shown are mean values from 3 mice/timepoint/group.

The in vitro potency and selectivity of compound 1 was assessed in kinase assays with multiple recombinant kinase domains and peptide substrates (Table 8). Compound 1 was found to inhibit members of the PDGFR, FGFR, and VEGFR families of receptor tyrosine kinases (such as FLT1, FLT4, and KDR) (Table 1). Compound 1 is a potent inhibitor of all four FGF receptors: FGFR1, FGFR 2, FGFR 3, FGFR 4, as well as FGFR1(V561M) and FGFR2(N549H) (Table 8), which is unique when compared to other multi-targeted kinase inhibitors that do not inhibit all four FGFRs (e.g. sunitinib, sorafenib, and dasatinib). Notably, however, compound 1 did not inhibit Aurora or insulin kinase family members, nor did it inhibit cyclin-dependent kinase 2 (CDK2)/Cyclin E.

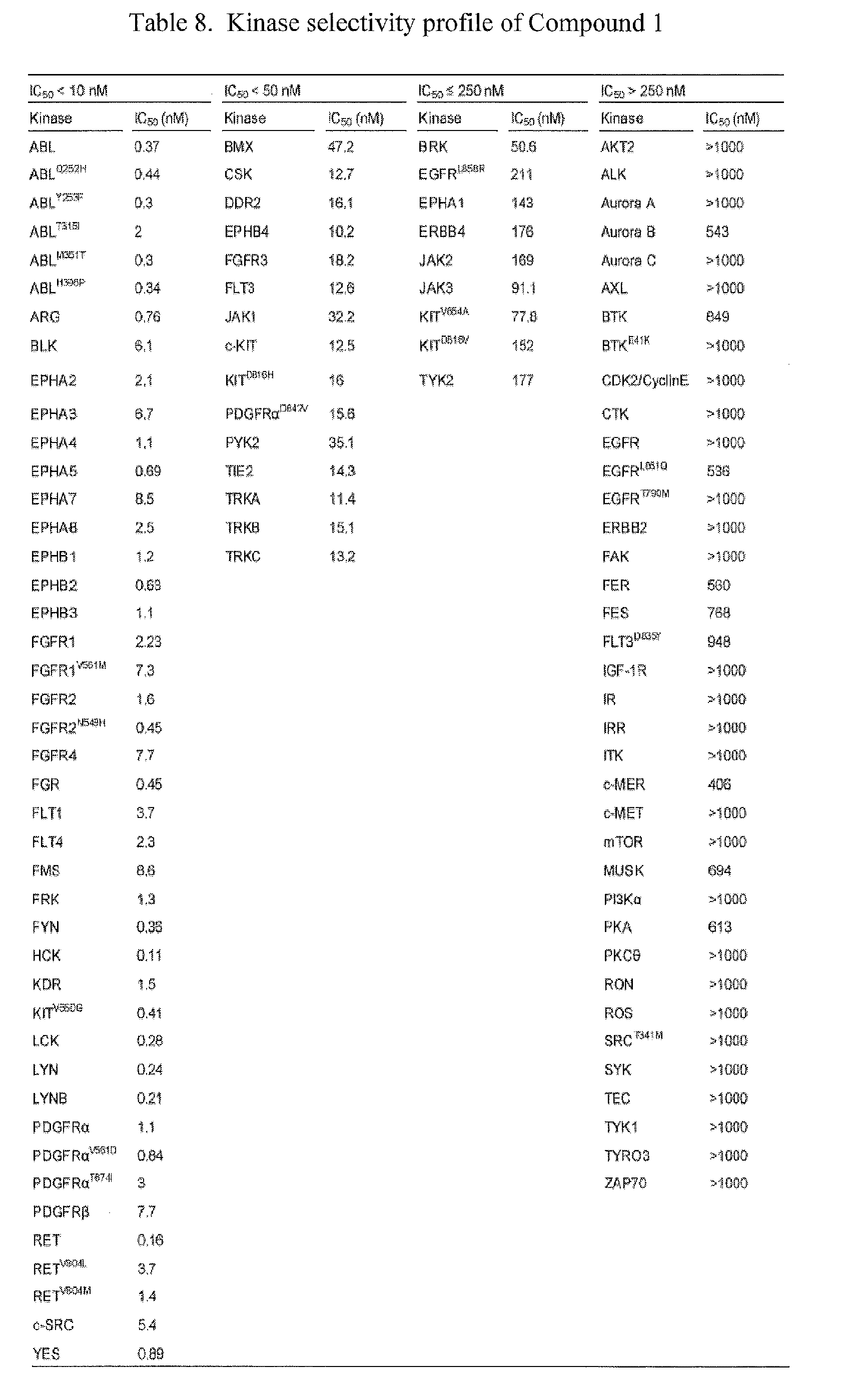

### Example 6: Effect of compound 1 in cellular models of cancer

Compound 1 affected cellular activity in various cancer cell lines. Experimental procedures were performed as described in Example 5. In the acute myelogenous leukemia-derived KG1 cell line that expressed the FGFR1-FGFR1OP2 fusion gene, compound 1 inhibited cell growth and the phosphorylation of FGFR1. Figure 13A shows the growth inhibition of compound 1 on the KG1 cell line with a determined GI50 of 10 nM. Compound 1 inhibited phosphorylation of FGFR1 with an IC50 of 10 nM, which was determined by Western immunoblot analysis of P-FGFR1, T-FGFR1, and glyceraldehyde-3-phosphate dehydrogenase ("GADPH") expression in KG1 cells treated with compound 1. Data for GAPDH was used as a control.

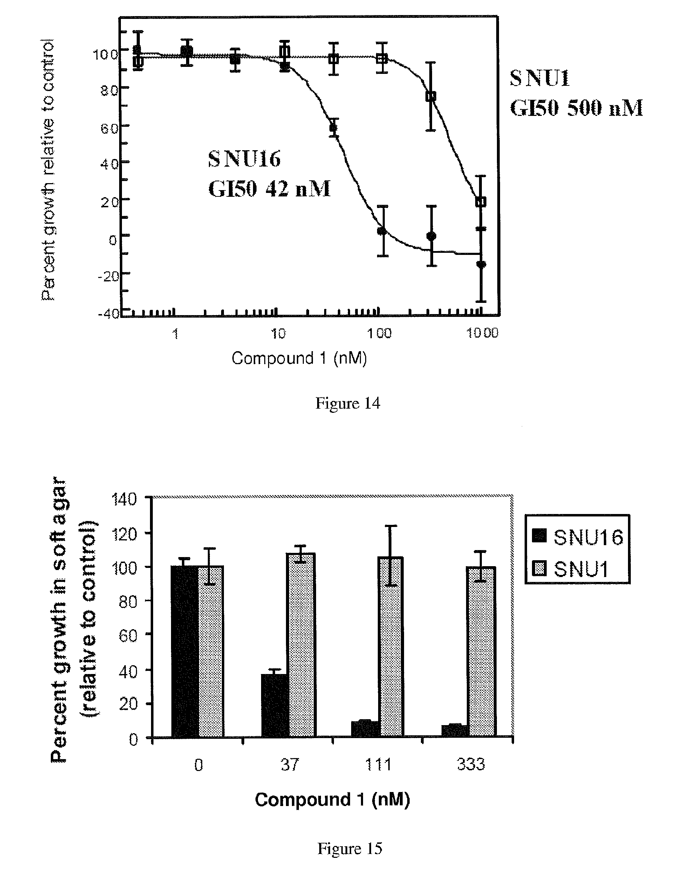

Compound 1 can also selectively affect the cellular activity of cancer cells, as compared to normal cells. Compound 1 selectively inhibited SNU16 gastric cancer cells with amplified FGFR2, when compared to wtFGFR2 SNU1 cells (Figure 14). Compound 1 inhibited signaling in SNU16, as determined by the reduced presence of phosphorylated FGFR2, FRS2a, and Erk 1/2 in a Western immunoblot analysis for protein expression in SNU16 gastric cancer cells. Compound 1 also selectively inhibited SNU16 colony formation in soft agar, when compared to wtFGFR2 SNU1 cells (Figure 15). Table 9 provides a summary of the activity of compound 1 in gastric cancer cell lines SNU16 and KatoIII, as compared to the wtFGFR2 SNU1 cell line. Compound 1 selectively inhibited cell growth and phosphorylation of FRS2a and Erk 1/2 of gastric cancer cells SNU16 and KatoIII, as compared to wt SNU1.

**Table 9. Summary of the Activity of Compound 1 in gastric cancer cell lines**

| | | Compound 1 | | | |
|---|---|---|---|---|---|
| Cell Line | FGFR2 Status | Cell Growth GI50 (nM) | Soft Agar IC50 (nM) | Phospho-FRS2a IC50 (nM) | Phospho-Erk1/2 IC50 (nM) |
| SNU16 | Amp | 42 | 29 | 12 | 8 |
| KatoIII | Amp/truncate | 7 | nd | 34 | 8 |
| SNU1 | Wt | 500 | >1000 | >1000 | >1000 |

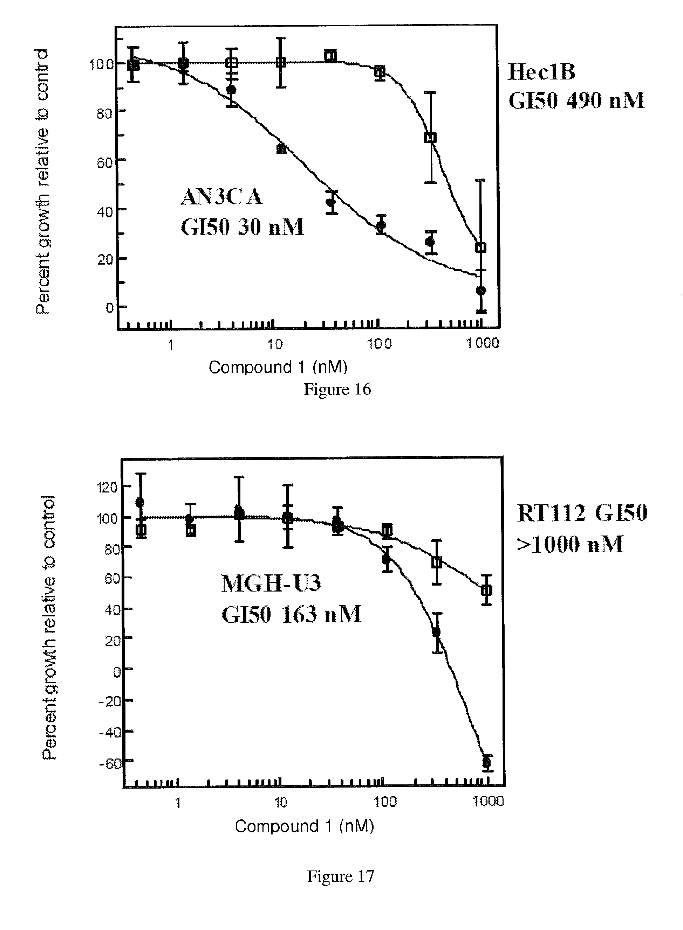

Compound 1 selectively inhibited AN3CA endometrial cancer cells with mutant FGFR2 (N549K), when compared to wtFGFR2 Hec1B cells. Compound 1 inhibited cell growth of AN3CA cells with a GI50 of 30 nM, as compared to Hec1B cells with a GI50 of 490 nM (Figure 16). Compound 1 also inhibited signaling in AN3CA cells, particularly the phosphorylation of FRS2a and Erk 1/2, as determined by Western immunoblot analysis of protein expression in AN3CA endometrial cancer cells treated with compound 1. Table 10 provides a summary of the activity of compound 1 in endometrial cancer cell line AN3CA, as compared to the wtFGFR2 Hec1B and RL95 cell lines.

**Table 10. Summary of the Activity of Compound 1 in endometrial cancer cell lines**

| | | Compound 1 | | | |
|---|---|---|---|---|---|
| Cell Line | FGFR2 Status | Cell Growth GI50 (nM) | Soft Agar IC50 (nM) | Phospho-FRS2a IC50 (nM) | Phospho-Erk1/2 IC50 (nM) |
| AN3CA | N549K | 30 | 23 | 3.7 | 5.8 |
| Hec1B | wt | 490 | >1000 | >1000 | >1000 |
| RL95 | wt | 428 | nd | >1000 | >1000 |

| | | | | | |
|---|---|---|---|---|---|
| nd: not determined | | | | | |

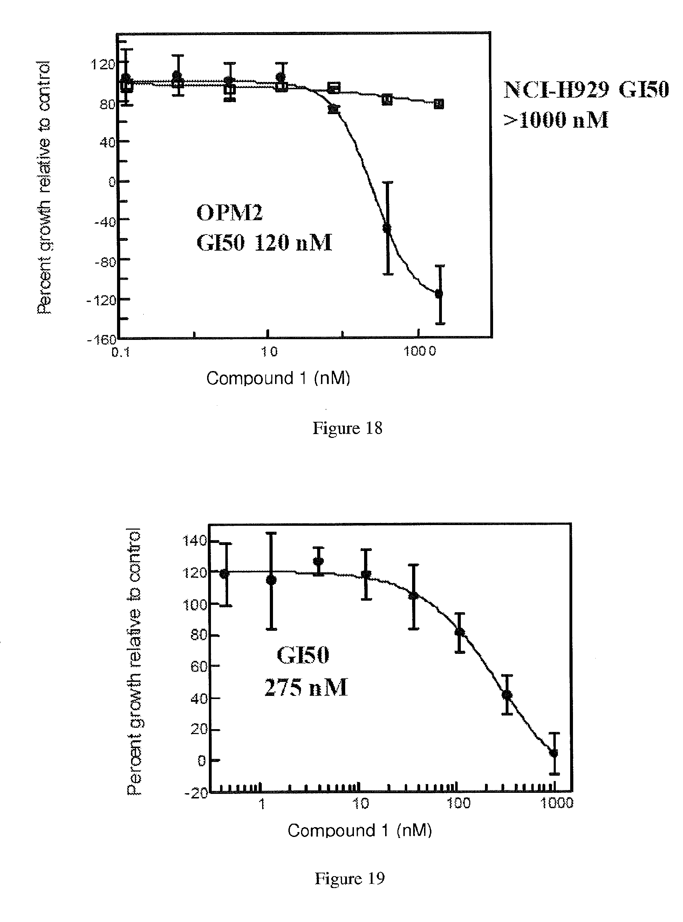

Compound 1 inhibited FGFR3 in cellular models for bladder cancer and multiple myeloma (MM). Compound 1 selectively inhibited the growth of bladder cancer MGH-U3 cells that express mutant FGFR3b (Y375C), when compared to wtFGFR3 RT112 cells (Figure 17). Compound 1 also inhibited signaling of FRS2a in MGH-U3 (IC50 = 41 nM for P-FRS2a), as determined by Western immunoblot analysis of protein expression in MGH-U3 cells treated with compound 1. Compound 1 selectively inhibited the growth of OPM2 MM cells that carry the t(4;14) translocation and express mutant FGFR3 (K650E), when compared to wtFGFR3 NCI-H929 cells (Figure 18). FGFR3 signaling was inhibited by compound 1 in OPM2 cells, as determined by Western immunoblot analysis of protein expression in OPM2 MM cells treated with compound 1 (data not shown). Furthermore, compound 1 inhibited signaling of FRS2a in OPM2 (IC50 = 65 nM for P-FRS2a).

Compound 1 inhibited FGFR4 in a cellular model for breast cancer. The effects of Compound 1 on MDA-MB-453 breast cancer cells that express mutant FGFR4 (Y367C) included inhibition of cell growth (Figure 19) and cellular signaling of FGFR4 and FRS2a, as determined by Western immunoblot analysis of protein expression in MDA-MB-453 cells treated with compound 1 (IC50 = 12 nM for P-FGFR4 and 14 nM for P-FRS2a).

Table 11 provides a comparison of RTK inhibitor activities in FGFR models for various kinase inhibitors, including CHIR-258, BIBF-1120, BMS-540215, and PD173074. IC50 (nM) data are shown for kinase assays performed by RBC and GI50 (nM) data shown for cell growth assays.

**Table 11. Comparison ofRTK inhibitor activities in FGFR models**

| FGF Receptor | Assay | Cell Line | Genotype | Compound 1 | CHIR-258 | BIBF 1120 | BMS-540215 | PD 173074 |
|---|---|---|---|---|---|---|---|---|
| FGFR1 | Kinase | - | - | 2 | 16 | 47 | 165 | 5 |
| | Cell growth | KG1 | Fusion | 10 | 40 | 221 | 900 | 10 |
| FGFR2 | Kinase | - | - | 2 | 50 | 63 | 202 | 13 |
| | Cell growth | SNU16 | Amplified | 42 | 138 | 496 | >1000 | 20 |
| | Cell growth | AN3CA | N549K | 30 | 122 | 684 | >1000 | 187 |
| FGFR3 | Kinase | - | - | 18 | 53 | 122 | 530 | 26 |
| | Cell growth | MGH-U3 | Y375C | 163 | 340 | >1000 | >1000 | 850 |
| | Cell growth | OPM2 | K650E | 120 | 319 | 297 | 6198 | 83 |
| FGFR4 | Kinase | - | - | 8 | 341 | 451 | 2023 | 367 |
| | Cell growth | MDA 453 | Y367C | 275 | 4227 | 1542 | >10000 | 1655 |

### Conclusion

Compound 1 is an orally active kinase inhibitor that exhibits potent activity against all four FGF receptors in kinase and cellular assays. Signaling and growth was inhibited in models expressing all four FGFRs with the most potent activity observed against FGFR1 and FGFR2. Activity of Compound 1 was observed in multiple cancer types, including gastric, endometrial, bladder and multiple myeloma. The activity of Compound 1 compared favorably to other RTK inhibitors with known FGFR activity that are being evaluated in the clinic.

### Example 7: Oral delivery of compound 1 was effective in reducing solid tumor growth in a FGFR2-driven AN3CA xenograft model.

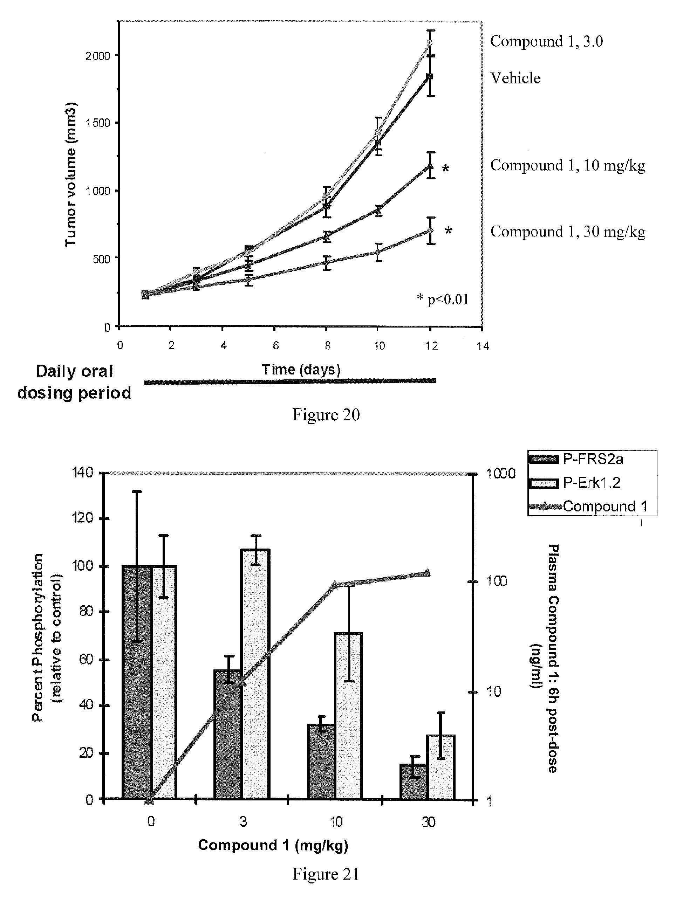

Compound 1 inhibited AN3CA tumor growth by 36% and 62% at 10 and 30 mg/kg oral dosages, respectively (Figure 20). Though daily dosage regimens are provided, intermittent dosage regimens may be also efficacious.

The in vivo pharmacodynamics and pharmacokinetics relationship was also determined, where plasma levels of compound 1 was determined 6 hours post-dose by Western immunoblot analysis (Figure 21). Oral dosing of compound 1 inhibited FRS2a and Erk1/2 signaling in the AN3CA xenograft (Figure 21).

Oral delivery of compound 1 potently inhibited tumor growth in an endometrial cancer model that expressed the clinically relevant FGFR2 (N549K) mutation. Inhibition of cellular growth correlated with inhibition of downstream signaling in the tumor. These data support the use of compound 1 in treating a number of tumor types characterized by alterations in FGF receptors.

### Example 8: Combination Therapy with Compound 1 and Ridaforolimus EXPERIMENTAL PROCEDURES

**Reagents:** Compound 1 and ridaforolimus (AP23573, MK-8669) were synthesized by ARIAD Pharmaceuticals. The following compounds were purchased: BMS-540215 (American Custom Chemical, San Diego, CA), CHIR-258 and AZD2171 and BIBF-1120 (Selleck Chemical Co., London ON, Canada).

**Kinase assay:** Kinase inhibition assays to determine IC50s were performed at Reaction Biology Corporation (RBC, Malvern, PA USA). Compounds were tested at 10 µM ATP using a 10-point curve with 3-fold serial dilutions starting at 1 µM. Average data from 2 assays are shown.

**Cell growth assay:** Cell growth was assessed using either Cell Titer 96 Aqueous One Solution Cell Proliferation Assay (Promega, Madison, WI) or CyQuant Cell proliferation Assay (Invitrogen, Carlsbad, CA). Twenty four hours after plating, cells were treated with compound and grown for 72 hours. The concentration that causes 50% growth inhibition (GI50) was determined by correcting for the cell count at time zero (time of treatment) and plotting data as percent growth relative to vehicle (DMSO) treated cells using XLfit version 4.2.2 for Microsoft Excel.

**Combination assay and analysis:** The Effective Dose @ 50% maximum inhibition (ED50) was determined for each compound tested and defined as 1x. The drug concentrations used ranged from 0.125x to 8x at a fixed ED ratio. Combinatorial effects on cell growth were analyzed using the Chou and Talalay method (CalcuSyn software, Biosoft).

**Western immunoblotting:** Cells were treated 24 hours after plating and incubated with inhibitor for 1 hour. Cells were lysed in either SDS buffer or Phospho-Safe (Novagen, Gibbstown, NJ) and protein lysates were immunoprecipitated overnight and/or immunoblotted with the indicated antibodies. Protein expression was quantified using Quantity One software (BioRad, Hercules, CA). The IC50 values (the concentration that causes 50% inhibition) were calculated by plotting percent inhibition of the phospho-signal normalized to the total protein signal using XLfit4. Data shown in the table are average values from 2-3 assays.

**Subcutaneous tumor models:** AN3CA cells were implanted into the right flank of nude mice. For analysis of efficacy, when the average tumor volume reached ∼200 mm³, inhibitor was administered by either daily oral dosing for 21 days for compound 1 or i.p. dosing QDX5 for 3 weeks. Mean tumor volumes (± SE; tumor volume= L x W² x 0.5) were calculated for each treatment group.

**Pharmacodynamics/Pharmacokinetics:** For pharmacodynamic analysis tumor samples were frozen upon collection, homogenized in Phospho-Safe and analyzed by Western immunoblotting. Inhibitor concentrations in plasma were determined by an internal standard LC/MS/MS method using protein precipitation and calibration standards prepared in blank mouse plasma. Data shown are mean values from 3 mice/timepoint/group.

Compound 1 affected cellular activity in various cancer cell lines. Table 12 provides a comparison of RTK inhibitor activities in FGFR cellular models for various kinase inhibitors, including AZD2171, CHIR-258, BIBF-1120, and BMS-540215. Compound 1 is a potent inhibitor for FGFR1-FGFR4. IC50 (nM) data for kinase assays were performed by RBC. Table 12 shows GI50 (nM) data for cell growth assay and IC50 (nM) data for signaling.

**Table 12. Comparison of RTK inhibitor activities in FGFR models**

| **FGF Receptor** | **Cell line** | **Genotype** | **Assay** | **Compound 1** | **AZD 2171** | **CHIR-258** | **BIBF 1120** | **BMS-540215** |
|---|---|---|---|---|---|---|---|---|
| **FGFR1** | - | wt | Kinase | **2** | 5 | 16 | 47 | 165 |
| | KG1 Leukemia | Fusion | Signaling | **3** | - | - | - | - |
| | | | Cell growth | **10** | 23 | 40 | 221 | 900 |
| | | | | | | | | |
| **FGFR2** | - | wt | Kinase | **2** | 33 | 50 | 63 | 202 |
| | SNU16 Gastric | Amplified | Signaling | **12** | - | - | - | - |
| | | | Cell growth | **42** | 148 | 138 | 496 | >1000 |
| | AN3CA N549K Endometrial | | Signaling | **4** | - | - | - | - |
| | | | Cell growth | **30** | 34 | 122 | 684 | >1000 |
| | | | | | | | | |
| **FGFR3** | - | wt | Kinase | **18** | 36 | 53 | 122 | 530 |
| | MGH-U3 Bladder | Y375C | Signaling | **40** | - | - | - | - |
| | | | Cell growth | **204** | 296 | 251 | >1000 | >1000 |
| | OPM2 myeloma | K650E | Signaling | **80** | - | - | - | - |
| | | | Cell growth | **120** | 296 | 319 | 297 | 6198 |
| | | | | | | | | |
| **FGFR4** | - | wt | Kinase | **8** | 697 | 341 | 451 | 2023 |
| | MDA-453 Breast | Y367C | Signaling | **30** | - | - | - | - |
| | | | Cell growth | **275** | >1000 | 4227 | 1542 | >10000 |

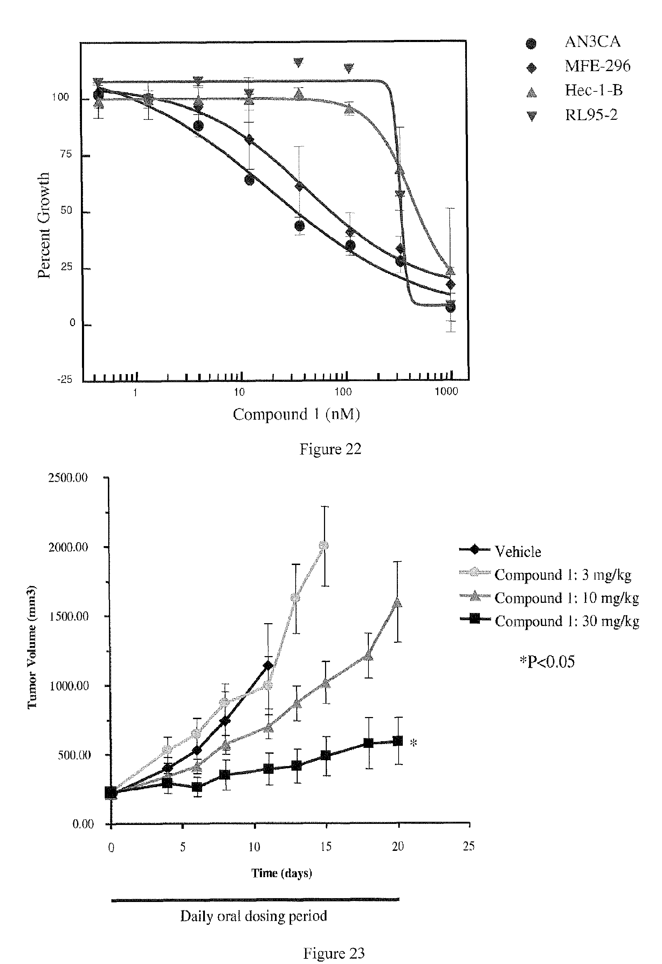

Compound 1 selectively inhibited growth and signaling of FGFR2-mutant endometrial cancer cell lines. Compound 1 inhibited endometrial cancer cell lines AN3CA and MFE-296, as compared to wtFGFR2 Hec-1-B and RL95-2 cell lines (Figure 22). Compound 1 inhibited signaling in AN3CA cells, particularly the phosphorylation of FRS2a and Erk 1/2, as determined by Western immunoblot analysis for the effect of various concentrations of compound 1 on AN3CA cells. Table 13 provides a summary of the activity of compound 1 in endometrial cancer cell lines AN3CA and MFE-296, as compared to the wtFGFR2 Hec-1-B and RL95-2 cell lines.

**Table 13. Summary of compound 1's activity in endometrial cancer cell lines**

| | | **Compound 1** | | |
|---|---|---|---|---|
| **Cell Line** | **FGFR2 Status** | **Phospho-FRS2a IC50 (nM)** | **Phospho-Erk1/2 IC50 (nM)** | **Growth GI50 (nM)** |
| AN3CA | N549K | 3.7 | 5.8 | 30 |
| MFE-296 | N549K | 2.7 | 5.5 | 72 |
| HEC-1-B | wt | >1000 | >1000 | 490 |
| RL95-2 | wt | >1000 | >1000 | 428 |

Oral delivery of compound 1 inhibited growth of FGFR-2 mutant AN3CA endometrial tumor xenograft. Compound 1 inhibited AN3CA tumor growth by 49% and 82% at 10 and 30 mg/kg, respectively (Figure 23). Compound 1 inhibited pharmacodynamic markers 6 hours post-dose. Oral dosing of compound 1 inhibited FRS2a and Erk1/2 signaling in the AN3CA xenograft, as determined by Western immunoblot analysis 6h post-dose for phosphorylated and non-phosphorylated FRS2a, phosphorylated and non-phosphorylated Erk1/, and glyceraldehyde-3-phosphate dehydrogenase as a control (data not shown).

These data show that compound 1 is a potent, orally available pan-FGFR inhibitor. Activity has been seen in multiple cancer types, including gastric, endometrial, bladder, and multiple myeloma. The activity of compound 1 compared favorably to other inhibitors with known FGFR activity, where these inhibitors are being evaluated in the clinic. In addition, oral compound 1 potently inhibits tumor growth in an endometrial cancer model that expresses the clinically relevant N549K mutation for FGFR2.

### Example 9: Synergistic anti-tumor activity of compound 1 with an mTOR inhibitor in cancer models.

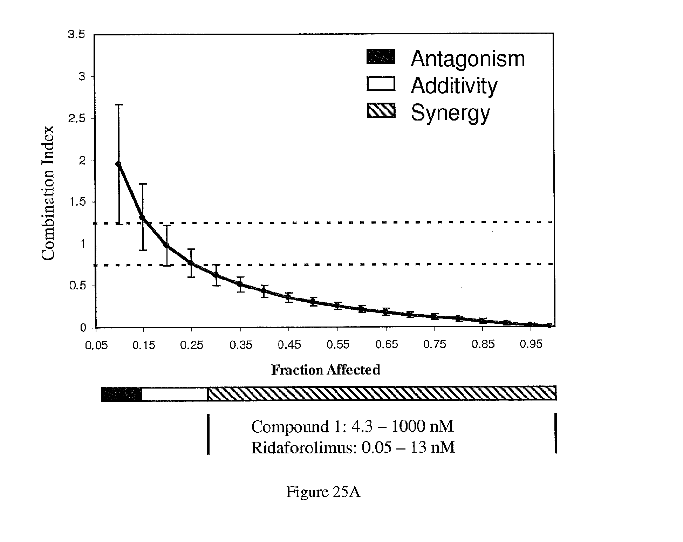

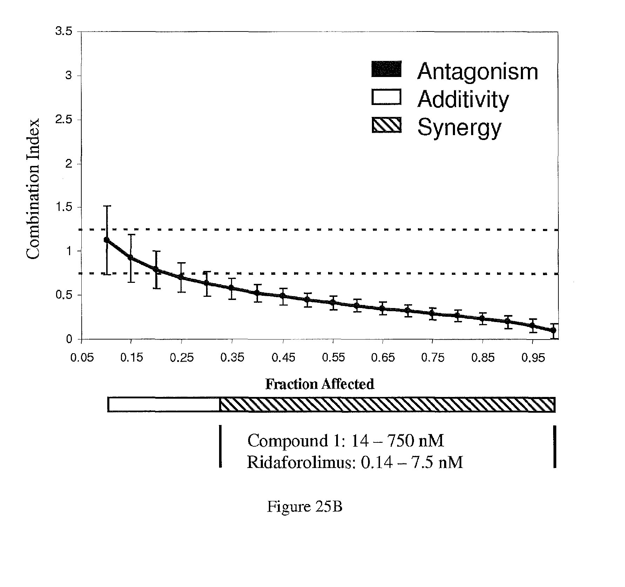

Figure 24A shows the growth inhibition of compound 1 on the AN3CA cell line for various concentrations ofridaforolimus, compound 1, and a combination of compound 1 with ridaforolimus. Figure 24B shows the growth inhibition of compound 1 on the MFE-296 cell line for various concentrations ofridaforolimus, compound 1, and a combination of compound 1 with ridaforolimus. Concentrations are given as function of EC50. The combination of compound 1 with ridaforolimus provided a synergistic effect, as compared to either compound alone, on both FGFR2-mutant endometrial cancer cell lines AN3CA and MFE-296. Median effect analyses of the combination of compound 1 with ridaforolimus on the AN3CA cell line are provided for the AN3CA cell line (Figure 25A) and the MFE-296 cell line (Figure 25B). For the AN3CA cell line, synergistic effect was observed within a concentration range of 4.3 to 1000 nM of compound 1 with 0.05 to 13 nM of ridaforolimus (Figure 25A). For the MFE-296 cell line, synergistic effect was observed within a concentration range of 14 to 750 nM of compound 1 with 0.14 to 7.5 nM of ridaforolimus (Figure 25B).

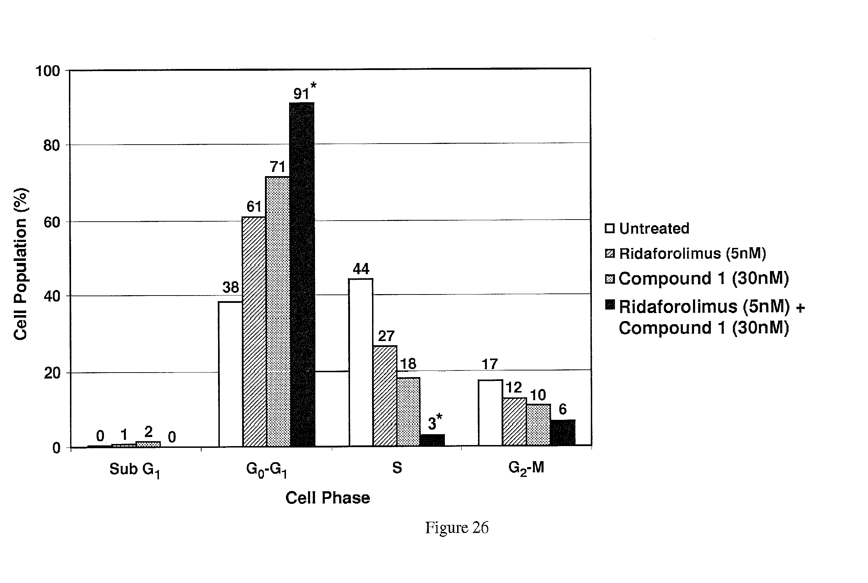

Cell cycle analyses were performed in AN3CA cells following 24 h treatment with ridaforolimus, compound 1, and the combination of compound 1 with ridaforolimus. Enhanced cell cycle arrest was observed during the G0-G1 cycle when treated with the combination of compound 1 with ridaforolimus, as compared to cells that were untreated or treated with one compound alone (Figure 26).

The effect of compound 1 and ridaforolimus on various signaling molecules in the AN3CA cell line was also determined by Western immunoblot analysis 24h post-dose. The combination of compound 1 (at 30 nM or 300 nM) with ridaforolimus (at 0.5 nM or 5 nM) resulted in inhibition of FRS2a, Erkl/2, and S6 signaling (data not shown).

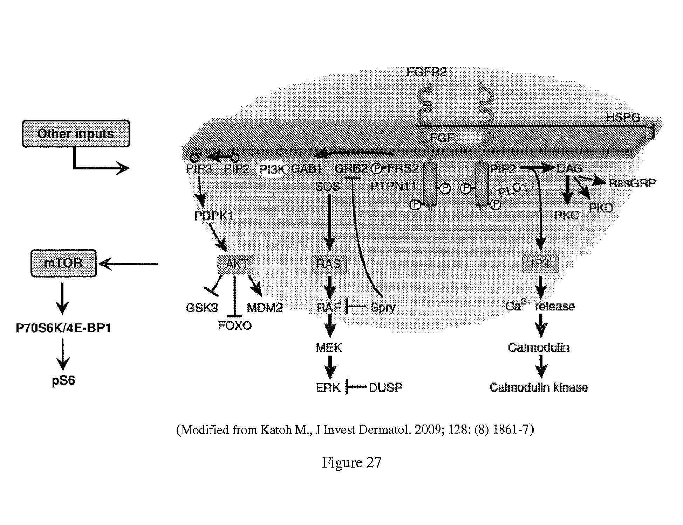

Figure 27 is a schematic showing a possible model of the FGFR2 and mTOR pathway. Without wishing to be limited by theory, compound 1 appears to inhibit the FGFR2/MAPK pathway and ridaforolimus appears to inhibit the mTOR pathway in this model.

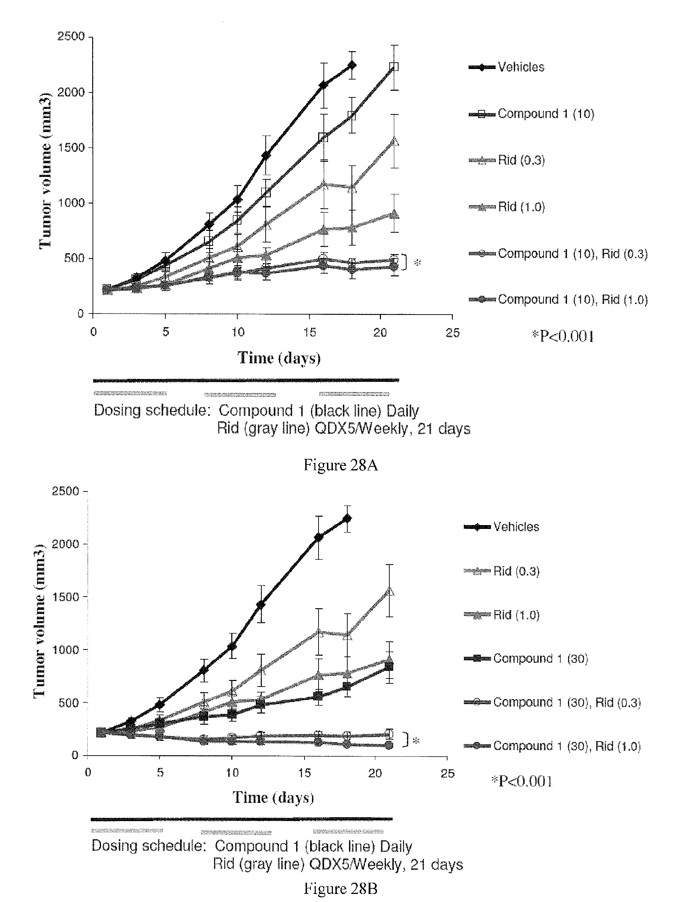

Upon oral delivery of the combination of compound 1 with ridaforolimus, enhanced anti-tumor activity was observed in the FGFR2-mutant AN3CA tumor xenograft. Figure 28A shows the inhibition of AN3CA tumor growth for a combination of a low dose of compound 1 (10 mg/kg) with ridaforolimus (0.3 mg/kg or 1.0 mg/kg). Figure 28B shows the inhibition of AN3CA tumor growth for a combination of a high dose of compound 1 (30 mg/kg) with ridaforolimus (0.3 mg/kg or 1.0 mg/kg). Results are shown for oral dosing of compound 1 daily (black lines in Figures 28A and 28B) and of ridaforolimus daily for five days of the week (gray lines in Figures 28A and 28B). Table 14 provides a summary of the efficacy of compound 1 and ridaforolimus in an AN3CA xenograft model, where "TGI" indicates tumor growth inhibition relative to vehicle.

**Table 14. Compound 1 and ridaforolimus efficacy in an AN3CA xenograft**

| model | | | |
|---|---|---|---|
| **Compound 1 (mg/kg)** | **Ridaforolimus (mg/kg)** | **TGI (%)** | **Regression (%)** |
| **10** | **-** | **26** | **-** |
| **30** | **-** | **81** | **-** |
| **-** | **0.3** | **48** | **-** |
| **-** | **1** | **70** | **-** |
| **10** | **0.3** | **84** | **-** |
| **10** | **1** | **88** | **-** |
| **30** | **0.3** | **-** | **10** |
| **30** | **1** | **-** | **43** |

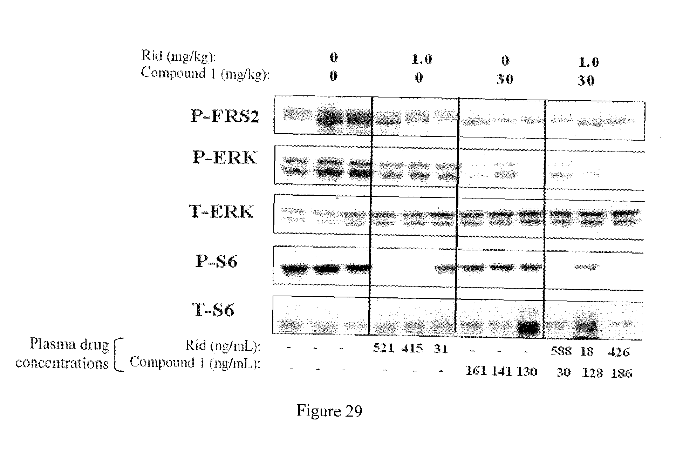

The in vivo pharmacodynamics and pharmacokinetics relationship was also determined at 6 hours post-dose (Figure 29). Also provided are plasma levels of compound 1 at 6 hours post-dose (Figure 29).

Synergistic activity of compound 1 and ridaforolimus was observed against FGFR2-mutant endometrial cancer cell growth. These data provide that compound 1 and ridaforolimus have potent combinatorial activity in FGFR2-mutant endometrial cancer models. Without wishing to be limited by theory, potent dual inhibition was achieved through the FGFR2/MAPK and mTOR pathways by compound 1 and ridaforolimus, respectively- Synergistic effects of the combination of compound 1 with ridaforolimus were observed via cell growth assays in vitro and tumor regression induced in vivo.

Compound 1 is a pan-FGFR inhibitor with potent activity in a variety of FGFR-driven tumor models. Dual inhibition of FGFR2 signaling by compound 1 and mTOR signaling by an mTOR inhibitor, such as ridaforolimus, leads to synergistic activity in FGFR2-driven endometrial cancer models in vitro and tumor regression in vivo.

These data provide support for the use of compound 1 in combination with an mTOR inhibitor for the treatment of disorders associated with pathological cellular proliferation, such as neoplasms, cancer, and conditions associated with pathological angiogenesis. Non-limiting examples of cancers which can be treated using the compositions, methods, or kits of the invention include carcinoma of the bladder, breast, colon, kidney, liver, lung, head and neck, gall-bladder, ovary, pancreas, stomach, cervix, thyroid, prostate, or skin; squamous cell carcinoma; endometrial cancer; multiple myeloma; a hematopoietic tumor of lymphoid lineage (e.g., leukemia, acute lymphocytic leukemia, acute lymphoblastic leukemia, B-cell lymphoma, T-cell-lymphoma, Hodgkin's lymphoma, non-Hodgkin's lymphoma, hairy cell lymphoma, or Burkitt's lymphoma); a hematopoietic tumor of myelogenous lineage (e.g., acute myelogenous leukemia, chronic myelogenous leukemia, multiple myelogenous leukemia, myelodysplastic syndrome, or promyelocytic leukemia); a tumor of mesenchymal origin (e.g., fibrosarcoma or rhabdomyosarcoma); a tumor of the central or peripheral nervous system (e.g., astrocytoma, neuroblastoma, glioma, or schwannomas); melanoma; seminoma; teratocarcinoma; osteosarcoma; and Kaposi's sarcoma. Non-limiting examples of conditions associated with aberrant angiogenesis which can be treated using the compositions, methods, or kits of the invention include solid tumors, diabetic retinopathy, rheumatoid arthritis, psoriasis, atherosclerosis, chronic inflammation, obesity, macular degeneration, and a cardiovascular disease.

### Other Embodiments

All publications, patents, and patent applications mentioned in this specification are herein incorporated by reference to the same extent as if each independent publication or patent application was specifically and individually indicated to be incorporated by reference. U.S. Provisional Patent Application Nos. 61/256,669, filed October 30, 2009, 61/256,690, filed October 30, 2009, and 61/261,014, filed November 13, 2009, are herein incorporated by reference.

While the invention has been described in connection with specific embodiments thereof, it will be understood that it is capable of further modifications and this application is intended to cover any variations, uses, or adaptations of the invention following, in general, the principles of the invention and including such departures from the present disclosure that come within known or customary practice within the art to which the invention pertains and may be applied to the essential features hereinbefore set forth, and follows in the scope of the claims.

Other embodiments are within the claims.

What is claimed is:

The present disclosure will now be further defined in the following numbered paragraphs:
1. A pharmaceutical composition suitable for oral administration comprising compound 1, or a pharmaceutically acceptable salt thereof, in an amount effective to treat a neoplasm, a cancer, or a hyperproliferative disorder when administered to a subject, and one or more pharmaceutically acceptable excipients.
2. The pharmaceutical composition of paragraph 1, comprising the hydrochloride salt of compound 1.
3. The pharmaceutical composition of paragraphs 1 or 2, which is formulated in unit dosage form.
4. The pharmaceutical composition of paragraph 3, comprising from 30 to 300 mg of compound 1.
5. The pharmaceutical composition of paragraph 3, comprising from 20±4 mg, 25±5 mg, 30±6 mg, 40±8 mg, or 45±9 mg of compound 1.
6. The pharmaceutical composition of paragraphs 1 or 2, which is formulated in a solid unit dosage form.
7. The pharmaceutical composition of paragraph 6, wherein said solid unit dosage form is a tablet, a soft capsule, or a hard capsule.
8. The pharmaceutical composition of paragraph 6, comprising from 30 to 300 mg of compound 1.
9. The pharmaceutical composition of paragraph 6, comprising from 20±4 mg, 25±5 mg, 30±6 mg, 40±8 mg, or 45±9 mg of compound 1.
10. A method of treating a neoplasm, a cancer, or a hyperproliferative disorder in a subject in need thereof, said method comprising orally administering to said subject from 30 to 300 mg of compound 1, or a pharmaceutically acceptable salt thereof.
11. The method of paragraph 10, wherein an average daily dose of 20±4 mg, 25±5 mg, 30±6 mg, 40±8 mg, or 45±9 mg of compound 1, or a pharmaceutically acceptable salt thereof, is orally administered to said subject in a unit dosage form.
12. The method of paragraph 10, wherein compound 1, or a pharmaceutically acceptable salt thereof, is administered to said subject more than one day a week or on average 4 to 7 times every 7 day period.
13. The method of paragraph 12, wherein compound 1, or a pharmaceutically acceptable salt thereof, is administered to said subject daily.
14. The method of any of paragraphs 10-13, wherein said subject has chronic myelogenous leukemia, acute lymphoblastic leukemia, acute myelogenous leukemia, a myelodysplastic syndrome, gastric cancer, endometrial cancer, bladder cancer, multiple myeloma, breast cancer, prostate cancer, lung cancer, colorectal cancer, renal cancer, or glioblastoma.
15. The method of paragraph 14, wherein said subject has chronic myelogenous leukemia, acute lymphoblastic leukemia, or acute myelogenous leukemia.
16. The method of any of paragraphs 10-15, wherein said subject has a condition refractory to treatment with imatinib, nilotinib, or dasatinib.
17. The method of any of paragraphs 10-16, wherein said subject has a Philadelphia chromosome positive condition.
18. The method of any of paragraphs 10-14, wherein said subject has a solid cancer refractory to treatment with a VEGF or VEGF-R inhibitor or antagonist.
19. The method of paragraph 18, wherein said solid cancer is refractory to treatment with bevacizumab, sorafenib, or sunitinib.
20. The method of any of paragraphs 10-17, wherein said subject has a cancer expressing a BCR-ABL mutant.
21. The method of paragraph 20, wherein said BCR-ABL mutant is BCR-ABL^{T1315I}, BCR-ABL^{F3I7L}, or BCR-ABL^{F359C}.
22. The method of any of paragraphs 10-19, wherein said subject has a cancer expressing a FLT3, KIT, FGFR1, or PDGFRα mutant.
23. The method of paragraph 22, wherein said mutant is FLT3-ITD, c-KIT, FGFR1OP2-FGFR1, or F1P1L1-PDGFRα.
24. The method of any of paragraphs 10-23, wherein said compound 1, or a pharmaceutically acceptable salt thereof, is administered together or concurrently with an mTOR inhibitor each in an amount that together is effective to treat said neoplasm, cancer, or hyperproliferative disorder.
25. The method of paragraph 24, wherein said mTOR inhibitor is selected from the group consisting of sirolimus, everolimus, temsirolimus, ridaforolimus, biolimus, zotarolimus, LY294002, Pp242, WYE-354, Ku-0063794, XL765, AZD8055, NVP-BEZ235, OSI-027, wortmannin, quercetin, myricentin, and staurosporine, and pharmaceutically acceptable salts thereof.
26. A kit comprising (i) a pharmaceutical composition suitable for oral administration of compound 1, or a pharmaceutically acceptable salt thereof, in an amount effective to treat a neoplasm, a cancer, or a hyperproliferative disorder when administered to a subject, and one or more pharmaceutically acceptable excipients; and (ii) instruction for administering said pharmaceutical composition to a subject for the treatment of said neoplasm, cancer, or hyperproliferative disorder.
27. The kit of paragraph 26, wherein said subject has chronic myelogenous leukemia, acute lymphoblastic leukemia, acute myelogenous leukemia, a myelodysplasia syndrome, gastric cancer, endometrial cancer, bladder cancer, multiple myeloma, breast cancer, prostate cancer, lung cancer, colorectal cancer, renal cancer, or glioblastoma.

## Claims

1. Ponatinib, or a pharmaceutically acceptable salt thereof, for use in treating a cancer **characterised by** the expression of RET or a mutant thereof.

2. Ponatinib, or a pharmaceutically acceptable salt thereof, for use in treating thyroid cancer.

3. Ponatinib, or a pharmaceutically acceptable salt thereof, for use according to claim 2, wherein the thyroid cancer is **characterised by** the expression of RET or a mutant thereof.

4. Ponatinib, or a pharmaceutically acceptable salt thereof, for use in treating non-small cell lung cancer.

5. Ponatinib, or a pharmaceutically acceptable salt thereof, for use according to claim 4, wherein the non-small cell lung cancer is **characterised by** the expression of RET or a mutant thereof.

6. Ponatinib, or a pharmaceutically acceptable salt thereof, for use according to claim 1, 3 or 5, wherein the RET or a mutant thereof is selected from RET, RET(V804L) or RET(V804M).


SEQUENCE LISTING

<110> ARIAD Pharmaceuticals, Inc.

<120> Methods and Compositions for Treating Cancer

<130> P100124EPA

<140> 13189355.4
<141> 2010-11-01

<150> US 61/256,669
<151> 2009-10-30

<150> US 61/256,690
<151> 2009-10-30

<150> US 61/261,014
<151> 2009-11-13

<160> 4

<170> PatentIn version 3.5

<210> 1
<211> 22
<212> DNA
<213> Artificial Sequence

<220>
<223> Synthetic primer B2A

<400> 1
ttcagaagct tctccctgac at 22

<210> 2
<211> 19
<212> DNA
<213> Artificial Sequence

<220>
<223> Synthetic primer ABL4317R

<400> 2
agctctcctg gaggtcctc 19

<210> 3
<211> 21
<212> DNA
<213> Artificial Sequence

<220>
<223> Synthetic primer ABL3335F

<400> 3
accacgctcc attatccagc c 21

<210> 4
<211> 18
<212> DNA

<213>  Artificial Sequence

<220>
<223>  Synthetic primer ABL4275R

<400>  4
cctgcagcaa ggtagtca                                                    18